(19) Europäisches Patentamt
European Patent Office
Office européen des brevets

(11) **EP 2 652 508 B1**

(12) **EUROPEAN PATENT SPECIFICATION**

(45) Date of publication and mention
of the grant of the patent:
**07.02.2018 Bulletin 2018/06**

(21) Application number: **11849217.2**

(22) Date of filing: **07.12.2011**

(51) Int Cl.:
***G01N 33/574*** *(2006.01)*  ***G01N 33/68*** *(2006.01)*
***G01N 33/53*** *(2006.01)*

(86) International application number:
**PCT/US2011/063761**

(87) International publication number:
**WO 2012/082494 (21.06.2012 Gazette 2012/25)**

(54) **THE USE OF ANTI-CXCL13 AND ANTI-CXCR5 ANTIBODIES FOR THE TREATMENT OR DETECTION OF CANCER**

VERWENDUNG VON ANTI-CXCL13- UND ANTI-CXCR5-ANTIKÖRPERN ZUR BEHANDLUNG ODER ZUM NACHWEIS VON KREBS

UTILISATION D'ANTICORPS ANTI-CXCL13 ET ANTI-CXCR5 POUR LE TRAITEMENT OU LA DÉTECTION DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2011 US 201113248904**
**06.12.2011 US 201113312343**
**15.09.2011 US 201113233769**
**14.12.2010 US 967273**

(43) Date of publication of application:
**23.10.2013 Bulletin 2013/43**

(73) Proprietor: **Jyant Technologies, Inc.**
**Marietta, Georgia 30008 (US)**

(72) Inventors:
• **LILLARD, James W.**
**Smyrna GA (US)**
• **SINGH, Shailesh**
**Powder Springs GA 30127 (US)**
• **SINGH, Rajesh**
**Atlanta GA 30329 (US)**

(74) Representative: **Fuller, Jessica**
**TL Brand & Co**
**17 Hanover Square**
**London W1S 1BN (GB)**

(56) References cited:
**WO-A1-01/37872**  **WO-A1-2004/015426**
**WO-A1-2008/102123**  **WO-A1-2009/066820**

WO-A1-2010/003771  WO-A2-2004/045526
WO-A2-2012/031099  US-A1- 2009 215 053

• W VERMI ET AL: "Identification of CXCL13 as a new marker for follicular dendritic cell sarcoma", THE JOURNAL OF PATHOLOGY, vol. 216, no. 3, 1 November 2008 (2008-11-01), pages 356-364, XP055164177, ISSN: 0022-3417, DOI: 10.1002/path.2420
• S. YUVARAJ ET AL: "A Novel Function of CXCL13 to Stimulate RANK Ligand Expression in Oral Squamous Cell Carcinoma Cells", MOLECULAR CANCER RESEARCH, vol. 7, no. 8, 1 August 2009 (2009-08-01), pages 1399-1407, XP055164181, ISSN: 1541-7786, DOI: 10.1158/1541-7786.MCR-08-0589
• RODRÍGUEZ-PINILLA SOCORRO MARÍA ET AL: "Peripheral T-cell lymphoma with follicular T-cell markers.", THE AMERICAN JOURNAL OF SURGICAL PATHOLOGY DEC 2008, vol. 32, no. 12, December 2008 (2008-12), pages 1787-1799, XP008174404, ISSN: 1532-0979
• A. BURKLE ET AL: "Overexpression of the CXCR5 chemokine receptor, and its ligand, CXCL13 in B-cell chronic lymphocytic leukemia", BLOOD, vol. 110, no. 9, 1 November 2007 (2007-11-01), pages 3316-3325, XP055005159, ISSN: 0006-4971, DOI: 10.1182/blood-2007-05-089409

- SUBRAMANYA N.M. PANDRUVADA ET AL: "Role of CXC chemokine ligand 13 in oral squamous cell carcinoma associated osteolysis in athymic mice", INTERNATIONAL JOURNAL OF CANCER, 1 January 2009 (2009-01-01), pages NA-NA, XP055164183, ISSN: 0020-7136, DOI: 10.1002/ijc.24920
- A. VACHANI ET AL: "A 10-Gene Classifier for Distinguishing Head and Neck Squamous Cell Carcinoma and Lung Squamous Cell Carcinoma", CLINICAL CANCER RESEARCH, vol. 13, no. 10, 15 May 2007 (2007-05-15), pages 2905-2915, XP055164188, ISSN: 1078-0432, DOI: 10.1158/1078-0432.CCR-06-1670
- L.-Y. FENG ET AL: "Involvement of a Novel Chemokine Decoy Receptor CCX-CKR in Breast Cancer Growth, Metastasis and Patient Survival", CLINICAL CANCER RESEARCH, vol. 15, no. 9, 21 April 2009 (2009-04-21), pages 2962-2970, XP055142752, ISSN: 1078-0432, DOI: 10.1158/1078-0432.CCR-08-2495
- H. CHUNSONG ET AL: "CXC Chemokine Ligand 13 and CC Chemokine Ligand 19 Cooperatively Render Resistance to Apoptosis in B Cell Lineage Acute and Chronic Lymphocytic Leukemia CD23+CD5+ B Cells", THE JOURNAL OF IMMUNOLOGY, vol. 177, no. 10, 2 November 2006 (2006-11-02), pages 6713-6722, XP055164186, ISSN: 0022-1767, DOI: 10.4049/jimmunol.177.10.6713
- ORTONNE NICOLAS ET AL: "Characterization of CXCL13+ neoplastic t cells in cutaneous lesions of angioimmunoblastic T-cell lymphoma (AITL)", AMERICAN JOURNAL OF SURGICAL PATHOLOGY, RAVEN PRESS, NEW YORK, NY, US, vol. 31, no. 7, 1 July 2007 (2007-07-01), pages 1068-1076, XP008174403, ISSN: 0147-5185, DOI: 10.1097/PAS.0B013E31802DF4EF
- R&D SYSTEMS: "Monoclonal anti-human CXCL13/BLC/BCA-1 antibody", R&D SYSTEMS DATA SHEET,, 11 March 2006 (2006-03-11), pages 1-2, XP002483019,
- MERAV DARASH-YAHANA ET AL: "The Chemokine CXCL16 and Its Receptor, CXCR6, as Markers and Promoters of Inflammation-Associated Cancers", PLOS ONE, vol. 4, no. 8, 19 August 2009 (2009-08-19), page e6695, XP055109095, DOI: 10.1371/journal.pone.0006695
- S. HOJO ET AL: "High-Level Expression of Chemokine CXCL16 by Tumor Cells Correlates with a Good Prognosis and Increased Tumor-Infiltrating Lymphocytes in Colorectal Cancer", CANCER RESEARCH, vol. 67, no. 10, 15 May 2007 (2007-05-15), pages 4725-4731, XP055165839, ISSN: 0008-5472, DOI: 10.1158/0008-5472.CAN-06-3424
- GUTWEIN P ET AL: "Tumoural CXCL16 expression is a novel prognostic marker of longer survival times in renal cell cancer patients", EUROPEAN JOURNAL OF CANCER, PERGAMON, vol. 45, no. 3, 1 February 2009 (2009-02-01), pages 478-489, XP025910630, ISSN: 0959-8049, DOI: 10.1016/J.EJCA.2008.10.023 [retrieved on 2008-12-11]
- J. MEIJER ET AL: "The CXCR5 Chemokine Receptor Is Expressed by Carcinoma Cells and Promotes Growth of Colon Carcinoma in the Liver", CANCER RESEARCH, vol. 66, no. 19, 1 October 2006 (2006-10-01), pages 9576-9582, XP055109084, ISSN: 0008-5472, DOI: 10.1158/0008-5472.CAN-06-1507
- SHAILESH SINGH ET AL.: 'Clinical and biological significance of CXCR5 expressed by prostate cancer specimens and cell lines.' INTERNATIONAL JOURNAL OF CANCER. vol. 125, no. 10, 2009, pages 2288 - 2295, XP055109090
- CHRISTELLE P EL HAIBI ET AL.: 'PI3Kpll0-, Src-, FAK-dependent and DOCK2- independent migration and invasion of CXCL13-stimulated prostate cancer cells.' MOLECULAR CANCER. vol. 9, no. 85, 22 April 2010, pages 1 - 9, XP021078056

**Description**

This application claims priority from the U.S. Patent Application No. 13/248,904, filed September 29, 2011, which is a continuation-in-part of Patent Application No. 13/233,769, filed on September 15, 2011, which is a continuation-in-part of Patent Application 12/967,273, filed on December 14, 2010. This application is also claims priority from the U.S. Patent Application No. 13/312,343, filed December 6, 2011, which is a continuation-in-part of U.S. Patent Application No. 12/967,273, filed on December 14, 2010.

**FIELD**

This application generally relates to the field of cancer. In particular, the application relates to a method for detecting or treating cancer using anti-chemokine and/or anti-chemokine receptor antibodies.

**BACKGROUND**

Cancer is one of the leading cause of death in the United States. Most cancer starts in just a single neoplastic cell. The neoplastic cell proliferate to form a local "tumor." A tumor simply means a swelling; it is not necessarily cancerous. A tumor which only grows in its place or origin, and cannot spread distantly, is a benign tumor and is not cancer. However, a tumor which has the capacity to spread (whether it actually does or not) is called a malignant tumor or cancer. A cancer may spread via the blood or lymphatic system to regional lymph nodes and to distant sites via a process called metastasis. A metastasized cancer is more difficult to treat because it now spreads into many different tissues and organs. It has been demonstrated that early treatment increase survival in many types of cancers, such as breast cancer, colon cancer, ovarian cancer and prostate cancer.

Chemokines are a superfamily of small, cytokine-like proteins that are resistant to hydrolysis, promote neovascularization or endothelial cell growth inhibition, induce cytoskeletal rearrangement, activate or inactivate lymphocytes, and mediate chemotaxis through interactions with G-protein coupled receptors. Chemokines can mediate the growth and migration of host cells that express their receptors.

CXCL13 is a small cytokine belonging to the CXC chemokine family. As its name suggests, this chemokine is selectively chemotactic for B cells belonging to both the B-1 and B-2 subsets, and elicits its effects by interacting with chemokine receptor CXCR5. CXCL13 and CXCR5 control the organization of B cells within follicles of lymphoid tissues. CXCR5 is expressed highly in the liver, spleen, lymph nodes, and gut of humans. CXCR5 plays an essential role in B cell migration.

In T-lymphocytes, CXCL13 expression may reflect a germinal center origin of the T-cell. Hence, expression of CXCL13 in T-cell lymphomas, such as angioimmunoblastic T-cell Lymphoma, is thought to reflect a germinal center origin of the neoplastic T-cells.

WO2010003771 discloses a method for the prediction of breast cancer by determining in a biological sample from said patient an expression level of a plurality of genes selected from the group consisting of ACTG1, CA12, CALM2, CCND1, CHPT1, CLEC2B, CTSB, CXCL13, DCN, DHRS2, EIF4B, ERBB2, ESR1, FBXO28, GABRP, GAPDH, H2AFZ, IGFBP3, IGHG1, IGKC, KCTD3, KIAA0101, KRT17, MLPH, MMP1, NAT1, NEK2, NR2F2, OAZ1, PCNA, PDLIM5, PGR, PPIA, PRC1, RACGAP1, RPL37A, SOX4, TOP2A, UBE2C and VEGF.

W. Vermi et al., "Identification of CXCL13 as anew marker for follicular dendritic cell sarcoma" discloses that CXCL13 is produced by dysplastic and neoplastic FDCs and can be instrumental in recruiting intratumoural CXCR5+ lymphocytes.

S Yuvaraj et al, "A novel function of CXCL13 to Stimulate RANK Ligand Expression in Oral Squamous Cell Carcinoma Cells" suggests that NFATc3 is a downstream target of the CXCL13/CXCR5 axis to stimulate RANKL expression in OSCC cells and implicates CXCL13 as a potential therapeutic target to prevent OSCC bone invasion/osteolysis.

Rodrigues-Pinilla Socorro Maria et al., "Peripheral T-cell lymphoma with follicular T-cell markers" discloses that CXCL13 expression was found in 96% of PD-1 expressing PTCL cases tested.

A Burkle et al., "Overexpression of the CXCR5 chemokine receptor, and its ligand, CXCL13 in B-cell chronic lymphocytic leukemia" suggests that CXCR5 plays a role in CLL cell positioning and cognate interactions between CLL and CXCL13-secreting CD68+ accessory cells in lymphoid tissues.

Subramanya N.M. Pandruvada et al., "Role of CXC chemokine ligand 13 in oral squamous cell carcinoma associated osteolysis in athymic mice" suggests a functional role for CXCL13 in bone invasion and may be a potential therapeutic target to prevent osteolysis associated with OSCC tumors *in vivo*.

R&D Systems, "Monoclonal anti-human CXCL13/BLC/BCA-1 antibody" discloses a monoclonal antibody for CXCL13.

WO2008102123 discloses binding members, especially antibody molecules, for CXCL13, which are useful for the treatment of disorders associated with CXCL13, including arthritic disorders such as rheumatoid arthritis.

WO2012031099 discloses compositions and methods for treating diseases associated with CXCL13 expression, including certain autoimmune diseases, inflammatory diseases, and cancers.

Merav Darash-Yahana et al., "The Chemokine CXCL16 and its Receptor, CXCR6, as Markers and Promoters of Inflammation-Associated Cancers" suggests that CXCL16 and CXCR6 may mark cancers arising in an inflammatory milieu and mediate pro-tumorigenic effects of inflammation through direct effects on cancer cell growth and by inducing the migration and proliferation of tumor-associated leukocytes.

S. Hojo et al., "High-Level Expression of Chemokine CXCL 16 by Tumor Cells Correlates with a Good Prognosis and Increased Tumor-Infiltrating Lymphocytes in Colorectal Cancer" suggests that the expression of CXCL16 by tumor cells enhances the recruitment of tumor-infiltrating lymphocytes, thereby bringing about a better prognosis in colorectal cancer.

Gutwein P et al., "Tumoural CXCL16 expression is a novel prognostic marker of longer survival times in renal cell cancer patients" suggests that downregulation of CXCL16 plays an important role in renal cancer development and progression, and that CXCL16 in renal cell carcinoma is an independent prognostic marker for better patient survival.

WO0137872 discloses an antibody or antigen-binding fragment thereof which binds to CXCR6 and blocks the binding of a ligand such as CXCL16 to the receptor.

WO2004015426 discloses a human CXCR5 which is associated with the cancer diseases, cardiovascular diseases, disorders of the peripheral and central nervous system, respiratory diseases and diseases of the hematological systems.

## SUMMARY

One aspect of the present invention relates to a method for treating melanoma, sarcoma, blastoma, carcinoma, lymphoma, myeloma or leukemia in a subject. In one embodiment, the method comprises the step of administering to the subject a therapeutically effective amount of an anti-CXCL13 antibody, or an anti-CXCR5 antibody, or a combination thereof. In another embodiment, the method comprises the step of immunizing the subject with an effective amount of CXCL13 and/or CXCR5 immunogen(s) as protein, peptide or encoded gene to induce antibodies that inhibit the biological activity of CXCL13 and/or CXCR5. In another embodiment, the method comprises the step of administering to the subject an expression vector that expresses an anti-CXCL13 antibody, or an anti-CXCR5 antibody, or a combination thereof in said subject. In another embodiment, the method comprises the step of administering to the subject an effective amount of an expression vector that expresses an agent capable of (1) inhibiting the expression of CXCL13 and/or CXCR5, (2) inhibiting the interaction between CXCL13 and CXCR5, or (3) inhibiting a biological activity of CXCL13 and/or CXCR5.

Another aspect of the present application relates to a method for prevention or inhibition of the migration or metastasis of cancer cells with elevated expression of CXCL13 and/or CXCR5 in a subject. In one embodiment, the method comprises the step of administering to the subject a therapeutically effective amount of an anti-CXCL13 antibody, an anti-CXCR5 antibody, or a combination thereof. In another embodiment, the method comprises the step of immunizing the subject with an effective amount of CXCL13 and/or CXCR5 immunogen(s) as protein, peptide or encoded gene to induce antibodies that inhibit the biological activity of CXCL13 and/or CXCR5. In another embodiment, the method comprises the step of administering to the subject an expression vector that expresses an anti-CXCL13 antibody, or an anti-CXCR5 antibody, or a combination thereof in said subject. In another embodiment, the method comprises the step of administering to the subject an effective amount of an expression vector that expresses an agent capable of (1) inhibiting the expression of CXCL13 and/or CXCR5, (2) inhibiting the interaction between CXCL13 and CXCR5, or (3) inhibiting a biological activity of CXCL13 and/or CXCR5.

Also described herein is a method for treating cancer in a subject. The method comprises the steps of detecting a level of CXCL13 expression and/or CXCR5 expression in a biological sample from said subject and, if an elevated level of CXCL13 expression and/or CXCR5 expression is detected in said biological sample, administering to the subject (1) a therapeutically effective amount of an antibody to CXCL13 and/or an antibody to CXCR5 or (2) an expression vector that expresses an anti-CXCL13 antibody, or an anti-CXCR5 antibody, or a combination thereof in said subject. The method may comprise the steps of detecting a level of CXCL13 expression and/or CXCR5 expression in a biological sample from said subject and, if an elevated level of CXCL13 and/or CXCR3 expression is detected in said biological sample, immunizing the subject with an effective amount of CXCL13 and/or CXCR5 immunogen to elicit an antibody response to inhibit the biological activity of CXCL13 and/or CXCR5. The method may comprise the step of detecting a level of CXCL13 expression and/or CXCR5 expression in a biological sample from said subject and, if an elevated level of CXCL13 expression and/or CXCR5 expression is detected in said biological sample, administering to the subject an effective amount of an expression vector that expresses an agent capable of (1) inhibiting the expression of CXCL13 and/or CXCR5, or (2) inhibiting the interaction between CXCL13 and CXCR5, or (3) inhibiting a biological activity of CXCL13 and/or CXCR5.

Also described herein is a method for enhancing the effect of chemotherapy. The method comprises administering to a subject who is under chemotherapy for a cancer, an effective amount of an anti-CXCL13 antibody, or an anti-CXCR5 antibody, or a combination thereof. The method may comprise the step of immunizing the subject who is under chemotherapy for a cancer with an effective amount of CXCL13 and/or CXCR5 immunogen(s) as protein, peptide or encoded gene to induce antibodies that inhibit the biological activity of CXCL13 and/or CXCR5. The method may comprise the step of administering to the subject who is under chemotherapy for a cancer an expression vector that expresses an anti-CXCL13 antibody, or an anti-CXCR5 antibody, or a combination thereof. The method may comprise the step of administering to the subject an effective amount of an expression vector that expresses an agent capable of (1) inhibiting the expression of CXCL13 and/or CXCR5, or (2) inhibiting the interaction between CXCL13 and CXCR5, or (3) inhibiting a biological activity of CXCL13 and/or CXCR5

Also described herein is a method for treating cancer in a subject by immunizing the subject with with an effective amount of one or more of CXCL13 and CXCR5 immunogens and an effective amount of one or more of CXCL16 and CXCR6 immunogens, wherein said cancer is melanoma, lymphoma, leukemia, sarcoma, blastoma, or carcinoma.

Also described herein is a method for treating cancer in a subject by immunizing the subject with an effective amount of a CXCL16 immunogen and/or a CXCR6 immunogen to induce antibodies that inhibit the biological activity of CXCL16 and/or CXCR6, wherein the cancer is melanoma, lymphoma, leukemia, sarcoma, blastoma, or carcinoma, wherein the CXCL16 immunogen is a peptide comprising one or more sequences selected from the group consisting of AAGPEAGENQKQPEKN (SEQ ID NO:87), SQASEGASSDIHTPAQ (SEQ ID NO:88), STLQSTQRPTLPVGSL (SEQ ID NO:89), SWSVCGGNKDPWVQEL (SEQ ID NO:90), GPTARTSATVPVLCLL (SEQ ID NO:91), SGIVAHQKHLLPTSPP (SEQ ID NO:92), RLRKHL (SEQ ID NO:93), LQSTQRP (SEQ ID NO:94), SSDKELTRPNETT (SEQ ID NO:95), AGENQKQPEKNA (SEQ ID NO:96), NEGSVT (SEQ ID NO:97), ISSDSPPSV (SEQ ID NO:98), CGGNKDPW (SEQ ID NO:99), LLPTSPPISQASEGASSDIHT (SEQ ID NO:100), STQRPTLPVGSLSSDKELTRPNETTIHT (SEQ ID NO:101), SLAAGPEAGENQKQPEKNAGPTARTSA (SEQ ID NO:102), TGSCYCGKR (SEQ ID NO:103), DSPPSVQ (SEQ ID NO:104), RKHLRAYHRCLYYTRFQLLSWSVCGG (SEQ ID NO:105), WVQELMSCLDLKECGHAYSGIVAHQKHLLPTSPPISQ (SEQ ID NO:106), SDIHTPAQMLLSTLQ (SEQ ID NO:107), RPTLPVGSL (SEQ ID NO:108), TAGHSLAAG (SEQ ID NO:109), GKRISSDSPPSVQ (SEQ ID NO:110) and KDPWVQELMSCLDLKECGHAYSGIVAHQKH (SEQ ID NO:111), and wherein the CXCR6 immunogen is a peptide comprising one or more sequences selected from the group consisting of HQDFLQFSKV (SEQ ID NO:112), AGIHEWVFGQVMCK (SEQ ID NO:113), PQIIYGNVFNLDKLICGYHDEAI (SEQ ID NO:114) and YYAMTSFHYTIMVTEA (SEQ ID NO:115).

One aspect of the present application relates to detecting cancer in a subject. The method comprises detecting the level of expression of one or more cancer markers in a biological sample obtained from the subject; and comparing the level of expression of the one or more cancer markers in the biological sample to a normal level of expression of the one or more cancer markers, wherein a higher than normal level of expression of said one or more cancer markers in the biological sample is indicative of the presence of cancer in the subject, wherein the normal level of expression of the one or more cancer markers is a predetermined value or is obtained from a control sample of known normal non-cancerous cells of the same origin or type as the biological sample, wherein the cancer is ovarian cancer, prostate cancer, colon cancer, breast cancer or lung cancer, and wherein the one or more cancer markers comprises CXCL13 or CXCR5 or both CXCL13 and CXCR5.

Another embodiment of the present application relates to a method for assessing the prognosis of a subject with a cancer. The method comprises determining the expression level of one or more cancer markers in a biological sample from the subject, and comparing the level of expression of the one or more cancer markers in the biological sample to a control level of expression of the one or more cancer markers, wherein a higher level of expression of the one or more cancer markers in the biological sample relative to the control level indicates that the prognosis of the subject is poor, and wherein a lower or similar level of expression of the one or more cancer markers in the biological sample relative to the control level indicates that the prognosis of the subject is good, wherein a poor prognosis indicates that the cancer is of an aggressive or invasive type, wherein the cancer is ovarian cancer, prostate cancer, colon cancer, breast cancer or lung cancer, and wherein the one or more cancer markers comprise CXCL13 or CXCR5 or both CXCL13 and CXCR5.

Another embodiment of the present application relates to a method for monitoring the course of cancer treatment in a subject. The method comprises determining the expression levels of one or more cancer markers in one or more biological samples obtained from the subject during or after the treatment, and comparing the level of expression of the one or more cancer markers in the one or more biological samples to a control level of expression of the one or more cancer markers, wherein the control level of the one or more cancer markers is a pre-treatment level of the one or more cancer markers in the subject or a predetermined reference level, wherein the treatment is deemed efficacious if the one or more cancer markers in the one or more biological samples is similar to or lower than the control level, wherein the cancer is ovarian cancer, prostate cancer, colon cancer, breast cancer or lung cancer, and wherein the one or more cancer markers comprise CXCL13 or CXCR5 or both CXCL13 and CXCR5.

Another aspect of the present application relates to a kit for detecting cancer or monitoring progression of cancer. The kit comprises reagents for determining expression of CXCL13 CXCR5, CXCL16 and/or CXCR6 in a biological sample; and instructions for how to use the reagents, wherein the reagents comprise an anti-CXCL13 antibody, an anti-CXCR5 antibody, or both and an anti-CXCL16 antibody, an anti-CXCR6 antibody, or both and wherein said cancer is ovarian cancer, prostate cancer, colon cancer, breast cancer, or lung cancer.

## BRIEF DESCRIPTION OF THE DRAWINGS

FIGS. 1A-B show expression of DOCK2 by prostate cancer (PCa) cell lines.

FIG. 2 shows that CXCL13-CXCR5 interaction promotes LNCaP and PC3 cell invasion independent of DOCK2.

FIG. 3 shows CXCL13 regulation of Akt and ERK1/2 activation.

FIG. 4 shows that CXCL13 induces JNK activation through DOCK2 in PC3 cells.

FIG. 5 shows CXCL13 regulation of PCa cell proliferation through JNK and DOCK2.

FIG. 6 shows that JNK inhibition and DOCK2 knockdown lead to reduction of PCa cell proliferation not due to cell death by apoptosis.

FIG. 7 shows CXCL13 modulation of signaling cascades in PCa cell lines.

FIGS. 8A-C show the expression of $\alpha$ subunit isoforms of G-protein by prostate cancer cell lines.

FIGS. 9A-B show G-protein $\beta$ and $\gamma$ subunit isoform expression of by prostate cancer cell lines.

FIGS. 10A-D show expression of CXCR5 and associated G proteins in prostate cancer cell lines treated with or without CXCL13.

FIGS. 11A-B depict validation of $G_{q/11}$ and $G_{\alpha i2}$ protein association with CXCR5 by immunoprecipitation.

FIGS. 12A-C show the identification of CXCR4 and CXCR5 coupled to $G_{\alpha 13}$ following CXCL13 stimulation.

FIG. 13 depicts a hypothetical model of CXCR5 interactions in prostate cancer cells.

FIG. 14 depicts how CXCL13 regulates key molecules involved in the cell cycle.

FIG. 15 depicts how CXCL13 regulates key molecules involved in cell migration.

FIG. 16 depicts how CXCL13 regulates key molecules involved in cell survival and growth.

FIG. 17 depicts the top Canonical pathways regulated by CXCL13 in PC3 cells.

FIG. 18 shows CXCL13 mediation of differential phosphorylation of proteins belonging to the PI3K/Akt and SAPK/JNK signaling pathways.

FIG. 19 is a summary diagram of signaling pathways modulated by CXCL13-CXCR5 interactions.

FIG. 20 shows confirmation of major CXCL13-CXCR5 cell signaling cascades.

FIG. 21 shows CXCL13-CXCR5 signaling events required for AKT activation.

FIGS. 22A-B depict CXCL13-mediated CXCR5 ligation and translocation to nuclei.

FIGS. 23A-B show that anti-CXCL13 antibody treatment inhibits prostate cancer progression and bone metastasis.

FIGS. 24A-B show that CXCL13 blockade inhibits prostate tumor growth in bone.

FIGS. 25A-B show that CXCL13 blockade abrogates osteolytic prostate tumor growth in bone.

FIGS. 26A-B show that CXCL13 blockade inhibits loss of bone mineral density (BMD) induced by prostate cancer metastasis to bone.

FIG. 27 depicts the levels of CXCL13 in serum of normal healthy controls and lung cancer subjects.

FIG. 28 shows CXCR5 expression by non-neoplastic lung and lung cancer tissue.

FIG. 29 shows CXCR5 expression by non-neoplastic mammary and breast cancer tissue.

FIG. 30 is a depiction of CXCR5 and CXCL13 expression by colon cancer tissue relative to nonneoplastic controls.

FIG. 31 is a depiction of CXCR5 and CXCL13 expression by ovarian cancer tissue relative to nonneoplastic controls.

## DETAILED DESCRIPTION

The following detailed description is presented to enable any person skilled in the art to make and use the invention. For purposes of explanation, specific nomenclature is set forth to provide a thorough understanding of the present invention. However, it will be apparent to one skilled in the art that these specific details are not required to practice the invention. Descriptions of specific applications are provided only as representative examples. The present invention is not intended to be limited to the embodiments shown, but is to be accorded the widest possible scope consistent with the principles and features disclosed herein.

Unless otherwise defined, scientific and technical terms used in connection with the present application shall have the meanings that are commonly understood by those of ordinary skill in the art. Further, unless otherwise required by context, singular terms shall include pluralities and plural terms shall include the singular.

**Definitions**

As used herein, the following terms shall have the following meanings:

The terms "treat," "treating" or "treatment" as used herein, refers to a method of alleviating or abrogating a disorder and/or its attendant symptoms. The terms "prevent", "preventing" or "prevention," as used herein, refer to a method of barring a subject from acquiring a disorder and/or its attendant symptoms. In certain embodiments, the terms "prevent," "preventing" or "prevention" refer to a method of reducing the risk of acquiring a disorder and/or its attendant symptoms.

As used herein, the term "antibody" refers to immunoglobulin molecules and immunologically active portions of immunoglobulin (Ig) molecules, *i.e.,* molecules that contain an antigen binding site that specifically binds (immunoreacts with) an antigen. The term "antibody" is used in the broadest sense and specifically covers monoclonal antibodies (including full length monoclonal antibodies), polyclonal antibodies, multispecific antibodies (*e.g.,* bispecific antibodies), and antibody fragments so long as they exhibit the desired biological activity. By "specifically bind" or "immunoreacts with" is meant that the antibody reacts with one or more antigenic determinants of the desired antigen and does not react (*i.e.,* bind) with other polypeptides or binds at much lower affinity with other polypeptides. The term "antibody" also includes antibody fragments that comprise a portion of a full length antibody, generally the antigen binding or variable region thereof. Examples of antibody fragments include Fab, Fab', F(ab')2, and Fv fragments; diabodies; linear antibodies; single-chain antibody (scFv) molecules; and multispecific antibodies formed from antibody fragments. In certain embodiments of the invention, it may be desirable to use an antibody fragment, rather than an intact antibody, to increase tumor penetration, for example. In this case, it may be desirable to use an antibody fragment that has been modified by any means known in the art in order to increase its serum half life.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al., Proc. Natl. Acad. Sci. USA 81:6851-6855 (1984)).

"Humanized" forms of non-human antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and/or capacity. Methods for making humanized and other chimeric antibodies are known in the art.

"Bispecific antibodies" are antibodies that have binding specificities for at least two different antigens. In the present case, one of the binding specificities is for CXCL16 or CXCR6. The second binding target is any other antigen, and advantageously is a cell-surface protein or receptor or receptor subunit. Methods for making bispecific antibodies are known in the art.

The use of "heteroconjugate antibodies" is also within the scope of the present invention. Heteroconjugate antibodies are composed of two covalently joined antibodies. Such antibodies have, for example, been proposed to target immune system cells to unwanted cells (U.S. Pat. No. 4,676,980). It is contemplated that the antibodies can be prepared in vitro using known methods in synthetic protein chemistry, including those involving crosslinking agents.

The use of "heteroconjugate antibodies" is also within the scope of the present invention. Heteroconjugate antibodies are composed of two covalently joined antibodies. Such antibodies have, for example, been proposed to target immune system cells to unwanted cells (U.S. Pat. No. 4,676,980). It is contemplated that the antibodies can be prepared *in vitro* using known methods in synthetic protein chemistry, including those involving crosslinking agents.

The present invention also contemplates the use of "immunoconjugates" comprising an antibody conjugated to a cytotoxic agent such as a toxin (*e.g.,* an enzymatically active toxin of bacterial, fungal, plant, or animal origin, or fragments thereof), or a radioactive isotope (*i.e.,* a radioconjugate). Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include 212Bi, 131I, 131In, 90Y, and 186Re.

In a pharmacological sense, in the context of the present invention, a "therapeutically effective amount" of an antibody refers to an amount effective in the prevention or treatment of a disorder for the treatment of which the antibody is effective. A "disorder" is any condition that would benefit from treatment with the antibody, including carcinoma and chemoresistance. This includes chronic and acute disorders or diseases including those pathological conditions which predispose the mammal to the disorder in question.

The term "tumor" as used herein refers to a neoplasm or a solid lesion formed by an abnormal growth of cells. A tumor can be benign, pre-malignant or malignant.

A "primary tumor" is a tumor appearing at a first site within the subject and can be distinguished from a "metastatic tumor" which appears in the body of the subject at a remote site from the primary tumor.

The term "cancer," as used herein, refer to or describe the physiological condition in mammals that is typically characterized by unregulated cell growth. Exemplary cancers include: carcinoma, melanoma, sarcoma, lymphoma, leukemia, germ cell tumor, and blastoma.. More particular examples of such cancers include squamous cell cancer (*e.g.,* epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, as well as head and neck cancer, and associated metastases.

The term "carcinoma" as used herein refers to an invasive malignant tumor consisting of transformed epithelial cells or transformed cells of unknown histogenesis, but which possess specific molecular or histological characteristics that are associated with epithelial cells, such as the production of cytokeratins or intercellular bridges. Exemplary carcinomas of the present application include ovarian cancer, vaginal cancer, cervical cancer, uterine cancer, prostate cancer, anal cancer, rectal cancer, colon cancer, stomach cancer, pancreatic cancer, insulinoma, adenocarcinoma, adenosquamous carcinoma, neuroendocrine tumor, breast cancer, lung cancer, esophageal cancer, oral cancer, brain cancer, medulloblastoma, neuroectodermal tumor, glioma, pituitary cancer, and bone cancer.

The term "lymphoma" as used herein is a cancer of lymphatic cells of the immune system. Lymphomas typically present as a solid tumor. Exemplary lymphomas include: small lymphocytic lymphoma, lymphoplasmacytic lymphoma, Waldenström macroglobulinemia, splenic marginal zone lymphoma, plasmacytoma, extranodal marginal zone B cell lymphoma, MALT lymphoma, nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma, B cell chronic lymphocytic lymphoma, classical Hodgkin lymphoma, nodular lymphocyte-predominant Hodgkin lymphoma, adult T cell lymphoma, nasal type extranodal NK/T cell lymphoma, enteropathy-type T cell lymphoma, hepatosplenic T cell lymphoma, blastic NK cell lymphoma, mycosis fungoide, Sezary syndrome, primary cutaneous CD30-positive T cell lympho-proliferative disorders, primary cutaneous anaplastic large cell lymphoma, lymphomatoid papulosis, angioimmunoblastic T cell lymphoma, unspecified peripheral T cell lymphoma, and anaplastic large cell lymphoma. Exemplary forms of classical Hodgkin lymphoma including: nodular sclerosis, mixed cellularity , lymphocyte-rich, and lymphocyte-depleted or not depleted.

The term "sarcoma" as used herein is a cancer that arises from transformed cells in one of a number of tissues that develop from embryonic mesoderm. Thus, sarcomas include tumors of bone, cartilage, fat, muscle, vascular, and hematopoietic tissues. For example, osteosarcoma arises from bone, chondrosarcoma arises from cartilage, liposarcoma arises from fat, and leiomyosarcoma arises from smooth muscle. Exemplary sarcomas include: Askin's tumor, botryodies, chondrosarcoma, Ewing's-PNET, malignant Hemangioendothelioma, malignant Schwannoma, osteosarcoma, soft tissue sarcomas. Subclases of soft tissue sarcomas include: alveolar soft part sarcoma, angiosarcoma, cystosarcoma phyllodes, dermatofibrosarcomadesmoid tumor, desmoplastic small round cell tumor, epithelioid sarcomaextraskeletal chondrosarcoma, extraskeletal osteosarcoma, fibrosarcoma, hemangiopericytoma, hemangiosarcoma, Kaposi's sarcoma, leiomyosarcoma, liposarcoma, lymphangiosarcomal, lymphosarcoma, malignant fibrous histiocytoma, neurofibrosarcoma, rhabdomyosarcoma, and synovial sarcoma.

The term "leukemia" as used herein is a cancer of the blood or bone marrow characterized by an abnormal increase of white blood cells. Leukemia is a broad term covering a spectrum of diseases. In turn, it is part of the even broader group of diseases called hematological neoplasms. Leukemia is subdivided into a variety of large groups; the first division is between acute and chronic forms of leukemia. Acute leukemia is characterized by a rapid increase in the numbers of immature blood cells. Crowding due to such cells makes the bone marrow unable to produce healthy blood cells. Chronic leukemia is characterized by the excessive build up of relatively mature, but still abnormal, white blood cells. Typically taking months or years to progress, the cells are produced at a much higher rate than normal cells, resulting in many abnormal white blood cells in the blood. Leukemia is also subdivided by the blood cells affected. This split divides leukemias into lymphoblastic or lymphocytic leukemias and myeloid or myelogenous leukemias. In lymphoblastic or lymphocytic leukemias, the cancerous change takes place in a type of marrow cell that normally goes on to form lymphocytes. In myeloid or myelogenous leukemias, the cancerous change takes place in a type of marrow cell that normally goes on to form red blood cells, some other types of white cells, and platelets. Combining these two classifications provides a total of four main categories. Within each of these four main categories, there are typically several subcategories. There are also rare types outside of this classification scheme. Exemplary leukemias include: acute lymphoblastic leukemia (ALL), chronic lymphocytic leukemia (CLL), acute myelogenous leukemia (AML), chronic myelogenous leukemia (CML), hairy cell leukemia (HCL), T-cell prolymphocytic leukemia, large granular lymphocytic leukemia, juvenile myelomonocytic leukemia, B-cell prolymphocytic leukemia, Burkitt leukemia, and adult T-cell leukemia.

The term "melanoma" as used herein is a cancer or malignant tumor of melanocytes. Melanocytes are cells that produce the dark pigment, melanin, which is responsible for the color of skin. They predominantly occur in skin, but are also found in other parts of the body, including the bowel and the eye. Melanoma is divided into the following stereotypes and subtypes: lentigo maligna, lentigo maligna melanoma, superficial spreading melanoma, acral lentiginous melanoma, mucosal melanoma, nodular melanoma, polypoid melanoma, desmoplastic melanoma, amelanotic melanoma, soft-tissue melanoma, melanoma with small nevus-like cells, melanoma with features of a Spitz nevus, and uveal melanoma.

The term "metastasis" as used herein refers to the spread of a cancer or carcinoma from one organ or part to another non-adjacent organ or part.

The term "inhibits" is a relative term, an agent inhibits a response or condition if the response or condition is quantitatively diminished following administration of the agent, or if it is diminished following administration of the agent, as compared to a reference agent. Similarly, the term "prevents" does not necessarily mean that an agent completely eliminates the response or condition, so long as at least one characteristic of the response or condition is eliminated. Thus, a composition that reduces or prevents an infection or a response, such as a pathological response, can, but does not necessarily completely eliminate such an infection or response, so long as the infection or response is measurably diminished, for example, by at least about 50%, such as by at least about 70%, or about 80%, or even by about 90% of (that is to 10% or less than) the infection or response in the absence of the agent, or in comparison to a reference agent.

The term "CXCL13 immunogen" and "CXCR5 immunogen" refers to an immunogenic composition comprising (1) an immunogenic peptide derived from CXCL13 or CXCR5 and/or (2) an expression vector that encodes, and is capable of expressing, an immunogenic peptide derived from CXCL13 or CXCR5. The immunogenic peptide derived from CXCL13 or CXCR5 may be fused to another moiety to enhance its immunogenicity. Examples of the CXCL13 immunogenic peptides include, but are not limited to, peptides consisting of, or comprising, one or more sequences selected from the group consisting of RSSSTLPVPVFKRKIP (SEQ ID NO:45), PRGNGCPRKEIIVWKK (SEQ ID NO:46), LPRGNGCPRKEIIVWK (SEQ ID NO:47), QILPRGNGCPRKEIIV (SEQ ID NO:48), ILPRGNGCPRKEIIVW (SEQ ID NO:49), RIQILPRGNGCPRKEI (SEQ ID NO:50), RGNGCPRKEIIVWKKN (SEQ ID NO:51), KRSSSTLPVPVFKRKI (SEQ ID NO:52), IQILPRGNGCPRKEII (SEQ ID NO:53), DRIQILPRGNGCPRKE (SEQ ID NO:54), RKRSSSTLPVPVFKRK (SEQ ID NO:55), RCRCVQESSVFIPRRF (SEQ ID NO:56), GNGCPRKEIIVWKKNK (SEQ ID NO:57), CVQESSVFIPRRFIDR (SEQ ID NO:58), IDRIQILPRGNGCPRK (SEQ ID NO:59), LRCRCVQESSVFIPRR (SEQ ID NO:60), FIDRIQILPRGNGCPR (SEQ ID NO:61), RCVQESSVFIPRRFID (SEQ ID NO:62), CRCVQESSVFIPRRFI (SEQ ID NO:63), QESSVFIPRRFIDRIQ (SEQ ID NO:64), RFIDRIQILPRGNGCP (SEQ ID NO:65), VQESSVFIPRRFIDRI (SEQ ID NO:66), ESSVFIPRRFIDRIQI (SEQ ID NO:67), SLRCRCVQESSVFIPR (SEQ ID NO:68), NGCPRKEIIVWKKNKS (SEQ ID NO:69), PQAEWIQRMMEVLRKR (SEQ ID NO:70), RRFIDRIQILPRGNGC (SEQ ID NO:71), LRKRSSSTLPVPVFKR (SEQ ID NO:72), VQESSVFIPRR (SEQ ID NO:73, EWIQRMMEVLRKRSSSTLPVPVFKRK (SEQ ID NO:74), KKNK (SEQ ID NO:75), RKRSSS (SEQ ID NO:76), RGNGCP (SEQ ID NO:77), VYYTSLRCRCVQESSVFIPRR (SEQ ID NO:78), DRIQILP (SEQ ID NO:79), RKEIIVW (SEQ ID NO:80) and KSIVCVDPQ (SEQ ID NO:81). Examples of the CXCR5 immunogenic peptides include, but are not limited to, peptides consisting of, or comprising, one or more sequences selected from the group consisting of TSLVENHLCPATE (SEQ ID NO:82), EGSVGWVLGTFLCKT (SEQ ID NO:83), LPRCTFS (SEQ ID NO:84), LARLKAVDNT (SEQ ID NO:85) and MASFKAVFVP (SEQ ID NO:86).

The term "CXCL16 immunogen" and "CXCR6 immunogen" refers to an immunogenic composition comprising (1) an immunogenic peptide derived from CXCL16 or CXCR6 and/or (2) an expression vector that encodes, and is capable of expressing, an immunogenic peptide derived from CXCL16 or CXCR6. The immunogenic peptide derived from CXCL16 or CXCR6 may be in the form of a fusion protein to enhance its immunogenicity. Examples of the CXCL16 immunogenic peptides include, but are not limited to, peptides consisting of, or comprising, one or more sequences selected from the group consisting of AAGPEAGENQKQPEKN (SEQ ID NO:87), SQASEGASSDIHTPAQ (SEQ ID NO:88), STLQSTQRPTLPVGSL (SEQ ID NO:89), SWSVCGGNKDPWVQEL (SEQ ID NO:90), GPTARTSATVPVLCLL (SEQ ID NO:91), SGIVAHQKHLLPTSPP (SEQ ID NO:92), RLRKHL (SEQ ID NO:93), LQSTQRP (SEQ ID NO:94), SSDKELTRPNETT (SEQ ID NO:95), AGENQKQPEKNA (SEQ ID NO:96), NEGSVT (SEQ ID NO:97), ISSDSPPSV (SEQ ID NO:98), CGGNKDPW (SEQ ID NO:99), LLPTSPPISQASEGASSDIHT (SEQ ID NO:100), STQRPTLPVGSLSSDKELTRPNETTIHT (SEQ ID NO:101), SLAAGPEAGENQKQPEKNAGPTARTSA (SEQ ID NO:102), TGSCYCGKR (SEQ ID NO:103), DSPPSVQ (SEQ ID NO:104), RKHLRAYHRCLYYTRFQLLSWSVCGG (SEQ ID NO:105), WVQELMSCLDLKECGHAYSGIVAHQKHLLPTSPPISQ (SEQ ID NO:106), SDIHTPAQMLLSTLQ (SEQ ID NO:107), RPTLPVGSL (SEQ ID NO:108), TAGHSLAAG (SEQ ID NO:109), GKRISSDSPPSVQ (SEQ ID NO:110), KDPWVQELMSCLDLKECGHAYSGIVAHQKH (SEQ ID NO:111). Examples of the CXCR6 immunogenic peptides include, but are not limited to, peptides consisting of, or comprising, one or more sequences selected from the group consisting of HQDFLQFSKV (SEQ ID NO:112), AGIHEWVFGQVMCK (SEQ ID NO:113), PQIIYGNVFNLDKLICGYHDEAI (SEQ ID NO:114) and YYAMTSFHYTIMVTEA (SEQ ID NO:115).

The term "biological sample" refers to a sample of biological material obtained from a mammal subject, preferably a human subject, including a tissue, a tissue sample, a cell sample, a tumor sample, a stool sample, and a biological fluid, *e.g.,* blood, plasma, serum, saliva, urine, cerebral or spinal fluid, lymph liquid and a nipple aspirate. A biological sample may be obtained in the form of, *e.g.,* a tissue biopsy, such as, an aspiration biopsy, a brush biopsy, a surface biopsy, a needle biopsy, a punch biopsy, an excision biopsy, an open biopsy, an incision biopsy and an endoscopic biopsy. In one embodiment, the biological sample is a blood, serum or plasma sample. In another embodiment, the biological sample is a saliva sample. In yet another embodiment, the biological sample is a urine sample.

An "isolate" of a biological sample (*e.g.,* an isolate of a tissue or tumor sample) refers to a material or composition (*e.g.,* a biological material or composition) which has been separated, derived, extracted, purified or isolated from the sample and preferably is substantially free of undesirable compositions and/or impurities or contaminants associated with the biological sample.

A "tissue sample" includes a portion, piece, part, segment, or fraction of a tissue which is obtained or removed from an intact tissue of a subject, preferably a human subject.

A "tumor sample" includes to a portion, piece, part, segment, or fraction of a tumor, for example, a tumor which is obtained or removed from a subject (*e.g.,* removed or extracted from a tissue of a subject), preferably a human subject. A tumor sample may be obtained from a primary tumor or a metastatic tumor.

The term "mammal" for purposes of treatment refers to any animal classified as a mammal, including humans, non-human primates, domestic and farm animals, and zoo, sports, or pet animals, such as dogs, horses, cats, cows, etc. Preferably, the mammal is human.

The term "increased level" refers to a level that is higher than a normal or control level customarily defined or used in the relevant art. For example, an increased level of immunostaining in a tissue is a level of immunostaining that would be considered higher than the level of immunostaining in a control tissue by a person of ordinary skill in the art.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed.

## METHOD FOR DETECTING CANCER BY MEASURING CXCL13 AND/OR CXCR5 EXPRESSION OR ACTIVITY

CXCL13, also known as B lymphocyte chemoattractant (BLC), is a ligand for the CXCR5 chemokine receptor. Both the chemokine and the receptor appear to play a role in the regulation of metastasis and invasion of cancer. Both CXCL13 and CXCR5 are locally up-regulated in multiple carcinoma tissue types compared to normal tissues, including ovarian, lung, breast, prostate, bone and pancreatic cancers. CXCL13 levels are also increased in the serum of patients with those cancers. Additionally, soluble CXCL13 chemokine enhances both in vivo and in vitro proliferation and migration of cancer cells.

CXCR5 (CD185), also known as Burkett lymphoma receptor 1 (BLR1), is a member of the chemokine receptor family of G protein coupled receptors (GPCRs) that may have a diverse role in cancer cell survival that presumably supports protection against chemotherapeutic drugs. Interaction of CXCR5 with CXCL13 activates DOCK2 (Dedicator of cytokenesis 2), which binds to the DOCK-binding protein ELMO1 (Engulfment and cell motility protein 1), allowing DOCK2-mediated Rac (Rac-related C3 botulinum toxin substrate proteins, a family of signaling G proteins that is a subfamily of the Rho family of GTPases) activation in lymphocytes. DOCK2 binds both the Rac1 and Rac2 isoforms and DOCK2-dependent Rac activation regulates neutrophil NADPH oxidase and is important for chemotaxis in neutrophils. In the present application, the term "CXCR5" is inclusive of the transcription variants of CXCR5, such as CXCR5a (CXCR5 transcript variant 2) and CXCR5b (CXCR5 transcript variant 1).

One aspect of the present application relates to methods for detecting the presence of a cancer in a subject. In one embodiment, the method comprises detecting the level of expression of one or more cancer markers in a biological sample obtained from the subject, and comparing the level of expression of one or more cancer markers in the biological sample to a normal level of expression of the one or more cancer markers, wherein a higher than normal level of expression of the one or more cancer markers in the biological sample is indicative of the presence of cancer in the subject, wherein the normal level of expression of the one or more cancer markers is a predetermined value or is obtained from a control sample of known normal non-cancerous cells of the same origin or type as the biological sample, wherein the one or more cancer markers include CXCL13 or CXCR5 or both CXCL13 and CXCR5.

In another embodiment, the one or more cancer markers include (1) CXCL13 or CXCR5 or both CXCL13 and CXCR5, and (2) CXCL16 or CXCR6 or both CXCL16 and CXCR6.

In another embodiment, the one or more cancer markers include (1) CXCL13 or CXCR5 or both CXCL13 and CXCR5, (2) CXCL16 or CXCR6 or both CXCL16 and CXCR6, and (3) one or more other cancer markers.

In the context of the present application, the term "detecting" is intended to encompass predictions and likelihood analysis. The present method is intended to be used clinically in making decisions concerning treatment modalities, including therapeutic intervention, diagnostic criteria such as disease stages, and disease monitoring and surveillance for cancer. According to the present application, an intermediate result for examining the condition of a subject may be provided. Such intermediate result may be combined with additional information to assist a doctor, nurse, or other practitioner to diagnose that a subject suffers from the disease. Alternatively, the present application may be used to detect cancerous cells in a subject-derived tissue, and provide a doctor with useful information to diagnose that the subject suffers from the disease. The subject to be diagnosed by the present method is preferably a human, but may also include other mammals such as non-human primate, mouse, rat, dog, cat, horse, and cow.

The cancer may be blastoma, carcinoma, leukemia, lymphoma, melanoma, myeloma or sarcoma. In some other embodiments, the biological sample is a plasma sample, a saliva sample or a urine sample.

## METHOD FOR PREDICTING THE PROGNOSIS OF A SUBJECT HAVING CANCER

The present method for diagnosing cancer may also be applied for assessing the prognosis of a patient with the cancer by comparing the expression level of one or more cancer markers in a patient-derived biological sample with that of a reference sample. In one embodiment, the method comprises determining the expression level of one or more cancer markers in a biological sample from the patient, wherein a higher level of expression of the one or more cancer markers in the biological sample relative to a control value, *e.g.*, level in a control, indicates that the prognosis of the subject is poor, whereas a lower or similar level of expression of the one or more cancer markers in the biological sample relative to that in the control indicates that the prognosis of the subject is good. A poor prognosis indicates that the cancer is of an aggressive or invasive type, likely to progress fast and/or likely to metastasize, wherein the one or more cancer markers includes CXCL13 or CXCR5 or both CXCL13 and CXCR5.

In another embodiment, the one or more cancer markers include (1) CXCL13 or CXCR5 or both CXCL13 and CXCR5, and (2) CXCL16 or CXCR6 or both CXCL16 and CXCR6.

In another embodiment, the one or more cancer markers include (1) CXCL13 or CXCR5 or both CXCL13 and CXCR5, (2) CXCL16 or CXCR6 or both CXCL16 and CXCR6, and (3) one or more other cancer markers.

Alternatively, the level of one or more cancer markers in the biological sample may be measured over a spectrum of disease stages to assess the prognosis of the patient. An increase in the expression level of one or more cancer markers as compared to a normal control level indicates less favorable prognosis. A similarity in the expression level of one or more cancer markers as compared to a normal control level indicates a more favorable prognosis of the patient.

The cancer may be blastoma, carcinoma, leukemia, lymphoma, melanoma, myeloma or sarcoma. In some other embodiments, the biological sample is a plasma sample, a saliva sample or a urine sample.

## METHOD FOR MONITORING THE COURSE OF CANCER TREATMENT

In certain embodiments, the level(s) of one or more cancer markers is used to monitor the course of treatment of cancer. In this method, a test biological sample is provided from a subject undergoing treatment for cancer. Preferably, multiple test biological samples are obtained from the subject at various time points before, during or after the treatment. The expression level of the cancer marker in the post-treatment sample may then be compared with the level of the cancer marker in the pre-treatment sample or, alternatively, with a reference sample (*e.g.,* a normal control level). For example, if the post-treatment marker level is lower than the pre-treatment marker level, one can conclude that the treatment was efficacious. Likewise, if the post-treatment marker level is similar to, or the same as, the normal control marker level, one can also conclude that the treatment was efficacious.

An "efficacious" treatment is one that leads to a reduction in the level of a cancer marker or a decrease in size, prevalence or metastatic potential of cancer in a subject. When a treatment is applied prophylactically, "efficacious" means that the treatment retards or prevents occurrence of cancer or alleviates a clinical symptom of cancer. The assessment of cancer can be made using standard clinical protocols. Furthermore, the efficaciousness of a treatment can be determined in association with any known method for diagnosing or treating cancer. For example, cancer is routinely diagnosed histopathologically or by identifying symptomatic anomalies such as weight loss and loss of appetite.

In one embodiment, the cancer marker level in the biological sample is compared with an cancer marker level associated with a reference sample, such as a normal control sample. The phrase "normal control level" refers to the level of a cancer marker typically found in a biological sample of a population not suffering from cancer. The reference sample is preferably of a similar nature to that of the test sample. For example, if the test sample comprises patient serum, the reference sample should also be serum. The cancer marker level in the biological samples from control and test subjects may be determined at the same time or, alternatively, the normal control level may be determined by a statistical method based on the results obtained by analyzing the level of the cancer marker in samples previously collected from a control group.

The cancer may be blastoma, carcinoma, leukemia, lymphoma, melanoma, myeloma or sarcoma. In some other embodiments, the biological sample is a plasma sample, a saliva sample or a urine sample.

**CANCER MARKERS**

The term "cancer marker" as used herein, refers to or describes a polypeptide or a polynuceotide whose expression level, alone or in combination with other polypeptides or a polynuceotides, is correlated with cancer or prognosis of cancer. The correlation may relate to either an increased or decreased expression of the polypeptide or a polynuceotide. For example, the expression of the polypeptide or a polynuceotide may be indicative of cancer, or lack of expression of the polypeptide or a polynuceotide may be correlated with poor prognosis in a cancer patient.

The term "expression level of a cancer marker" may be measured at the transcription level, in which case the presence and/or the amount of a polynucleotide is determined, or at the translation level, in which case the presence and/or the amount of a polypeptide is determined. Cancer marker expression may be characterized using any suitable method.

Examples of the cancer marker include CXCL13, CXCR5, other chemokines and chemokine receptors such as CXCL1, CXCL2, CXCL3, CXCL4, CXCL6, CXCL7, CXCL8, CXCL9, CXCL10, CXCL11, CXCL12, CXCL14, CXCL15, CXCL16, CXCR1, CXCR2, CXCR3, CXCR4, CXCR6, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, CX3CL1, RNA binding motif 3 ("RBM3"), carcinoembryonic Antigen (CEA), prostate specific antigen (PSA), chromgranin A (CGA), dehydroepiandrosterone (DHEA), neuron-specific enolase (NSE), prostatic acid phosphatase (PAP), prolactin, B7-H3, seprase polypeptide, anti-p53, osteopontin, ferritin, lysophosphatidyl choline, kinesin family member 4A (KIF4A), Neural pentraxin I (NPTX1) and fibroblast growth factor receptor 1 oncogene partner (FGFR1OP) protein.

In one embodiment, the cancer markers described above are selected from a melanoma marker panel that includes CXCL13, CXCR5, CCL25, CCL27, CXCL1, CXCL2, CXCL3, CXCL5, CXCL6, CXCL7, CXCL8, CXCL12, CXCL16, CX3CL1, CCR9, CCR10, CXCR1, CXCR2, CXCR4, CXCR6 and CX3CR1. The markers in the melanoma panel may be used for detecting melanoma or predicting the prognosis of a subject having melanoma.

In one embodiment, the cancer markers described above are selected from a carcinoma marker panel that includes CXCL13, CXCR5, CCL1, CCL4, CCL17, CCL19, CCL21, CCL22, CCL25, CXCL12, CXCL16, CCR7, CCR8, CCR9, CXCR4, CXCR6 and CX3CR1. The markers in the carcinoma panel may be used for detecting carcinoma or predicting the prognosis of a subject having carcinoma.

In another embodiment, the cancer markers described above are selected from a breast cancer marker panel that includes CXCL13, CXCR5, CCL1, CCL4, CCL17, CCL19, CCL21, CCL22, CCL25, CXCL12, CXCL16, CCR7, CCR8, CCR9, CXCR4, CXCR6, CX3CR1, RNA binding motif 3 ("RBM3") and carcinoembryonic Antigen (CEA). The markers in the breast cancer panel may be used for detecting breast cancer or predicting the prognosis of a subject having breast cancer.

In another embodiment, the cancer markers described above are selected from a prostate cancer marker panel that includes CXCL13, CXCR5, CXCL16, CXCR6, CCL1, CCL4, CCL17, CCL19, CCL21, CCL22, CCL25, CXCL12, CCR7, CCR8, CCR9, CXCR4, CX3CR1, PSA, CEA, CGA, DHEA, NSE, PAP, prolactin and B7-H3. The markers in the breast cancer panel may be used for detecting prostate cancer or predicting the prognosis of a subject having prostate cancer.

In another embodiment, the one or more cancer markers described above are selected from a colonrectal cancer marker panel that includes CXCL13, CXCR5, CXCL16, CXCR6, CCL1, CCL4, CCL17, CCL19, CCL21, CCL22, CCL25, CXCL12, CCR7, CCR8, CCR9, CXCR4, CX3CR1, seprase polypeptide, anti-p53, osteopontin, and ferritin. The markers in the colonrectal cancer panel may be used for detecting colonrectal cancer or predicting the prognosis of a subject having colonrectal cancer.

In another embodiment, the cancer markers described above are selected from an ovarian cancer marker panel that includes CXCL13, CXCR5, CXCL16, CXCR6, CCL1, CCL4, CCL17, CCL19, CCL21, CCL22, CCL25, CXCL12, CCR7, CCR8, CCR9, CXCR4, CX3CR1, cancer antigen 125 (CA-125), HE-4, OVX-1 macrophage colony stimulating factor (M-CSF) and lysophosphatidyl choline. The markers in the ovarian cancer panel may be used for detecting ovarian cancer or predicting the prognosis of a subject having ovarian cancer.

In another embodiment, the cancer markers described above are selected from a lung cancer marker panel that includes CXCL13, CXCR5, CXCL16, CXCR6, CCL1, CCL4, CCL17, CCL19, CCL21, CCL22, CCL25, CXCL12, CCR7, CCR8, CCR9, CXCR4, CX3CR1, kinesin family member 4A (KIF4A), Neural pentraxin I (NPTX1), fibroblast growth factor receptor 1 oncogene partner (FGFR1OP) protein and CEA. The markers in the lung cancer panel may be used for detectsing lung cancer or predicting the prognosis of a subject having lung cancer.

In another embodiment, the one or more cancer markers described above are selected from a pancreatic cancer marker panel that includes CXCL13, CXCR5, CXCL16, CXCR6, CCL1, CCL4, CCL17, CCL19, CCL21, CCL22, CCL25, CXCL12, CCR7, CCR8, CCR9, CXCR4, CX3CR1 and CEA. The markers in the pancreatic cancer panel may be used for detecting pancreatic cancer or predicting the prognosis of a subject having pancreatic cancer.

In another embodiment, the one or more cancer markers described above are selected from a gastric cancer marker panel that includes CXCL13, CXCR5, CCL1, CCL4, CCL17, CCL19, CCL21, CCL22, CCL25, CXCL12, CXCL16, CCR7, CCR8, CCR9, CXCR4, CXCR6 and CX3CR1 and CEA. The markers in the gastric cancer panel may be used for detecting gastric cancer or predicting the prognosis of a subject having gastric cancer.

## DETECTION METHODS

The expression of the cancer marker(s) can be determined at the transcription level (*i.e.,* the amount of mRNA) or the translation level (*i.e.,* the amount of protein). In certain embodiments, expression of the cancer marker(s) is determined at the mRNA level by quantitative RT-PCR, Northern blot or other methods known to a person of ordinary skill in the art. In other embodiments, the expression of the cancer marker(s) is determined at the protein level by ELISA, Western blot or other types of immnuo-detection methods using anti-cancer marker antibodies, such as anti-CXCL13 and anti-CXCR5 antibodies.

In certain embodiments, the anti-CXCL13 and/or anti-CXCR5 antibodies include antibodies that bind specifically to a CXCL13 peptide or a CXCR5 peptide. Examples of the CXCL13 peptides include, but are not limited to, peptides consisting of, or comprising, one or more sequences selected from the group consisting of RSSSTLPVPVFKRKIP (SEQ ID NO:45), PRGNGCPRKEIIVWKK (SEQ ID NO:46), LPRGNGCPRKEIIVWK (SEQ ID NO:47), QILPRGNGCPRKEIIV (SEQ ID NO:48), ILPRGNGCPRKEIIVW (SEQ ID NO:49), RIQILPRGNGCPRKEI (SEQ ID NO:50), RGNGCPRKEIIVWKKN (SEQ ID NO:51), KRSSSTLPVPVFKRKI (SEQ ID NO:52), IQILPRGNGCPRKEII (SEQ ID NO:53), DRIQILPRGNGCPRKE (SEQ ID NO:54), RKRSSSTLPVPVFKRK (SEQ ID NO:55), RCRCVQESSVFIPRRF (SEQ ID NO:56), GNGCPRKEIIVWKKNK (SEQ ID NO:57), CVQESSVFIPRRFIDR (SEQ ID NO:58), IDRIQILPRGNGCPRK (SEQ ID NO:59), LRCRCVQESSVFIPRR (SEQ ID NO:60), FIDRIQILPRGNGCPR (SEQ ID NO:61), RCVQESSVFIPRRFID (SEQ ID NO:62), CRCVQESSVFIPRRFI (SEQ ID NO:63), QESSVFIPRRFIDRIQ (SEQ ID NO:64), RFIDRIQILPRGNGCP (SEQ ID NO:65), VQESSVFIPRRFIDRI (SEQ ID NO:66), ESSVFIPRRFIDRIQI (SEQ ID NO:67), SLRCRCVQESSVFIPR (SEQ ID NO:68), NGCPRKEIIVWKKNKS (SEQ ID NO:69), PQAEWIQRMMEVLRKR (SEQ ID NO:70), RRFIDRIQILPRGNGC (SEQ ID NO:71), LRKRSSSTLPVPVFKR (SEQ ID NO:72), VQESSVFIPRR (SEQ ID NO:73, EWIQRMMEVLRKRSSSTLPVPVFKRK (SEQ ID NO:74), KKNK (SEQ ID NO:75), RKRSSS (SEQ ID NO:76), RGNGCP (SEQ ID NO:77), VYYTSLRCRCVQESSVFIPRR (SEQ ID NO:78), DRIQILP (SEQ ID NO:79), RKEIIVW (SEQ ID NO:80) and KSIVCVDPQ (SEQ ID NO:81). Examples of the CXCR5 peptides include, but are not limited to, peptides consisting of, or comprising, one or more sequences selected from the group consisting of TSLVENHLCPATE (SEQ ID NO:82), EGSVGWVLGTFLCKT (SEQ ID NO:83), LPRCTFS (SEQ ID NO:84), LARLKAVDNT (SEQ ID NO:85) and MASFKAVFVP (SEQ ID NO:86).

In other embodiments, the anti-CXCL13 and/or anti-CXCR5 antibody is used in conjunction with an anti-CXCL16 antibody and/or anti-CXCR6 antibody. The anti-CXCL16 and/or anti-CXCR6 antibodies include antibodies that bind specifically to a CXCL16 peptide or a CXCR6 peptide. Examples of the CXCL16 peptides include, but are not limited to, peptides consisting of, or comprising, one or more sequences selected from the group consisting of AAGPEAGENQKQPEKN (SEQ ID NO:87), SQASEGASSDIHTPAQ (SEQ ID NO:88), STLQSTQRPTLPVGSL (SEQ ID NO:89), SWSVCGGNKDPWVQEL (SEQ ID NO:90), GPTARTSATVPVLCLL (SEQ ID NO:91), SGIVAHQKHLLPTSPP (SEQ ID NO:92), RLRKHL (SEQ ID NO:93), LQSTQRP (SEQ ID NO:94), SSDKELTRPNETT (SEQ ID NO:95), AGENQKQPEKNA (SEQ ID NO:96), NEGSVT (SEQ ID NO:97), ISSDSPPSV (SEQ ID NO:98), CGGNKDPW (SEQ ID NO:99), LLPTSPPISQASEGASSDIHT (SEQ ID NO:100), STQRPTLPVGSLSSDKELTRPNETTIHT (SEQ ID NO:101), SLAAGPEAGENQKQPEKNAGPTARTSA (SEQ ID NO:102), TGSCYCGKR (SEQ ID NO:103), DSPPSVQ (SEQ ID NO:104), RKHLRAYHRCLYYTRFQLLSWSVCGG (SEQ ID NO:105), WVQELMSCLDLKECGHAYSGIVAHQKHLLPTSPPISQ (SEQ ID NO:106), SDIHTPAQMLLSTLQ (SEQ ID NO:107), RPTLPVGSL (SEQ ID NO:108), TAGHSLAAG (SEQ ID NO:109), GKRISSDSPPSVQ (SEQ ID NO:110), KDPWVQELMSCLDLKECGHAYSGIVAHQKH (SEQ ID NO:111). Examples of the CXCR6 peptides include, but are not limited to, peptides consisting of, or comprising, one or more sequences selected from the group consisting of HQDFLQFSKV (SEQ ID NO:112), AGIHEWVFGQVMCK (SEQ ID NO:113), PQIIYGNVFNLDKLICGYHDEAI (SEQ ID NO:114) and YYAMTSFHYTIMVTEA (SEQ ID NO:115).

In one embodiment, the antibody is conjugated to a solid support. By "solid support" is meant a non-aqueous matrix to which an antibody of the present application can adhere or attach. Examples of solid phases encompassed herein include those formed partially or entirely of glass (*e.g.,* controlled pore glass), polysaccharides (*e.g.,* agarose), polyacrylamides, silicones, and plastics such as polystyrene, polypropylene and polyvinyl alcohol.

**Enzyme-linked Immunosorbent Assay (ELISA)**

In certain embodiments, the cancer markers are detected using enzyme-linked immunosorbent assay (ELISA) which is typically carried out using antibody coated assay plate or wells. Commonly used ELISA assay employs either a sandwich immunoassay or a competitive binding immunoassay.

Briefly, a sandwich immunoassay is a method using two antibodies, which bind to different sites on the antigen or ligand. The primary antibody, which is highly specific for the antigen, is attached to a solid surface. The antigen is then added followed by addition of a second antibody referred to as the detection antibody. The detection antibody binds the antigen to a different epitope than the primary antibody. As a result the antigen is 'sandwiched' between the two antibodies. The antibody binding affinity for the antigen is usually the main determinant of immunoassay sensitivity. As the antigen concentration increases the amount of detection antibody increases leading to a higher measured response. The standard curve of a sandwich-binding assay has a positive slope. To quantify the extent of binding different reporters can be used. Typically an enzyme is attached to the secondary antibody which must be generated in a different species than primary antibodies (*i.e.* if the primary antibody is a rabbit antibody than the secondary antibody would be an anti-rabbit from goat, chicken, etc., but not rabbit). The substrate for the enzyme is added to the reaction that forms a colorimetric readout as the detection signal. The signal generated is proportional to the amount of target antigen present in the sample.

The antibody linked reporter used to measure the binding event determines the detection mode. A spectrophotometric plate reader may be used for colorimetric detection. Several types of reporters have been recently developed in order to increase sensitivity in an immunoassay. For example, chemiluminescent substrates have been developed which further amplify the signal and can be read on a luminescent plate reader. Also, a fluorescent readout where the enzyme step of the assay is replaced with a fluorophor tagged antibody is becoming quite popular. This readout is then measured using a fluorescent plate reader.

A competitive binding assay is based upon the competition of labeled and unlabeled ligand for a limited number of antibody binding sites. Competitive inhibition assays are often used to measure small analytes. These assays are also used when a matched pair of antibodies to the analyte does not exist. Only one antibody is used in a competitive binding ELISA. This is due to the steric hindrance that occurs if two antibodies would attempt to bind to a very small molecule. A fixed amount of labeled ligand (tracer) and a variable amount of unlabeled ligand are incubated with the antibody. According to law of mass action the amount of labeled ligand is a function of the total concentration of labeled and unlabeled ligand. As the concentration of unlabeled ligand is increased, less labeled ligand can bind to the antibody and the measured response decreases. Thus the lower the signal, the more unlabeled analyte there is in the sample. The standard curve of a competitive binding assay has a negative slope.

**Microbeads**

In certain other embodiments, the cancer markers are detected using antibody coated microbeads. In some embodiments, the microbeads are magnetic beads. In other embodiments, the beads are internally color-coded with fluorescent dyes and the surface of the bead is tagged with an anti-cancer marker antibody (*e.g.,* an anti-CXCL13 or anti-CXCR5 antibody) that can bind a cancer marker in a test sample. The cancer marker, in turn, is either directly labeled with a fluorescent tag or indirectly labeled with an anti-marker antibody conjugated to a fluorescent tag. Hence, there are two sources of color, one from the bead and the other from the fluorescent tag. Alternatively, the beads can be internally coded by different sizes.

By using a blend of different fluorescent intensities from the two dyes, as well as beads of different sizes, the assay can measure up to hundreds of different cancer markers. During the assay, a mixture containing the color/size-coded beads, fluorescence labeled anti-marker antibodies, and the sample are combined and injected into an instrument that uses precision fluidics to align the beads. The beads then pass through a laser and, on the basis of their color or size, either get sorted or measured for color intensity, which is processed into quantitative data for each reaction.

When samples are directly labeled with fluorophores, the system can read and quantitate only fluorescence on beads without removing unbound fluorophores in solution. The assays can be multiplexed by differentiating various colored or sized beads. Real time measurement is achievable when a sample is directly required for unlabeled samples. Standard assay steps include incubation of a sample with anti-marker antibody coated beads, incubation with biotin or fluorophore-labeled secondary antibody, and detection of fluorescence signals. Fluorescent signals can be developed on bead (by adding streptavidin-fluorophore conjugates for biotinylated secondary antibody) and read out by a bead analyzer. Depending on the anti-marker immobilized on the bead surface, a bead-based immunoassay can be a sandwich type or a competitive type immunoassay.

**Test Stick**

In some other embodiments, the cancer markers in a liquid biosample are detected using a test stick. The test stick typically contains a fluid impermeable housing and a fluid permeable "stick" having one or more detection zones. In one embodiment, each detection zone contains a dried binding reagent that binds to a cancer marker in a biosample. In another embodiment, the dried binding reagent is a labeled binding reagent. In another embodiment, the test stick may further comprise a control zone to indicate that the assay test has been carried out satisfactorily, namely the reagents were present in the test stick and that they become mobilized during running the test and have been transported along the flow path. The control zone can also indicate that the reagents within the device are capable of immunochemical interactions, confirming the chemical integrity of the device. This is important when considering the storage and shipment of the device under desiccated conditions within a certain temperature range. The control zone is typically positioned downstream from the detection zone(s) and may, for example, comprise an immobilized binding reagent for a labeled binding reagent. The labeled binding reagent may be present in a mobilizable form upstream from the control zone and detection zone. The labeled binding reagent may be the same or different to the labeled binding reagent for the cancer marker.

In one embodiment, the test stick comprise a porous sample receiver in fluid connection with and upstream from one or more flow-paths. The porous sample receiver may be common to all assays. Thus a fluid sample applied to the common sample application region of the device is able to travel along the one or more flow-paths to the respective detection zones. The porous sample receiver may be provided within a housing or may at least partially extend out of said housing and may serve for example to collect a body fluid. The porous sample receiver may also act as a fluid reservoir. The porous sample receiving member can be made from any bibulous, porous or fibrous material capable of absorbing liquid rapidly. The porosity of the material can be unidirectional (*i.e.* with pores or fibers running wholly or predominantly parallel to an axis of the member) or multidirectional (omnidirectional, so that the member has an amorphous sponge-like structure). Porous plastics material, such as polypropylene, polyethylene (preferably of very high molecular weight), polyvinylidene fluoride, ethylene vinylacetate, acrylonitrile and polytetrafluoroethylene can be used. Other suitable materials include glass-fiber.

If desired, an absorbent "sink" can be provided at the distal end of the carrier material. The absorbent sink may comprise, for example, Whatman 3MM chromatography paper, and should provide sufficient absorptive capacity to allow any unbound labeled binding reagent to wash out of the detection zone(s). As an alternative to such a sink it can be sufficient to have a length of porous solid phase material which extends beyond the detection zone(s).

Following the application of a binding reagent to a detection zone, the remainder of the porous solid phase material may be treated to block any remaining binding sites. Blocking can be achieved by treatment for example with protein (*e.g.* bovine serum albumin or milk protein), or with polyvinyl alcohol or ethanolamine, or combinations thereof. To assist the free mobility of the labeled binding reagent when the porous carrier is moistened with the sample, the porous carrier may further comprise a sugar such as sucrose or lactose and/or other substances, such as polyvinyl alcohol (PVA) or polyvinyl pyrrolidone (PVP). Such material may be deposited, for example, as an aqueous solution in the region to which the labeled binding reagent is to be applied. Such materials could be applied to the porous carrier as a first application followed by the application of the label; alternatively, such materials could be mixed with the label and applied to the porous carrier or combinations of both. Such material may be deposited upstream from or at the labeled binding reagent.

Alternatively, the porous carrier may not be blocked at the point of manufacture; instead the means for blocking the porous carrier are included in a material upstream from the porous carrier. On wetting the test strip, the means for blocking the porous carrier are mobilized and the blocking means flow into and through the porous carrier, blocking as the flow progresses. The blocking means include proteins such as BSA and casein as well as polymers such as PVP, PVA as well as sugars and detergents such as Triton-X100. The blocking means could be present in the macroporous carrier material.

The dried binding reagents may be provided on a porous carrier material provided upstream from a porous carrier material comprising the detection zone. The upstream porous carrier material may be macroporous. The macroporous carrier material should be low or non-protein-binding, or should be easily blockable by means of reagents such as BSA or PVA, to minimize non-specific binding and to facilitate free movement of the labeled reagent after the macroporous body has become moistened with the liquid sample. The macroporous carrier material can be pre-treated with a surface active agent or solvent, if necessary, to render it more hydrophilic and to promote rapid uptake of the liquid sample. Suitable materials for a macroporous carrier include plastic materials such as polyethylene and polypropylene, or other materials such as paper or glass-fiber. In the case that the labeled binding reagent is labeled with a detectable particle, the macroporous body may have a pore size at least ten times greater than the maximum particle size of the particle label. Larger pore sizes give better release of the labeled reagent. As an alternative to a macroporous carrier, the labeled binding reagent may be provided on a non-porous substrate provided upstream from the detection zone, said non-porous substrate forming part of the flow-path.

In another embodiment, the test stick may further comprise a sample receiving member for receiving the fluid sample. The sample receiving member may extend from the housing.

The housing may be constructed of a fluid impermeable material. The housing will also desirably exclude ambient light. The housing will be considered to substantially exclude ambient light if less than 10%, preferably less than 5%, and most preferably less than 1%, of the visible light incident upon the exterior of the device penetrates to the interior of the device. A light-impermeable synthetic plastics material such as polycarbonate, ABS, polystyrene, polystyrol, high density polyethylene, or polypropylene containing an appropriate light-blocking pigment is a suitable choice for use in fabrication of the housing. An aperture may be provided on the exterior of the housing which communicates with the assay provided within the interior space within the housing. Alternatively, the aperture may serve to allow a porous sample receiver to extend from the housing to a position external from the housing.

## Microarray

In other embodiments, the cancer markers are detected by a protein microarray containing immobilized cancer marker-specific antibodies on its surface. The microarray can be used in a "sandwich" assay in which the antibody on the microarray captures a cancer marker in the test sample and the captured marker is detected by a labeled secondary antibody that specifically binds to the captured marker. In a preferred embodiment, the secondary antibody is biotinylated or enzyme-labeled. The detection is achieved by subsequent incubation with a streptavidin-fluorophore conjugate (for fluorescence detection) or an enzyme substrate (for colorimetric detection).

Typically, a microarray assay contains multiple incubation steps, including incubation with the samples and incubation with various reagents (*e.g.,* primary antibodies, secondary antibodies, reporting reagents, etc.). Repeated washes are also needed between the incubation steps. In one embodiment, the microarray assays is performed in a fast assay mode that requires only one or two incubations. It is also conceivable that the formation of a detectable immune complex (*e.g.,* a captured cancer marker/anti-marker antibody/label complex) may be achieved in a single incubation step by exposing the protein microarray to a mixture of the sample and all the necessary reagents. In one embodiment, the primary and secondary antibodies are the same antibody.

In another embodiment, the protein microarray provides a competitive immunoassay. Briefly, a microarray comprising immobilized anti-marker antibodies is incubated with a test sample in the presence of a labeled cancer marker standard. The labeled cancer marker competes with the unlabeled cancer marker in the test sample for the binding to the immobilized antigen-specific antibody. In such a competitive setting, an increased concentration of the specific cancer marker in the test sample would lead to a decreased binding of the labeled cancer marker standard to the immobilized antibody and hence a reduced signal intensity from the label.

The microarray can be processed in manual, semi-automatic or automatic modes. Manual mode refers to manual operations for all assay steps including reagent and sample delivery onto microarrays, sample incubation and microarray washing. Semi-automatic modes refer to manual operation for sample and reagent delivery onto microarray, while incubation and washing steps operate automatically. In an automatic mode, three steps (sample/reagent delivery, incubation and washing) can be controlled by a computer or an integrated breadboard unit with a keypad. For example, the microarray can be processed with a ProteinArray Workstation (PerkinElmer Life Sciences, Boston, Mass.) or Assay 1200™. Workstation (Zyomyx, Hayward, Calif.). Scanners by fluorescence, colorimetric and chemiluminescence, can be used to detect microarray signals and capture microarray images. Quantitation of microarray-based assays can also be achieved by other means, such as mass spectrometry and surface plasma resonance. Captured microarray images can be analyzed by stand-alone image analysis software or with image acquisition and analysis software package. For example, quantification of an antigen microarray can be achieved with a fluorescent PMT-based scanner--ScanArray 3000 (General Scanning, Watertown, Mass.) or colorimetric CCD-based scanner--VisionSpot (Allied Biotech, Ijamsville, Md.). Typically, the image analysis would include data acquisition and preparation of assay report with separate software packages. To speed up the whole assay process from capturing an image to generating an assay report, all the analytical steps including image capture, image analysis, and report generation, can be confined in and/or controlled by one software package. Such an unified control system would provide the image analysis and the generation of assay report in a user-friendly manner.

## Implantable Biosensors

In other embodiments, the cancer markers are detected using implantable biosensors. Biosensors are electronic devices that produce electronic signals as the result of biological interactions. In one embodiment, the biosensors use antibodies, receptors, nucleic acids, or other members of a binding pair to bind with a cancer marker, which is typically the other member of the binding pair. Biosensors may be used with a blood sample to determine the presence of a cancer marker without the need for sample preparation and/or separation steps typically required for the automated immunoassay systems.

In one embodiment, the sensor is a nanoscale device. The sensor system includes a biological recognition element attached to a nanowire and a detector that is capable of determining a property associated with the nanowire. The biological recognition element is one member of a binding pair (*e.g.,* a receptor of the cancer marker or an anti-cancer marker antibody) where the cancer marker being measured is the other member of the binding pair. Preferably, the nanowire sensor includes a semiconductor nanowire with an exterior surface formed thereon to form a gate electrode and a first end in electrical contact with a conductor to form a source electrode and a second end in contact with a conductor to form a drain electrode. In one embodiment the sensor is a field effect transistor comprising a substrate formed of an insulating material, a source electrode, a drain electrode and a semiconductor nanowire disposed there between with a biological recognition element attached on a surface of the nanowire. When a binding event occurs between the biological recognition element and its specific binding partner, a detectable change is caused in a current-voltage characteristic of the field effect transistor.

In another embodiment, the sensor system includes an array of sensors. One or more of the sensors in the array is associated with a protective member that prevents the associated sensor from interacting with the surrounding environment. At a selected time, the protective member may be disabled, thereby allowing the sensor to begin operating to interact with the surrounding fluid or tissue so that the biological recognition element can interact with the other member of its binding pair if that pair member is present.

In another embodiment, the protective member is formed of a conductive material that can oxidize, is biocompatible, bio-absorbable, and that may be dissolved in solution such as blood upon application of an electric potential. For example, a sensor may be formed within a well of a substrate that is capped by a conductive material such as a biocompatible metal or an electrically-erodible polymer. In another embodiment, the protective member is formed using a material that dissolves over a predetermined period of time.

Mass Spectrometry

In other embodiments, the cancer markers are detected using mass spectrometry (MS) such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g.,* MS/MS, MS/MS/MS, ESI-MS/MS, *etc*.).

Mass spectrometry methods are well known in the art and have been used to quantify and/or identify biomolecules, such as proteins. Further, mass spectrometric techniques have been developed that permit at least partial de novo sequencing of isolated proteins. In certain embodiments, a gas phase ion spectrophotometer is used. In other embodiments, laser-desorption/ionization mass spectrometry is used to analyze the sample. Modem laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. In SELDI, the substrate surface is modified so that it is an active participant in the desorption process. In one embodiment, the surface is derivatized with adsorbent and/or capture reagents that selectively bind the protein of interest. In another embodiment, the surface is derivatized with energy absorbing molecules that are not desorbed when struck with the laser. In another embodiment, the surface is derivatized with molecules that bind the protein of interest and that contain a photolytic bond that is broken upon application of the laser. In each of these methods, the derivatizing agent generally is localized to a specific location on the substrate surface where the sample is applied. See, *e.g.,* U.S. Pat. No. 5,719,060 (Hutchens & Yip) and WO 98/59361 (Hutchens & Yip). The two methods can be combined by, for example, using a SELDI affinity surface to capture an analyte and adding matrix-containing liquid to the captured analyte to provide the energy absorbing material.

Detection of the presence of a cancer marker will typically involve detection of signal intensity. This, in turn, can reflect the quantity and character of a polypeptide bound to the substrate. For example, in certain embodiments, the signal strength of peak values from spectra of a first sample and a second sample can be compared (e.g., visually, by computer analysis etc.), to determine the relative amounts of particular biomolecules. Software programs such as the Biomarker Wizard program (Ciphergen Biosystems, Inc., Fremont, Calif.) can be used to aid in analyzing mass spectra. The mass spectrometers and their techniques are well known to those of skill in the art.

A person skilled in the art understands that any of the components of a mass spectrometer (e.g., desorption source, mass analyzer, detect, etc.) and varied sample preparations can be combined with other suitable components or preparations described herein, or to those known in the art. For example, in some embodiments a control sample may contain heavy atoms (*e.g.* 13C) thereby permitting the test sample to be mixed with the known control sample in the same mass spectrometry run.

In one preferred embodiment, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

In some embodiments the relative amounts of one or more cancer markers present in a first or second sample is determined, in part, by executing an algorithm with a computer. The algorithm identifies at least one peak value in the first mass spectrum and the second mass spectrum. The algorithm then compares the signal strength of the peak value of the first mass spectrum to the signal strength of the peak value of the second mass spectrum of the mass spectrum. The relative signal strengths are an indication of the amount of the cancer marker that is present in the first and second samples. A standard containing a known amount of a cancer marker can be analyzed as the second sample to better quantify the amount of the biomolecule present in the first sample. In certain embodiments, the identity of the cancer markers in the first and second sample can also be determined.

**Determination of Standard Value, Specificity and Sensitivity**

In the present application, the standard expression level of a cancer marker, such as the blood concentration of CXCL13, can be determined statistically. For example, the blood concentration of CXCL13 in healthy individuals can be measured to determine the standard blood concentration of CXCL13 statistically. When a statistically sufficient population can be gathered, a value in the range of twice or three times the standard deviation (S.D.) from the mean value is often used as the standard value. Therefore, values corresponding to the mean value + 2 x .S.D. or mean value + 3 x S.D. may be used as standard values. The standard values set as described theoretically comprise 90% and 99.7% of healthy individuals, respectively.

Alternatively, standard values can also be set based on the actual expression level (e.g., blood concentration of CXCL13) in cancer patients. Generally, standard values set this way minimize the percentage of false positives, and are selected from a range of values satisfying conditions that can maximize detection sensitivity. Herein, the percentage of false positives refers to a percentage, among healthy individuals, of patients whose blood concentration of CXCL13 is judged to be higher than a standard value. On the contrary, the percentage, among healthy individuals, of patients whose blood concentration of CXCL13 is judged to be lower than a standard value indicates specificity. That is, the sum of the false positive percentage and the specificity is always 1. The detection sensitivity refers to the percentage of patients whose blood concentration of CXCL13 is judged to be higher than a standard value, among all cancer patients within a population of individuals for whom the presence of cancer has been determined.

As used herein, the term "test sensitivity" is the ability of a screening test to identify true disease, also characterized by being a test with high sensitivity has few false negatives, additionally a test independent of disease prevalence. The test sensitivity is calculated as true positive tests per total affected patients tested, expressed as a percentage.

The term "Test Specificity" is a screening test which is correctly negative in the absence of disease, has high specificity and few false positives, is independent of disease prevalence. The test specificity is calculated as true negative tests per unaffected individual s tested, expressed as a percentage.

The term "PPV" (Positive Predictive Value) is the percent of patients with positive test having disease, and thus assesses reliability of positive test. Calculation:

$$1. \text{PPV} = (\text{True positive})/(\text{True} + \text{False positives}).$$

The term "NPV" (Negative Predictive Value) refers to patients with negative test that do not have disease, and assesses reliability of negative test. Calculation:

$$2. \text{NPV} = (\text{True negative})/(\text{true and false negatives}).$$

As the relationship shown above indicates, each of the values for sensitivity, specificity, positive predictive value, and negative predictive value, which are indexes for evaluating the diagnostic accuracy, varies depending on the standard value for judging the level of the blood concentration of CXCL13.

A standard value is usually set such that the false positive ratio is low and the sensitivity is high. However, as also apparent from the relationship shown above, there is a trade-off between the false positive ratio and sensitivity. That is, if the standard value is decreased, the detection sensitivity increases. However, since the false positive ratio also increases, it is difficult to satisfy the conditions to have a "low false positive ratio". Considering this situation, for example, values that give the following predicted results may be selected as the preferable standard values in the present application: (1) standard values for which the false positive ratio is 50% or less (that is, standard values for which the specificity is not less than 50%) and (2) standard values for which the sensitivity is not less than 20%.

The standard values can be set using receiver operating characteristic (ROC) curve. An ROC curve is a graph that shows the detection sensitivity on the vertical axis and the false positive ratio (that is, "1--specificity") on the horizontal axis. A ROC curve can be obtained by plotting the changes in the sensitivity and the false positive ratio, which were obtained after continuously varying the standard value for determining the high/low degree of the blood concentration of a cancer marker, such as CXCL13.

The "standard value" for obtaining the ROC curve is a value temporarily used for the statistical analyses. The "standard value" for obtaining the ROC curve can generally be continuously varied within a range that allows to cover all selectable standard values. For example, the standard value can be varied between the smallest and largest measured blood CXCL13 values in an analyzed population.

Based on the obtained ROC curve, a preferable standard value to be used in the present application can be selected from a range that satisfies the above-mentioned conditions. Alternatively, a standard value can be selected based on a ROC curve produced by varying the standard values from a range that comprises most of the measured blood CXCL13.

## KITS FOR DETECTING CANCER OR MONITORING CANCER PROGRESSION

Another aspect of the present application relates to a kit for detecting cancer or monitoring cancer progression. In one embodiment, the kit includes reagents for determining expression of CXCL13 and/or CXCR5 in a biological sample, and instructions for how to use the reagents, wherein the reagents include an anti-CXCL13 antibody, an anti-CXCR5 antibody, or both.

## METHODS FOR TREATING OR PREVENTING CANCER USING ANTI-CXCL13, ANTI-CXCR5, ANTI-CXCL16 AND/OR ANTI-CXCR6 ANTIBODIES

Also described herein are methods for treating or preventing cancer using an anti-CXCL13 antibody and/or an anti-CXCR5 antibody. The method comprises administering to a subject in need of such treatment, a therapeutically effective amount of an anti-CXCL13 antibody, an anti-CXCR5 antibody, or a combination thereof. The cancer may be melanoma, lymphoma, myeloma, leukemia, sarcoma, blastoma or a carcinoma. Examples of carcinoma include, but are not limited to, acinic cell carcinoma, adenoid cystic carcinoma, adenocarcinoma, adenosquamous carcinoma, adrenocortical adenoma, adrenocortical carcinoma, anaplastic carcinoma, apudoma, basal cell carcinoma, carcinoid, carcinosarcoma, clear cell carcinoma, cylindroma, cystadenocarcinoma, ductal carcinoma, gastrinoma, giant cell carcinoma, glioma, glucagonoma, Hurthle cell carcinoma, insulinoma, large cell carcinoma, lobular carcinoma, medulloblastoma, medullary carcinoma, mucinous cystadenoma, mucoepidermoid carcinoma, neuroectodermal tumor, oncocytoma, papillary hidradenoma, papilloma, pleomorphic carcinoma, pulmonary blastoma, sarcomatoid carcinoma, serous cystadenoma, Signet ring cell carcinoma, small cell carcinoma, somatostatinoma, spindle cell carcinoma, squamous cell carcinoma, thymoma, verrucous carcinoma, and of organs or tissues that line the inner or outer surfaces of the body originating from endodermal, extodermal, or epithelial cells. These organs and tissues include, but are not limited to: bone, breast, central nervous system, cervix, colon, endometrium, esophagus, fallopian tube, gastrointestinal tract, kidney, lung, lymphoid, , mammary gland, oral cavity, ovary, pancreas, pituitary gland, prostate, rectum, reproductive tract, respiratory tract, stomach, sweat gland, thymus, thyroid, uterus, vagina.

The subject may be diagnosed with a cancer that results in elevated CXCL13 and/or CXCR5 expression in the cancer cells. Examples of such cancer include, but are not limited to, melanoma, lymphoma, myeloma, leukemia, sarcoma, blastoma and carcinoma. The subject may be diagnosed with brain cancer. The subject may be diagnosed with prostate cancer. The method may comprise the step of immunizing the subject with an effective amount of CXCL13 and/or CXCR5 immunogen(s) as protein, peptide or encoded gene to induce antibodies that inhibit the biological activity of CXCL13 and/or CXCR5The subject may be diagnosed with bone cancer. The subject may be diagnosed with pituitary cancer. The subject may be diagnosed with ovarian cancer. The subject may be diagnosed with lung cancer. The subject may be diagnosed with breast cancer. The subject may be diagnosed with colon cancer. The subject may be diagnosed with lymphoma or myeloma. The subject may be diagnosed with leukemia.

The method may comprise the step of immunizing the subject with an effective amount of CXCL16 and/or CXCR6 immunogen(s) as protein, peptide or encoded gene to induce antibodies that inhibit the biological activity of CXCL16 and/or CXCR6.

The method may comprise the step of immunizing the subject with an effective amount of one or more of CXCL13 and CXCR5 immunogens and an effective amount of one or more of CXCL16 and CXCR6 immunogens.

Also described herein are methods for inhibiting or preventing metastasis of a cancer by treatment of a subject in need thereof with an anti-CXCL13 antibody and/or an anti-CXCR5 antibody. An anti-CXCL13 antibody and/or an anti-CXCR5 antibody may inhibit or prevent invasion of a tissue by a cancer. The method may comprise the step of immunizing the subject with an effective amount of CXCL13 and/or CXCR5 immunogen(s) as protein, peptide or encoded gene to induce antibodies that inhibit the biological activity of CXCL13 and/or CXCR5. The cancer may be prostate cancer. The cancer may be melanoma. Said tissue may be bone.

Also described herein are methods for treatment of a subject in need thereof with an anti-CXCL13 antibody and/or an anti-CXCR5 antibody causing regression of an established tumor. The method may comprise the step of immunizing the subject with an effective amount of CXCL13 and/or CXCR5 immunogen(s) as protein, peptide or encoded gene to induce antibodies that inhibit the biological activity of CXCL13 and/or CXCR5. The established tumor may be an advanced tumor. The established tumor may be a metastatic tumor.

Also described herein are methods for treatment of a subject in need thereof with an anti-CXCL13 antibody and/or an anti-CXCR5 antibody to prevent or inhibit osteolytic growth of a tumor in bone. The method may comprise the step of immunizing the subject with an effective amount of CXCL13 and/or CXCR5 immunogen(s) as protein, peptide or encoded gene to induce antibodies that inhibit the biological activity of CXCL13 and/or CXCR5. The tumor may be a melanoma, lymphoma, sarcoma, blastoma or carcinoma. The tumor may be a carcinoma. The tumor may be prostate cancer. The treatment with an anti-CXCL13 antibody and/or an anti-CXCR5 antibody may prevent osteolysis or bone resorption by a tumor.

The method may further comprise determining the level of CXCL13 and/or CXCR5 expression in a tissue from the subject, and, if an increased level of CXCL13 and/or CXCR5 is detected, administering to the subject a therapeutically effective amount of an anti-CXCL13 antibody, an anti-CXCR5 antibody, or a combination thereof. The method may comprise the step of immunizing the subject with an effective amount of CXCL13 and/or CXCR5 immunogen(s) as protein, peptide or encoded gene to induce antibodies that inhibit the biological activity of CXCL13 and/or CXCR5.

A preferred antibody of the present invention is one which binds to human CXCL13 and preferably blocks (partially or completely) the ability of CXCL13 to bind to a receptor, including, but not limited to, CXCR5. Another preferred antibody of the present invention is one which binds to human CXCR5 and preferably blocks (partially or completely) the ability of a cell, such as a tumor or carcinoma cell, expressing the CXCR5 chemokine receptor at its cell surface to bind to a ligand, including, but not limited to, CXCL13. Yet another preferred antibody of the present invention is one which binds to human CXCR5 and preferably blocks (partially or completely) the ability of soluble CXCR5 chemokine receptor to bind to a ligand, including, but not limited to, CXCL13.

The anti-CXCL13 antibody and/or anti-CXCR5 antibody may be a monoclonal antibody. The anti-CXCL13 antibody and/or anti-CXCR5 antibody may be a humanized antibody. The anti-CXCL13 antibody and/or anti-CXCR6 antibody may be a humanized antibody fragment.

Treatment of a subject with an anti-CXCL13 and/or anti-CXCR5 antibody may be in conjunction with the treatment of the subject beforehand, at the same time, or afterward with a therapeutically effective amount of at least one other antibody that is specific for another antigen. The another antigen may be another chemokine or chemokine receptor, such as CXCL1, CXCL2, CXCL3, CXCL4, CXCL5, CXCL6, CXCL7, CXCL8, CXCL9, CXCL10, CXCL11, CXCL12, CXCL14, CXCL15, CXCL16, CXCR1, CXCR2, CXCR3, CXCR4, CXCR5a, CXCR5b, CXCR6, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, or CX3CL1.

The another antigen may be a chemokine or chemokine receptor associated with a carcinoma and selected from the group consisting of CCL1, CCL2, CCL4, CCL17, CCL19, CCL21, CCL22, CCL25, CXCL12, CXCL16, CCR2, CCR7, CCR8, CCR9, CXCR4, CXCR6, CXCR7, CX3CL1 and CX3CR1.

The another antigen may be a chemokine or chemokine receptor associated with a melanoma and selected from the group consisting of CCL25, CCL27, CXCL1, CXCL2, CXCL3, CXCL5, CXCL6, CXCL7, CXCL8, CXCL12, CXCL16, CX3CL1, CCR2, CCR9, CCR10, CXCR1, CXCR2, CXCR4, CXCR6, CXCR7 and CX3CR1.

The another antigen may be a chemokine or chemokine receptor associated with a lymphoma and selected from the group consisting of CXCL12, CXCR4, CXCR7, CCR2.

The another antigen may be a chemokine or chemokine receptor associated with a myeloma or leukemia.

The another antigen may be selected from the polypeptides recited in Table 1 and/or Table 2, and fragments of any of said polypeptides.

Other exemplary antigens include molecules such as renin; a growth hormone, including human growth hormone and bovine growth hormone; growth hormone releasing factor; parathyroid hormone; thyroid stimulating hormone; lipoproteins; a-1-antitrypsin; insulin A-chain; insulin B-chain; proinsulin; follicle stimulating hormone; calcitonin; luteinizing hormone; glucagon; clotting factors such as factor VIII, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-$\alpha$ and -$\beta$; enkephalinase; a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT4, NT-5, or NT-6), or a nerve growth factor such as NGF-$\beta$; platelet-derived growth factor (PDGF); fibroblast growth factor such as aFGF and bFGF; epidermal growth factor (EGF); members of the ErbB receptor family such as the EGF receptor; transforming growth factor (TGF)such as TGF-$\alpha$ and TGF-$\beta$, including TGF-$\beta$1, TGF-$\beta$2, TGF-$\beta$3, TGF-$\beta$4, or TGF-$\beta$5; insulin-like growth factor-I and -II (IGF-I and IGF-II); des(1-3)-IGF-I (brain IGF-I), insulin-like growth factor binding proteins; CD proteins such as CD3, CD4, CD8, CD19, CD20 and CD34; erythropoietin; osteoinductive factors; immunotoxins; a bone morphogenetic protein (BMP); an interferon such as interferon-a, -$\beta$, and -y; colony stimulating factors (CSFs), *e.g.,* M-CSF, GM-CSF, and G-CSF; interleukins (ILs), *e.g.,* IL-1, IL-2, IL-3, IL-4, IL-5, IL-6, IL-7, IL-8, IL-9 and/or IL-10; superoxide dismutase; T-cell receptors; surface membrane proteins; decay accelerating factor; viral antigen such as, for example, a portion of the AIDS envelope; transport proteins; homing receptors; addressins; regulatory proteins; $\alpha v/\beta 3$ integrin including either a or b subunits thereof, such as CD11a, CD11b, CD11c, CD 18, an ICAM, VLA-4 and VCAM; prostate specific antigen (PSA); a tumor associated antigen such as carcinoembryonic antigen (CEA), CK2, CA125, TA90, HER2, HER3 or HER4 receptor; blood group antigens; flk2/flt3 receptor; obesity (OB) receptor; mpl receptor; CTLA-4; protein C; any one of the proteins from the classical, lectin or alternative complement pathways; and fragments of any of the above-listed polypeptides.

The antibody may be administered to the subject with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. The antibody may be administered directly to a tumor or cancer tissue, including administration directly to the tumor bed during invasive procedures. The antibody may also be placed on a solid support such as a sponge or gauze for administration against the target chemokine to the affected tissues.

Antibodies of the invention can be administered in the usually accepted pharmaceutically acceptable carriers. Acceptable carriers include, but are not limited to, saline, buffered saline, and glucose in saline. Solid supports, liposomes, nanoparticles, microparticles, nanospheres or microspheres may also be used as carriers for administration of the antibodies.

The appropriate dosage ("therapeutically effective amount") of the antibody will depend, for example, on the condition to be treated, the severity and course of the condition, whether the antibody is administered for preventive or therapeutic purposes, previous therapy, the patient's clinical history and response to the antibody, the type of antibody used, and the discretion of the attending physician. The antibody is suitably administered to the patent at one time or over a series of treatments and may be administered to the patient at any time from diagnosis onwards. The antibody may be administered as the sole treatment or in conjunction with other drugs or therapies useful in treating the condition in question.

As a general proposition, the therapeutically effective amount of the antibody administered will be in the range of about 1 ng/kg body weight/day to about 100 mg/kg body weight/day whether by one or more administrations. The range of antibody administered may be from about 1 ng/kg body weight/day to about 1 $\mu$g/kg body weight/day, 1 ng/kg body weight/day to about 100 ng/kg body weight/day, 1 ng/kg body weight/day to about 10 ng/kg body weight/day, 10 ng/kg body weight/day to about 1 $\mu$g/kg body weight/day, 10 ng/kg body weight/day to about 100 ng/kg body weight/day, 100 ng/kg body weight/day to about 1 $\mu$g/kg body weight/day, 100 ng/kg body weight/day to about 10 $\mu$g/kg body weight/day, 1 $\mu$g/kg body weight/day to about 10 $\mu$g/kg body weight/day, 1 $\mu$g/kg body weight/day to about 100 $\mu$g/kg body weight/day, 10 $\mu$g/kg body weight/day to about 100 $\mu$g/kg body weight/day, 10 $\mu$g/kg body weight/day to about 1 mg/kg body weight/day, 100 $\mu$g/kg body weight/day to about 10 mg/kg body weight/day, 1 mg/kg body weight/day to about 100 mg/kg body weight/day and 10 mg/kg body weight/day to about 100 mg/kg body weight/day.

The antibody may be administered at a dosage range of 1 ng-10 ng per injection, 10 ng to 100 ng per injection, 100 ng to 1 $\mu$g per injection, 1 $\mu$g to 10 $\mu$g per injection, 10 $\mu$g to 100 $\mu$g per injection, 100 $\mu$g to 1 mg per injection, 1 mg to 10 mg per injection, 10 mg to 100 mg per injection, and 100 mg to 1000 mg per injection. The antibody may be injected daily, or every 2, 3, 4, 5, 6 and 7 days, or every 1, 2, 3 or 4 weeks.

The dose range of antibody administered may be from about 1 ng/kg to about 100 mg/kg The range of antibody administered may be from about 1 ng/kg to about 10 ng/kg, about 10 ng/kg to about 100 ng/kg, about 100 ng/kg to about

1 μg/kg, about 1 μg/kg to about 10 μg/kg, about 10μg/kg to about 100 μg/kg, about 100 μg/kg to about 1 mg/kg, about 1 mg/kg to about 10 mg/kg, about 10 mg/kg to about 100 mg/kg, about 0.5 mg/kg to about 30 mg/kg, and about 1 mg/kg to about 15 mg/kg.

The amount of antibody administered may be, or is about, 0.0006, 0.001, 0.003, 0.006, 0.01, 0.03, 0.06, 0.1, 0.3, 0.6, 1, 3, 6, 10, 30, 60, 100, 300, 600 and 1000 mg/day. As expected, the dosage will be dependant on the condition, size, age and condition of the patient.

The antibody may be administered, as appropriate or indicated, a single dose as a bolus or by continuous infusion, or as multiple doses by bolus or by continuous infusion. Multiple doses may be administered, for example, multiple times per day, once daily, every 2, 3, 4, 5, 6 or 7 days, weekly, every 2, 3, 4, 5 or 6 weeks or monthly. However, other dosage regimens may be useful. The progress of this therapy is easily monitored by conventional techniques.

Therapeutically effective amount of anti-CXCL13 and/or anti-CXCR5 antibody may be administered to a subject in need thereof as a sole therapeutic agent. The therapeutically effective amount of anti-CXCL13 and/or anti-CXCRS antibody may kill or promote apoptosis of the tumor or carcinoma cells. The therapeutically effective amount of anti-CXCL13 and/or anti-CXCRS antibody may inhibit or prevent the establishment of a tumor or carcinoma. The therapeutically effective amount of anti-CXCL13 and/or anti-CXCR5 antibody may inhibit or prevent the migration or metastasis of tumor or carcinoma cells from an existing tumor or carcinoma. The therapeutically effective amount of anti-CXCL13 and/or anti-CXCRS antibody may inhibit or prevent the invasion of tumor or carcinoma cells into non-cancerous tissues.

Therapeutically effective amount of anti-CXCL13 and/or anti-CXCRS antibody may be administered to a subject in need thereof in conjunction with one or more additional therapeutically effective antibodies. Said one or more additional therapeutically effective antibodies may be directed to additional determinants on CXCL13 and/or CXCR5, other chemokines, other chemokine receptors, other soluble or cell surface ligands or receptors including, but not limited to, tumor or carcinoma specific antigens, viral, bacterial or parasite antigens, products of cancer cells or remnants of apoptosis. The anti-CXCL13 and/or anti-CXCRS antibody may be administered before, concurrently with, and/or after the one or more additional therapeutically effective antibodies.

The therapeutically effective amount of anti-CXCL13 and/or anti-CXCRS antibody may augment the effectiveness of the one or more additional therapeutically effective antibodies in killing tumor or carcinoma cells. The therapeutically effective amount of anti-CXCL13 and/or anti-CXCRS antibody may reduce the amount of the one or more additional therapeutically effective antibodies required for killing tumor or carcinoma cells. The therapeutically effective amount of anti-CXCL13 and/or anti-CXCRS antibody may inhibit or prevent the migration or metastasis of tumor or carcinoma cells from an established tumor or carcinoma, enhancing the local effectiveness of the one or more additional therapeutically effective antibodies in killing tumor or carcinoma cells. The therapeutically effective amount of anti-CXCL13 and/or anti-CXCRS antibody may inhibit or prevent the invasion of tumor or carcinoma cells into non-cancerous tissues, enhancing the local effectiveness of the one or more additional therapeutically effective antibodies in killing tumor or carcinoma cells.

The anti-CXCL13 antibody and/or anti-CXCRS antibody may be an antibody conjugated to a cytotoxic agent. The anti-CXCL13 antibody and/or anti-CXCR5 antibody may be administered with another anti-cancer agent, such as chemotherapy agent.

Also described herein is a method of inhibiting the interaction of the chemokine CXCL13 with a receptor therefore, comprising contacting the cell with an effective amount of an antibody or functional fragment thereof which binds to a mammalian CXCL13 or a portion of CXCL13.

Also described herein is a method of inhibiting the interaction of a cell bearing CXCR5 with a ligand thereof, comprising contacting the cell with an effective amount of an antibody or functional fragment thereof which binds to a mammalian CXCR5 or a portion of CXCR5.

The method of treating cancer may comprise administering to an subject in need of such treatment, an effective amount of an expression vector that expresses an anti-CXCL13 antibody, an anti-CXCR5 antibody, or a combination thereof in a cancer or malignant cell. The method of treating cancer may comprise the step of immunizing the subject with an effective amount of CXCL13 and/or CXCR5 immunogen(s) to induce the host to produce antibodies that inhibit the biological activity of CXCL13 and/or CXCR5.

The expression vectors can be any vector that is capable of nucleotide deliver nucleotides encoding an anti-CXCL13 antibody and/or an anti-CXCR5 antibody into a target cell and express the anti-CXCL13 antibody and/or anti-CXCR5 antibody in the target cell. The expression vector may be capable of delivering nucleotides encoding CXCL13 and/or CXCR6 into a target cell to induce the host to produce anti-CXCL13 and/or CXCR5 antibodies. Examples of expression vectors include viral vectors and non-viral vectors.

Viral vectors include, but are not limited to, retrovirus vectors, adenovirus vectors, adeno-associated virus vectors, and other large capacity viral vectors, such as herpes virus and vaccinia virus. Also included are any viral families which share the properties of these viruses which make them suitable for use as expression vectors.

**Retroviral Vectors**

A retrovirus is an animal virus belonging to the virus family of Retroviridae, including any types, subfamilies, genus, or tropisms. Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)).

**Adenoviral Vectors**

Recombinant adenoviruses have been shown to achieve high efficiency gene transfer after direct, *in vivo* delivery to airway epithelium, hepatocytes, vascular endothelium, CNS parenchyma and a number of other tissue sites. Recombinant adenoviruses achieve gene transduction by binding to specific cell surface receptors, after which the virus is internalized by receptor-mediated endocytosis, in the same manner as wild type or replication-defective adenovirus.
A viral vector can be one based on an adenovirus which has had one or more viral genes removed and these virions are generated in a complement cell line, such as the human 293 cell line. In one embodiment, the E1 gene is removed from the adneoviral vector. In another embodiment, both the E1 and E3 genes are removed from the adneoviral vector. In another embodiment, both the E1 and E4 genes are removed from the adneoviral vector. In another embodiment, the adenovirus vector is a gutless adenovirus vector.

**Adeno-associated Viral Vectors**

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.
In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.
Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. United States Patent No. 6,261,834 discloses material related to the AAV vector.

**Large Payload Viral Vectors**

Molecular genetic experiments with large human herpes viruses have provided a means whereby large heterologous DNA fragments can be cloned, propagated and established in cells permissive for infection with herpes viruses (Sun et al., Nature genetics 8: 33-41, 1994; Cotter and Robertson, Curr Opin Mol Ther 5: 633-644, 1999). These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable. The maintenance of these episomes requires a specific EBV nuclear protein, EBNA1, constitutively expressed during infection with EBV. Additionally, these vectors can be used for transfection, where large amounts of protein can be generated transiently in vitro. Herpesvirus amplicon systems are also being used to package pieces of DNA > 220 kb and to infect cells that can stably maintain DNA as episomes. Other useful systems include, for example, replicating and host-restricted non-replicating vaccinia virus vectors.
Non-Viral vectors include plasmid expression vectors. Plasmid vectors typically include a circular double-stranded DNA loop into which additional DNA segments can be inserted.
In both viral and non-viral expression vectors, the polynucleotide encoding the antibody or antibodies is typically arranged in proximity and orientation to an appropriate transcription control sequence (promoter, and optionally, one or more enhancers) to direct mRNA synthesis. That is, the polynucleotide sequence of interest is operably linked to an appropriate transcription control sequence. Examples of such promoters include: viral promoters such as the immediate early promoter of CMV, LTR or SV40 promoter, polyhedron promoter of baculovirus, *E. coli* lac or trp promoter, phage T7 and lambda PL promoter, and other promoters known to control expression of genes in eukaryotic cells or their viruses. The promoters may be a tissue specific promoter.
The expression vector typically also contains a ribosome binding site for translation initiation, and a transcription terminator. The vector optionally includes appropriate sequences for amplifying expression. In addition, the expression vectors optionally comprise one or more selectable marker genes to provide a phenotypic trait for selection of transformed host cells, such as dihydrofolate reductase or neomycin resistance for eukaryotic cell culture, or such as tetracycline or ampicillin resistance in *E. coli.*

The expression vector can also include additional expression elements, for example, to improve the efficiency of translation. These signals can include, *e.g.*, an ATG initiation codon and adjacent sequences. In some cases, for example, a translation initiation codon and associated sequence elements are inserted into the appropriate expression vector simultaneously with the polynucleotide sequence of interest (*e.g.,* a native start codon). In such cases, additional translational control signals are not required. However, in cases where only a polypeptide coding sequence, or a portion thereof, is inserted, exogenous translational control signals, including an ATG initiation codon is provided. The initiation codon is placed in the correct reading frame to ensure translation of the polynucleotide sequence of interest. Exogenous transcriptional elements and initiation codons can be of various origins, both natural and synthetic. If desired, the efficiency of expression can be further increased by the inclusion of enhancers appropriate to the cell system in use (Scharf et al. (1994) Results Probl Cell Differ 20:125-62; Bitter et al. (1987) Methods in Enzymol 153:516-544).

The expression vector may contain an inducible or regulatable expression system. Examples of regulatable expression systems are briefly described below:

*Ecdysone system.* The ecdysone system is based on the molting induction system found in Drosophila, but modified for inducible expression in mammalian cells. The system uses an analog of the drosophila steroid hormone ecdysone, muristerone A, to activate expression of the gene of interest via a heterodimeric nuclear receptor. Expression levels have been reported to exceed 200-fold over basal levels with no effect on mammalian cell physiology.

*Progesterone system.* The progesterone receptor is normally stimulated to bind to a specific DNA sequence and to activate transcription through an interaction with its hormone ligand. Conversely, the progesterone antagonist mifepristone (RU486) is able to block hormone-induced nuclear transport and subsequent DNA binding. A mutant form of the progesterone receptor that can be stimulated to bind through an interaction with RU486 has been generated. To generate a specific, regulatable transcription factor, the RU486-binding domain of the progesterone receptor has been fused to the DNA-binding domain of the yeast transcription factor GAL4 and the transactivation domain of the HSV protein VP16. The chimeric factor is inactive in the absence of RU486. The addition of hormone, however, induces a conformational change in the chimeric protein, and this change allows binding to a GAL4-binding site and the activation of transcription from promoters containing the GAL4-binding site.

*Rapamycin system.* Immunosuppressive agents, such as FK506 and rapamycin, act by binding to specific cellular proteins and facilitating their dimerization. For example, the binding of rapamycin to FK506-binding protein (FKBP) results in its heterodimerization with another rapamycin binding protein FRAP, which can be reversed by removal of the drug. The ability to bring two proteins together by addition of a drug potentiates the regulation of a number of biological processes, including transcription. A chimeric DNA-binding domain has been fused to the FKBP, which enables binding of the fusion protein to a specific DNA-binding sequence. A transcriptional activation domain also has been fused to FRAP. When these two fusion proteins are co-expressed in the same cell, a fully functional transcription factor can be formed by heterodimerization mediated by addition of rapamycin. The dimerized chimeric transcription factor can then bind to a synthetic promoter sequence containing copies of the synthetic DNA-binding sequence. This system has been successfully integrated into adenoviral and AAV vectors. Long term regulatable gene expression has been achieved in both mice and baboons.

## METHODS FOR TREATING OR PREVENTING CANCER USING AGENTS THAT INHIBITS THE EXPRESSION OR ACTIVITY OF CXCL13 OR CXCR5

Also described herein are methods for treating or preventing cancer by using agents that inhibits the expression or activity of CXCL13 or CXCR5. The method may comprise administering to a subject in need of such treatment, an effective amount of an expression vector that expresses an agent that (1) inhibits the expression of CXCL13 and/or CXCR5, or (2) inhibits the interaction between CXCL13 and CXCR5, or (3) inhibits a biological activity of CXCL13 and/or CXCR5. The biological activity of CXCL13 and CXCR5 may include the interaction between CXCL13 and CXCR5.

The subject may be diagnosed with a cancer that results in elevated CXCL13 and/or CXCR5 expression in the cancer cells. Examples of such cancer include, but are not limited to, melanoma, lymphoma, myeloma, leukemia and carcinoma, such as ovarian cancer, vaginal cancer, cervical cancer, uterine cancer, prostate cancer, anal cancer, rectal cancer, colon cancer, stomach cancer, pancreatic cancer, insulinoma, glucagonoma, adenocarcinoma, adenosquamous carcinoma, neuroendocrine tumor, breast cancer, lung cancer, esophageal cancer, oral cancer, brain cancer, medulloblastoma, neuroectodermal tumor, glioma, pituitary cancer, and bone cancer.

The method may further comprise determining the level of CXCL13 and/or CXCR5 expression in a tissue from the subject, and administering the agent to the subject only if an increased level of CXCL13 and/or CXCR5 is detected in the tissue.

The expression vector may be a viral vector. The expression vector may be a non-vector vector. The expression vector may be capable of delivering nucleotides encoding CXCL13 and/or CXCR5 into a target cell to induce the host to produce anti-CXCL13 and/or CXCR5 antibodies.

The agent may be an anti-CXCL13 antibody, an anti-CXCR5 antibody, or a combination thereof.

The agent may be a functional nucleic acid. Functional nucleic acids are nucleic acid molecules that have a specific function, such as binding a target molecule or catalyzing a specific reaction. The functional nucleic acid molecules can act as inhibitors of a specific activity possessed by a target molecule. Functional nucleic acid molecules can interact with any macromolecule, such as DNA, RNA and polypeptides. Thus, functional nucleic acids can interact with mRNA or the genomic DNA of CXCL13 or CXCR5 to inhibit expression or interact with CXCL13 or CXCR5 protein to inhibit activity. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place. Examples of functional nucleic acid molecules include siRNA, antisense molecules, aptamers, ribozymes, triplex forming molecules, and external guide sequences.

siRNA is involved in RNA interference (RNAi) which involves a two-step mechanism: an initiation step and an effector step. In the first step, input double-stranded (ds) RNA (siRNA) is processed into small fragments, such as 21-23-nucleotide 'guide sequences'. RNA amplification occurs in whole animals. Typically then, the guide RNAs can be incorporated into a protein RNA complex which is capable of degrading RNA, the nuclease complex, which has been called the RNA-induced silencing complex (RISC). This RISC complex acts in the second effector step to destroy mRNAs that are recognized by the guide RNAs through base-pairing interactions. RNAi involves the introduction by any means of double stranded RNA into the cell which triggers events that cause the degradation of a target RNA. RNAi is a form of post-transcriptional gene silencing. In addition to the siRNAs disclosed herein, disclosed are RNA hairpins that can act in RNAi. For description of making and using RNAi molecules see, e.g., Hammond et al., Nature Rev Gen 2: 110-119 (2001); Sharp, Genes Dev 15: 485-490 (2001), Waterhouse et al., Proc. Natl. Acad. Sci. USA 95(23): 13959-13964 (1998) all of which at least form material related to delivery and making of RNAi molecules.

RNAi has been shown to work in many types of cells, including mammalian cells. For work in mammalian cells it is preferred that the RNA molecules which will be used as targeting sequences within the RISC complex are shorter. For example, less than or equal to 50 or 40 or 30 or 29, 28, 27, 26, 25, 24, 23, ,22, 21, 20, 19, 18, 17, 16 , 15, 14, 13 , 12, 11, or 10 nucleotides in length. These RNA molecules can also have overhangs on the 3' or 5' ends relative to the target RNA which is to be cleaved. These overhangs can be at least or less than or equal to 1, 2, 3, 4, 5, 6, 7, 8, 9, 10, 15, or 20 nucleotides long.

Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be *in vitro* selection experiments and DNA modification studies using DMS and DEPC. It is preferred that antisense molecules bind the target molecule with a dissociation constant (kd)less than or equal to 10-6, 10-8, 10-10, or 10-12. A representative sample of methods and techniques which aid in the design and use of antisense molecules can be found in the following non-limiting list of United States patents: 5,135,917, 5,994,320, 6,046,319, and 6,057,437.

Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind a chemokines and block its function (*see, e.g.,* Marro et al., Biochem Biophys Res Commun. 2006 Oct 13;349:270-6). Aptamers can bind very tightly with kds from the target molecule of less than $10\text{-}1^2$ M. It is preferred that the aptamers bind the target molecule with a $k_d$ less than $10^{-6}$, $10^{-8}$, $10^{-10}$, or $10^{-12}$. Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a $k_d$ with the target molecule at least 10, 100, 1000, 10,000, or 100,000 fold lower than the $k_d$ with a background binding molecule. Representative examples of how to make and use aptamers to bind a variety of different target molecules can be found in the following non-limiting list of United States patents: 5,476,766, 5,861,254, 6,030,776, and 6,051,698.

Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (*see, e.g.,* United States patents: 5,334,711 and 5,861,288, WO 9858058 and WO 9718312) hairpin ribozymes (see, *e.g.,* United States patents: 5,631,115 and 6,022,962), and tetrahymena ribozymes (see, *e.g.,* United States patents: 5,595,873 and 5,652,107). There are also a number of ribozymes that are not found in natural systems, but which have been engineered to catalyze specific reactions de novo (see, *e.g.,* United States patents: 5,580,967 and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence. Representative examples of how to make and use ribozymes to catalyze a variety of different reactions can be found in United States patents: 5,646,042, 5,869,253, 5,989,906, and 6,017,756.

Triplex forming functional nucleic acid molecules are molecules that can interact with either double-stranded or single-stranded nucleic acid. When triplex molecules interact with a target region, a structure called a triplex is formed, in which three strands of DNA are forming a complex dependant on both Watson-Crick and Hoogsteen base-pairing. Triplex molecules are preferred because they can bind target regions with high affinity and specificity. It is preferred that the triplex forming molecules bind the target molecule with a kd less than 10-6, 10-8, 10-10, or 10-12. Representative examples of how to make and use triplex forming molecules to bind a variety of different target molecules can be found in United States patents: 5,176,996, 5,683,874, 5,874,566, and 5,962,426.

External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate (see, e.g., WO 92/03566 by Yale, and Forster and Altman, Science 238:407-409 (1990)).

Similarly, eukaryotic EGS/RNAse P-directed cleavage of RNA can be utilized to cleave desired targets within eukaryotic cells. (Yuan et al., Proc. Natl. Acad. Sci. USA 89:8006-8010 (1992); WO 93/22434 by Yale; WO 95/24489 by Yale; Yuan and Altman, EMBO J 14:159-168 (1995), and Carrara et al., Proc. Natl. Acad. Sci. USA 92:2627-2631 (1995)). Representative examples of how to make and use EGS molecules to facilitate cleavage of a variety of different target molecules be found in the following non-limiting list of United States patents: 5,168,053, 5,624,824, 5,683,873, 5,728,521, 5,869,248, and 5,877,162.

## METHODS FOR PREVENTION OR INHIBITION OF MIGRATION OR METASTASIS OF CANCER CELLS WITH ELEVATED EXPRESSION OF CXCL13 AND/OR CXCR5

Also described herein is a method for prevention or inhibition of the migration or metastasis of cancer cells with elevated expression of CXCL13 and/or CXCR5 in a subject.

The method may comprise the step of administering to the subject a therapeutically effective amount of an anti-CXCL13 antibody, or an anti-CXCR5 antibody, or a combination thereof.

The method may comprise the step of administering to the subject an expression vector that expresses an anti-CXCL13 antibody, or an anti-CXCR5 antibody, or a combination thereof in said subject.

The method may comprise administering to the subject an expression vector that expresses an agent capable of inhibiting the expression of CXCL13 or CXCR5, or a biological activity of CXCL13 or CXCR5, or the interaction between CXCL13 and CXCR5. The expression vector may be capable of delivering nucleotides encoding CXCL13 and/or CXCR5 into a target cell to induce the host to produce anti-CXCL13 and/or CXCR5 antibodies.

Expression of CXCL13 and/or CXCR5 in cancer cells can be determined using methods well known in the art, such as immunostaining or quantitative PCR. Cancer cells that are known to overexpress CXCL13 and/or CXCR5 include, but are not limited to, melanoma cells and carcinoma cells. Examples of carcinoma include, but are not limited to, ovarian cancer, vaginal cancer, cervical cancer, uterine cancer, prostate cancer, anal cancer, rectal cancer, colon cancer, stomach cancer, pancreatic cancer, insulinoma, adenocarcinoma, adenosquamous carcinoma, neuroendocrine tumor, breast cancer, lung cancer, esophageal cancer, oral cancer, brain cancer, medulloblastoma, neuroectodermal tumor, glioma, pituitary cancer, and bone cancer.

The cancer cells may be brain cancer cells. The cancer cells may be bone cancer cells. The cancer cells may be pituitary cancer cells. The cancer cells may be ovarian cancer cells.

## METHOD FOR ENHANCING THE EFFECT OF CHEMOTHERAPY

Also described herein is a method for enhancing the effect of chemotherapy. The method may comprise administering to a subject who is under chemotherapy for a cancer, an effective amount of an anti-CXCL13 antibody, or an anti-CXCR5 antibody, or a combination thereof.

The method may comprise administering to a subject who is under chemotherapy for a cancer, an effective amount of an expression vector that expresses anti-CXCL13 antibody, or an anti-CXCR5 antibody, or a combination thereof.

The method may comprise administering to a subject who is under chemotherapy for a cancer an expression vector that expresses an agent capable of inhibiting the expression of CXCL13 or CXCR5, or a biological activity of CXCL13 or CXCR5, or the interaction between CXCL13 and CXCR5. The expression vector may be capable of delivering nucleotides encoding CXCL13 and/or CXCR5 into a target cell to induce the host to produce anti-CXCL13 and/or CXCR5 antibodies.

The subject may be under chemotherapy for melanoma, lymphoma, myeloma, leukemia or carcinoma. The subject may be under chemotherapy for brain cancer. The subject may be under chemotherapy for bone cancer. The subject may be under chemotherapy for pituitary cancer. The subject may be under chemotherapy for ovarian cancer.

**COMPOSITIONS AND KITS FOR TREATING OF PREVENTING CANCER**

Another aspect of the present invention relates to compositions and kits for treating or preventing cancer. In one embodiment, the composition comprises (1) an anti-CXCL13 antibody, an anti-CXCR5 antibody, or a combination thereof, and (2) a pharmaceutically acceptable carrier. In another embodiment, the composition comprises (1) an expression vector carrying the coding sequence for an anti-CXCL13 antibody, an anti-CXCR5 antibody, or a combination thereof, and (2) a pharmaceutically acceptable carrier. In another embodiment, the composition comprises (1) an expression vector carrying the coding sequence for an agent that inhibits the expression of CXCL13 or CXCR5, or a biological activity of CXCL13 or CXCR5, or the interaction between CXCL13 and CXCR5, and (2) a pharmaceutically acceptable carrier.

The composition of the present invention may contain a single type of antibody, such as an anti-CXCL13 or anti-CXCR5 antibody alone, or both types of antibodies. The composition may also contain therapeutically effective amounts of antibodies specific for one or more additional antigens as described above as necessary for the particular indication being treated, preferably those with complementary activities that do not adversely affect one another. For example, where the carcinoma being treated is ovarian cancer, it may be desirable to prepare a therapeutic formulation comprising anti-CXCL13 and/or -CXCR5 with one or more further anti-cancer determinant antibodies, such as an anti-CEA, anti-CA125 and/or anti-TA90 in a single formulation. In some embodiments of the present invention, a therapeutic antibody may be combined with an chemotherapy agent or a cytotoxic agent. In other embodiments of the present invention, a therapeutic antibody may be combined with an anti-inflammatory agent or a thrombolytic agent. Such agents are suitably present in combination in amounts that are effective for the purpose intended.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration. In certain embodiments, the pharmaceutical composition is administered directly into a tumor tissue.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a neuregulin) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. In certain embodiments, single dosage contains 0.01 ug to 50 mg of a chimeric neuregulin. The pharmaceutical compositions can be included in a container, pack, or dispenser together with instructions for administration.

The present invention is further illustrated by the following examples that should not be construed as limiting.

EXAMPLE 1: *In Vitro* Analysis of DOCK2 Expression in Cancer Cell Lines and CXCL13:CXCR5 Mediation of Cancer Cell Invasion

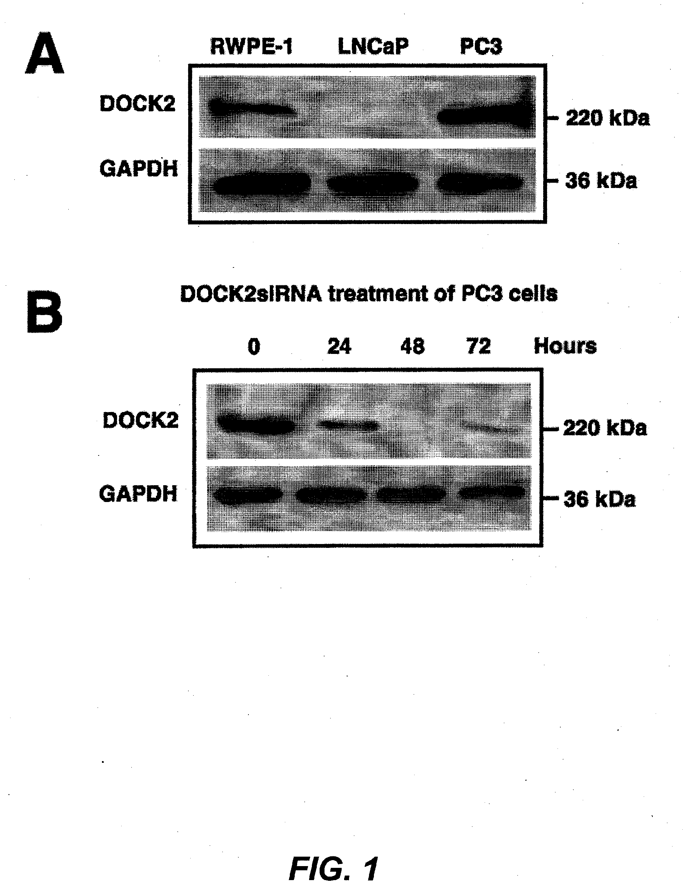

Total cell lysates (60 $\mu$g) from RWPE-1, LNCaP, and PC3 cells were resolved by SDS-PAGE and subjected to immunoblotting using antibodies against DOCK2 (FIG. 1A). GAPDH served as loading control. In FIG. 1B, DOCK2 silencing conditions were optimized by transfecting PC3 cells with 2$\mu$M of DOCK2 siRNA duplex following manufacturer's protocol (Santa Cruz), and incubating cells for 0, 24, 48, and 72 hours. The efficacy of DOCK2 silencing was determined by Western blot analysis.

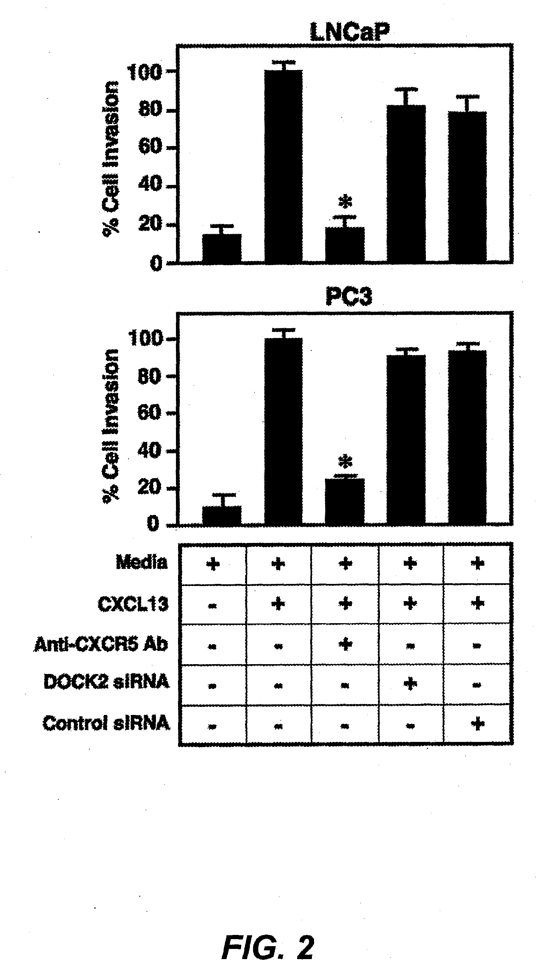

While CXCL13-CXCR5 interaction is known to mediate DOCK2-dependent chemotaxis of neutrophils, it was also found that CXCL13-CXCR5 interaction is also capable of promoting cancer cell metastasis and invasion independent of DOCK2. In FIG. 2, LNCaP and PC3 cells were tested for their ability to invade MATRIGEL™ Matrix and migrate through an 8.0 $\mu$m porous membrane in the presence of CXCL13 (100 ng/ml), anti-human CXCR5 antibody (1 $\mu$g/ml), DOCK2 or control siRNA. Cells which invaded to the lower surface of the membrane were stained with crystal violet and counted by microscopy at 40X magnification. Percent cell invasion was calculated following manufacturer's instructions (BD Biosciences). Error bars represent standard error of means of 3 independent experiments. Asterisks (*) indicate significant differences (p < 0.05) relative to CXCL13-treated cells (control).

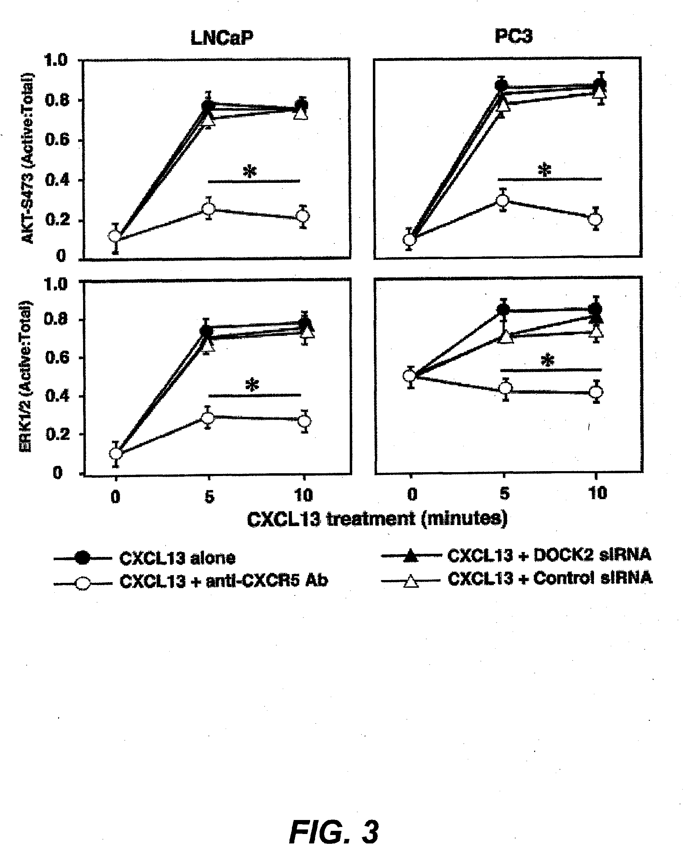

CXCL13 also regulates activation of Akt and ERK1/2, as shown in FIG. 3. FACE assays were performed to measure active and total Akt or ERK1/2 in LNCaP and PC3 cell lines. Cells were treated with anti-human CXCR5 antibody, DOCK2 siRNA, Control siRNA, or JNK inhibitor in the presence of CXCL13 (100 ng/ml) for 0, 5 or 10 minutes. Experiments were performed in triplicate and results show the ratio of active (phosphorylated) to total Akt or ERK1/2. Error bars represent $\pm$ standard error of means of 3 independent experiments. Asterisks (*) indicate significant (p < 0.05) decrease in phosphorylation.

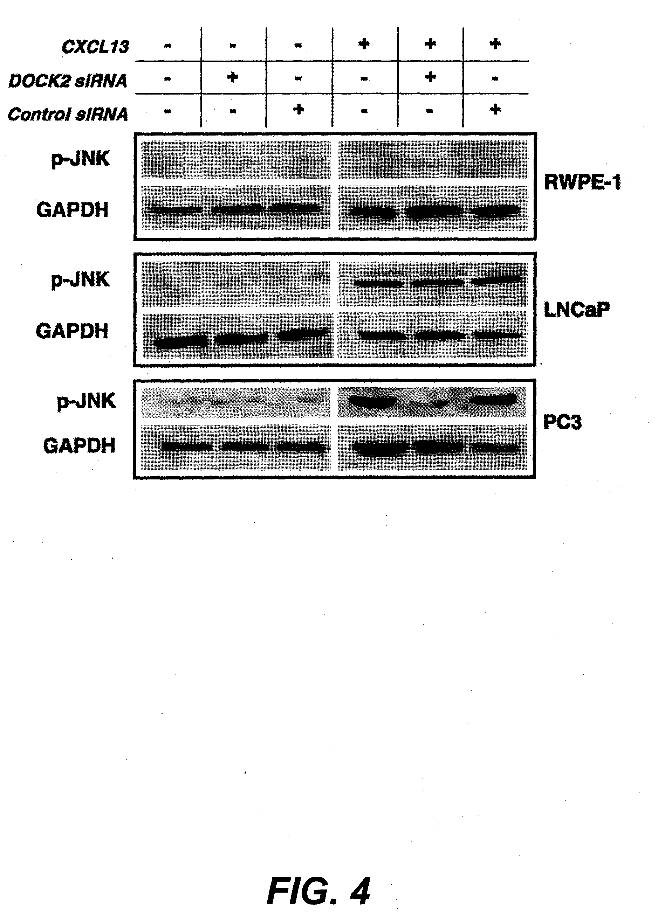

FIG. 4 shows that CXCL13 induces JNK activation through DOCK2 in PC3 cells. RWPE-1, LNCaP, and PC3 cells were treated with DOCK2 siRNA and corresponding control in the presence or absence of CXCL13 (100 ng/ml). Lysates were collected 5 minutes following CXCL13 stimulation and samples were resolved on SDS-PAGE. Membranes were blotted for phospho-JNK (46 kDa). GAPDH serves as loading control.

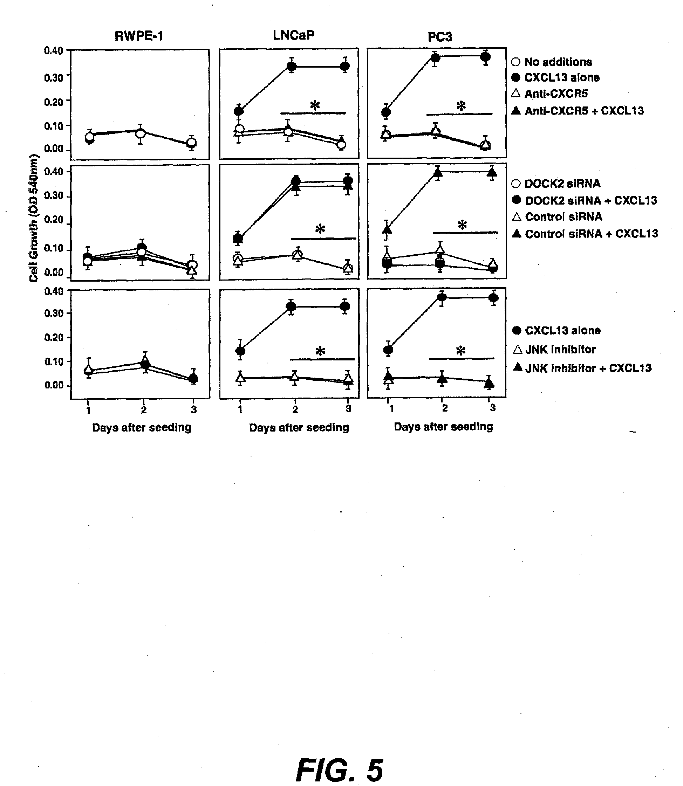

In FIG. 5, it is shown that CXCL13 regulates PCa cell proliferation through JNK and DOCK2. RWPE-1, LNCaP, and PC3 cells were grown in reduced serum conditions (2% FBS) in the presence or absence of 100 ng/ml CXCL13, 1 $\mu$g/ml anti-CXCR5 antibody, DOCK2 siRNA, and/or 10 $\mu$M JNK inhibitor. MTT assay was done over 3 days to assess cell proliferation. Error bars represent $\pm$ standard error of means of 3 independent experiments. Asterisks (*) indicate significant (p < 0.05) changes relative to CXCL13 treated cells.

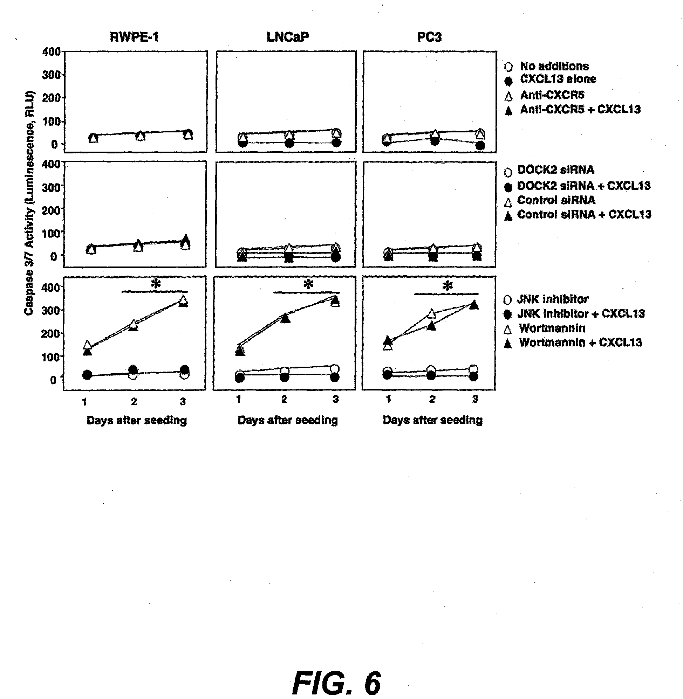

FIG. 6 shows JNK inhibition and DOCK2 knockdown lead to reduction of PCa cell proliferation that is not due to apoptosis. RWPE-1, LNCaP, and PC3 cells were grown in reduced serum conditions (2% FBS) in the presence or absence of 100 ng/ml CXCL13, 1$\mu$g/ml anti-CXCR5 antibody, DOCK2 siRNA, 10 $\mu$M JNK inhibitor, or 1 $\mu$M Wortmannin. Caspase activity was measured using the CASPASE-GLO 3/7 Assay (Promega, Madison, WI) according to manufacturer's directions. Asterisks (*) indicate significant (p < 0.05) changes relative to no additions.

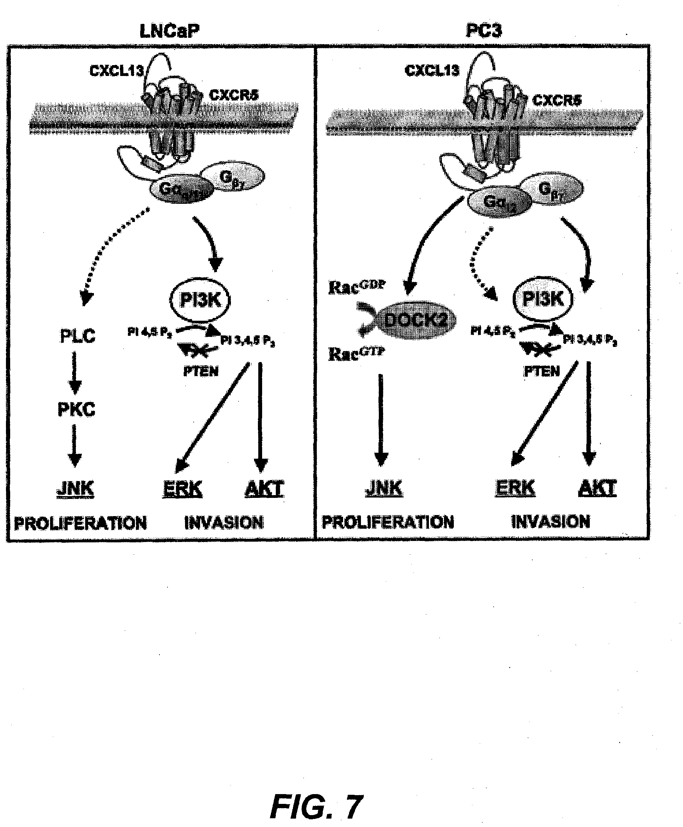

FIG. 7 shows CXCL13 modulation of signaling cascades in PCa cell lines. CXCL13 through its cognate receptor CXCR5 elicits Akt and ERK1/2 activation. In LNCaP cells CXCL13 also regulates JNK activation, presumably via G$\alpha$q/11 coupled to CXCR5, which mediates activation of phospholipase C (PLC) and protein kinase C (PKC). In PC3 cells, however, JNK activation is mediated through DOCK2.

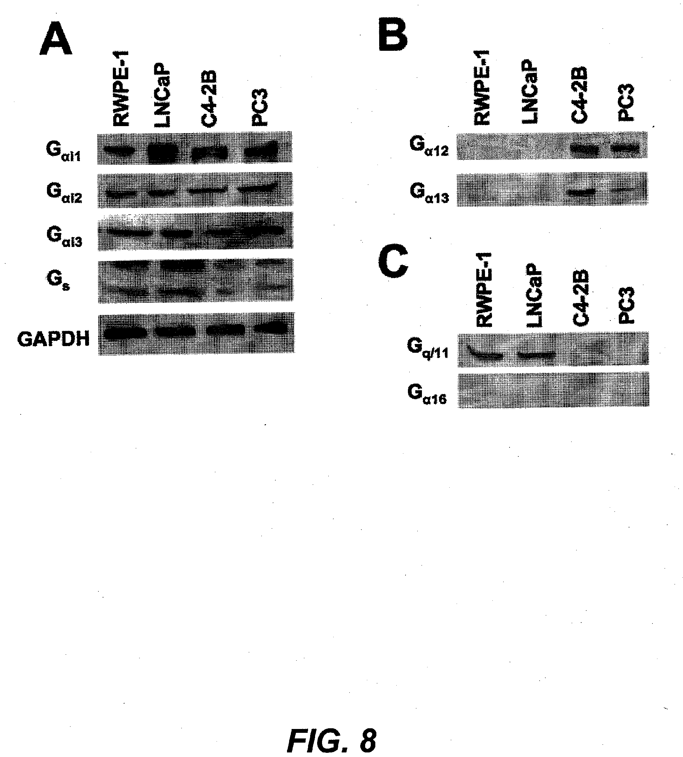

FIG. 8A-C demonstrate the expression of G-protein $\alpha$ subunit isoforms in prostate cancer cell lines. Equal amounts of protein (50 $\mu$g) from RWPE-1, LNCaP, C4-2B, and PC3 cells were resolved by SDS-Page. Expression of (A) G$_{\alpha i1,2,3}$, G$_{\alpha s;}$ (B) G$_{\alpha 12}$, G$_{\alpha 1}$3 and (C) G$_{\alpha q/11}$ and G$_{\alpha 16}$ were determined by immunoblot. GAPDH served as a loading control.

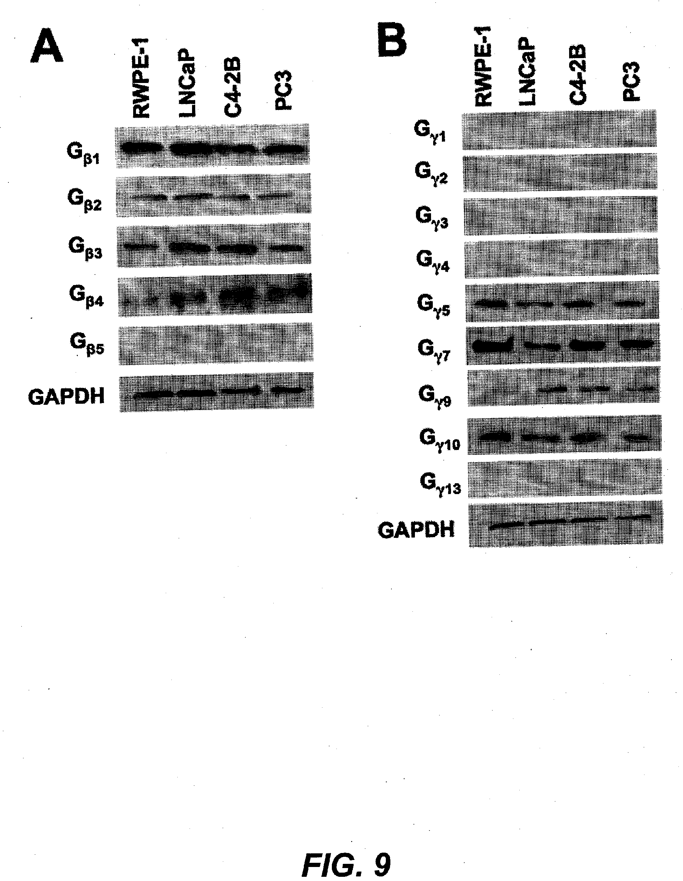

FIG. 9 demonstrates the expression of G-protein $\beta$ and $\gamma$ subunit isoforms in prostate cancer cell lines. Equal amounts of protein (50 $\mu$g) from RWPE-1, LNCaP, C4-2B, and PC3 cells were resolved by SDS-Page. Expression of G$_{\alpha 1,2,3,4,5}$ and G$_{\alpha 1,2,3,4,5,7,9,10,1}$3 were determined by immunoblot. GAPDH served as a loading control.

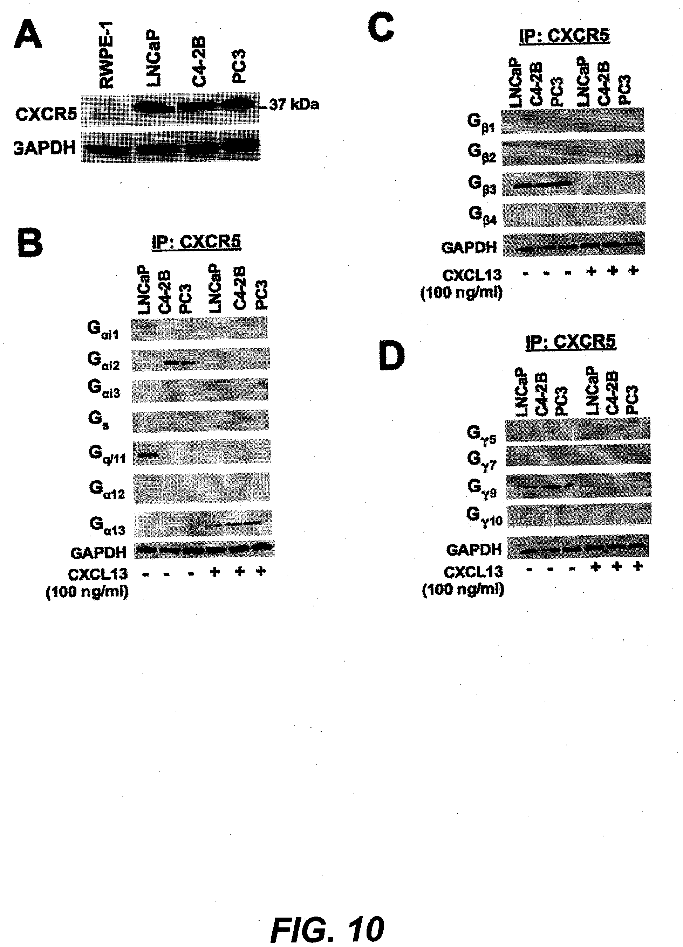

FIGS. 10A-D show expression of CXCR5 and associated G proteins in prostate cancer cell lines treated with or without CXCL13. (FIG. 10A) CXCR5 protein levels were analyzed by Western blot of RWPE-1, LNCaP, C4-2B, and PC3 cell lysates (50 $\mu$g). GAPDH served as loading control. Cell lines were treated with or without CXCL13 and lysed. CXCR5 was immuno-precipitated (IP) to pull down associated proteins from total cell lysates. The IP cell lysates were resolved by SDS-PAGE and the expression of (FIG. 10B) G$_{\alpha i1}$, G$_{\alpha i2}$, G$_{\alpha i3}$, G$_{\alpha s}$, G$_{\alpha q/11}$, G$_{\alpha}$12, G$_{\alpha 13}$, (FIG. 10C) G$_{\beta 1}$,

$G_{\beta2}$, $G_{\beta3}$, $G_{\beta4}$, and (FIG. 10D) $G_{\gamma5}$, $G_{y7}$, $G_{\gamma9}$, $G_{\gamma10}$ were examined by immunoblot.

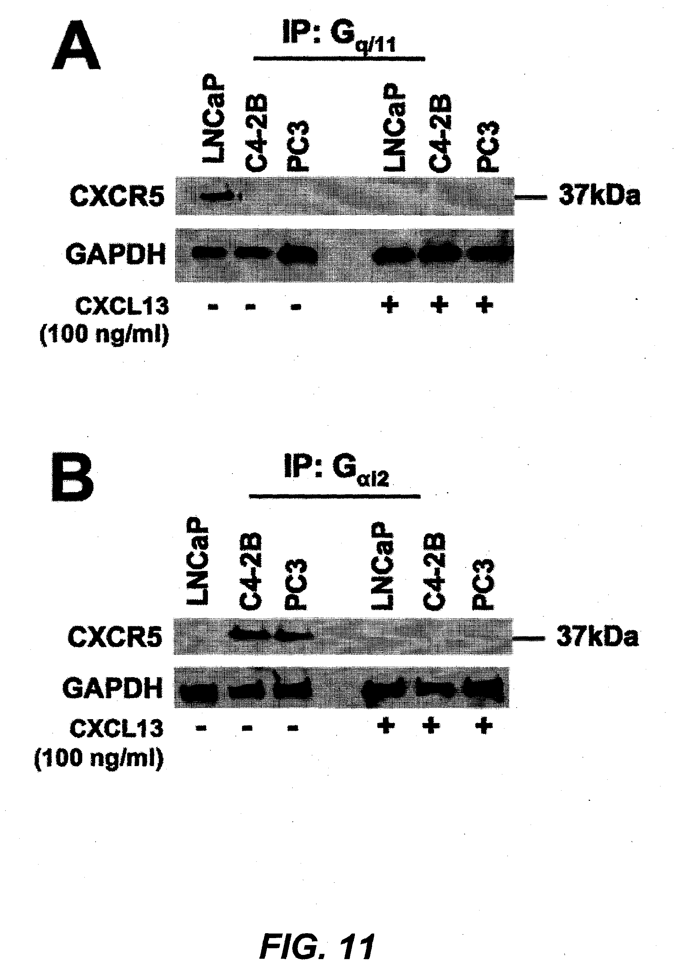

The validation of Gq/11 and $G_{\gamma5}$i2 protein association with CXCR5 by immunoprecipitation is shown in FIG. 11. Cell lines were treated with or without CXCL13 and lysed (A) $G_{\alpha q/11}$ and (B) $G_{\alpha i2}$ were immunoprecipitated (IP) from total cell lysates. The IP cell lysates were resolved by SDS-PAGE and CXCR5 expression was examined by immunoblot.

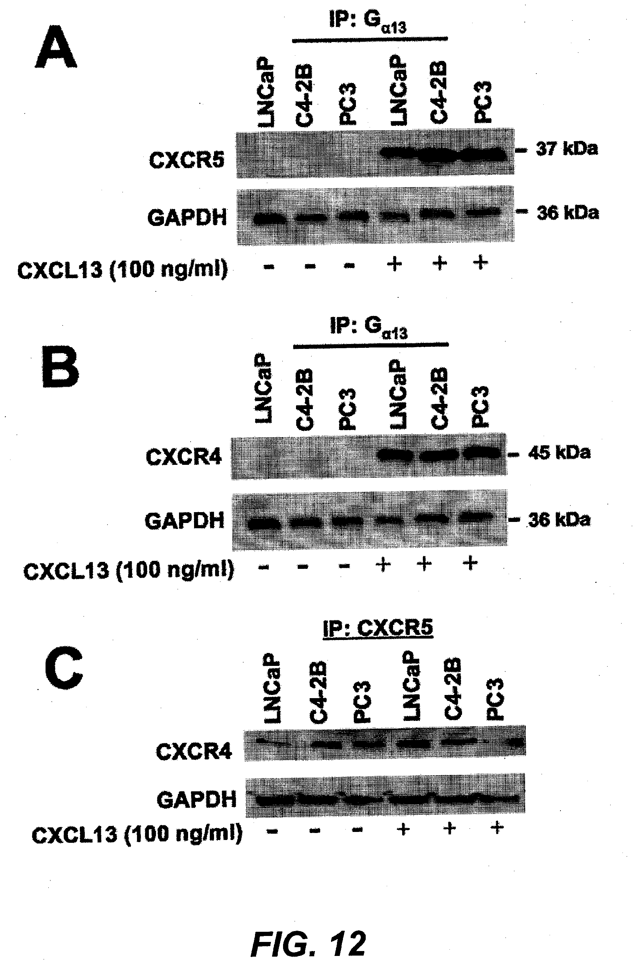

Identification of CXCR4 and CXCR5 coupled to $G_{\alpha 13}$ following CXCL13 stimulation is shown in FIGS. 12A-C. Cell lines were treated with or without CXCL13 and lysed. Antibody against $G_{\alpha 13}$ was used to immunoprecipitate (IP) it from total cell lysates. The IP cell lysates were resolved by SDS PAGE and immunoblotted for (FIG. 12A) CXCR5 and (FIG. 12B) CXCR4. (FIG. 12C) Western blot analysis of CXCR4 expression was also performed for CXCR5 IP lysates before and after CXCL13 treatment. GAPDH served as a loading control.

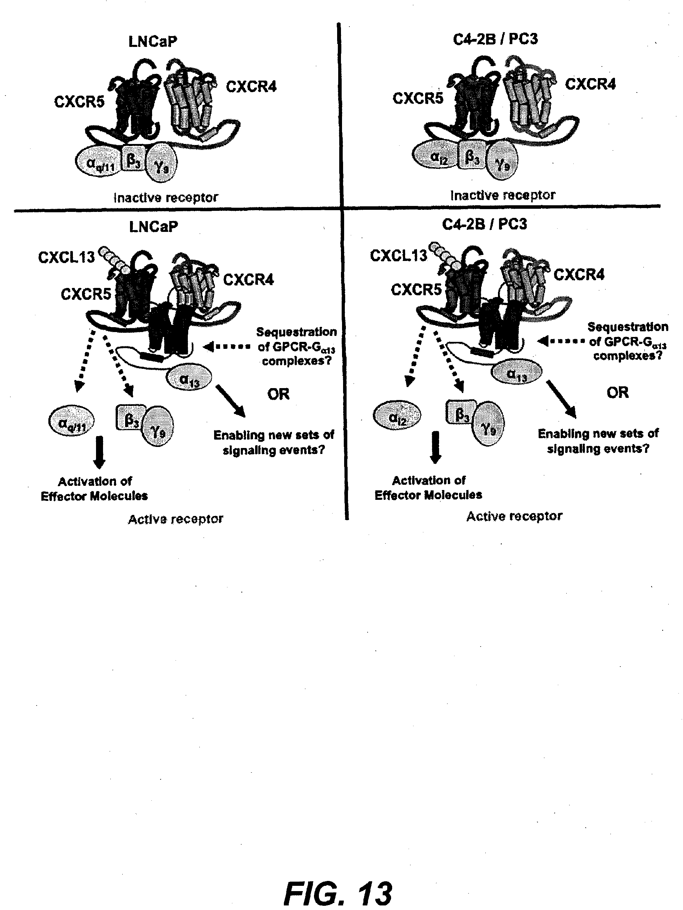

FIG. 13 depicts a hypothetical model of CXCR5 interactions in prostate cancer cells. CXCR5 associates with CXCR4 and couples with $G_{\alpha q/11}$/$G_{\beta3}$/$G_{\gamma9}$ heterotrimers in androgen-dependent LNCaP cell lines or $G_{\alpha i2}$/ $G_{\beta3}$/$G_{\gamma9}$ heterotrimers in hormone refractory C4-2B and PC3 cell lines in the absence of its specific ligand, CXCL13. Upon CXCL13 stimulation, G-proteins dissociate from CXCR5 to activate effector molecules. In addition, CXCL13-activated CXCR5 causes, associates or sequesters $G_{\alpha 13}$ protein favoring signals that would promote PCa cell motility.

Table 1 shows the different networks that are affected by anti-CXCL13 and/or anti-CXCR5 treatment of prostate cancer cells, and the functions in prostate cancer cells that each of those networks is involved in. Score indicates the number of molecules known to participate in each respective network. Focus molecules (indicated in bold) are those molecules of particular interest or importance in each network.

Table 1. Highest scoring networks involved in CXCL13-treated metastatic prostate cancer

| Network ID | Molecules in Network (Focus Molecules underlined) | Score | # of Focus Molecules | Top Functions |
|---|---|---|---|---|
| 1 | AKT1, AKT2, RTK, AMPK, ATM/ATR, BRCA1, CDC2, CDC25A, CDC25B/C, CDC25C, CDK2, CDKN1B, CHEK1, CHEK2, Cyclin A, Cyclin B, Cyclin D, Cyclin E, E2f, Fcer1, Foxo, Ige, Laminin, LIMK1, MAP2K2, MAP2K3, MEF2, Mek, Pkg, PRKAA1, RAF1, Rb, RB1, Scf, STMN1 | 31 | 19 | Cancer, Cell Cycle |
| 2 | Actin, α Actinin, β Arrestin, Calpain, CAV1, CFL1, Cofilin, Collagen(s), CTTN, Dynamin, Erm, EZR, F Actin, FAK-Src. FCGR1A/2A/3A, G3BP1, Integrin αVβ3, KRT18. MAP2K1/2, NF2, NTRK2, Pak, phosphatase, PTEN, PTK2, PXN, Rac, Ras homolog, Rock, SRC, Talin, VASP | 26 | 20 | Cellular Movement, Cell Morphology |
| 3 | AKT1, ALP, Calmodulin, CaMKII, Caspase, CDKN1A, Ck2, Creb, CREB1, CTNNB1, Cytochrome c, ERBB2, ESR1, FSH, GLRX2, HDAC8, Histone h3, Histone h4, Hsp70, HSP84-2, HSP90AB1, ICAM1, JUN, Nfat, PDPK1, Pp2b, Proteasome, RNA polymerase II, Rxr, Smad, SYN1, TFIIH, Tubulin, YWHAZ | 21 | 14 | Cancer, Reproductive System Disease |

Table 2 shows the proteins that have been found to be regulated by CXCL13 and CXCR5 in prostate cancer cells. The molecules are arranged according to the particular biological functions they are associated with in the cells and the functions or diseases for which their increased expression in the cells can be used as a marker.

Table 2. Proteins regulated by CXCL13 and their relevant biological functions in PC3 cells

| Biological functions and diseases | Molecules | p-value |
|---|---|---|
| Growth of tumor cell lines | AKT1, AKT2, BAD, BCL2, BCL2L1, CAV1, CDC2, CDK2, ELK1, JUN, MAPK3, MAPK8, NF2, PTK2, RAF1, SRC, STMN1 | 1.11E-09 |
| Proliferation of tumor cell lines | AKT1, AKT2, BAD, CAV1, GJA1, ITGB3, JUN, JUNB, LIMK1, MAPK3, MAPK8, PDPK1, SRC | 6.66E-08 |
| Anti-Apoptosis | AKT1, AKT2, BAD, BCL2, BCL2L1, CAV1, CDC2, ITGB3, JUN, MAPK3, MAPK8, PDPK1, PTK2, SRC, STMN1, VAV1 | 4.26E-07 |

(continued)

| Biological functions and diseases | Molecules | p-value |
|---|---|---|
| Prostate carcinoma | AKT1, AKT2, CDC2, CDK2, ITGB3, JUN, RAF2, SRC | 9.16E-07 |
| Metastasis | AKT1, ITGB1, NF2, PTK2, RELA, SRC | 9.29E-07 |
| Cell cycle progression | BCL2, CAV1, CDC25C, CDK2, MAPK8, RAF1, VAV1 | 1.85E-05 |
| Survival of tumor cell lines | AKT1, AKT2, BCL2, BCL2L1, CAV1, CDK2, CDKN1A, CDKN1B, CHEK1, CHEK2, CREB1, EGFR, ERBB2, FRAP1, JAK1, MET, NFKB1, NFKB2, NTRK2, PDGFRB, PRKAA1, PTK2, RELA, RELB, SRC, STAT3 | 2.05E-05 |

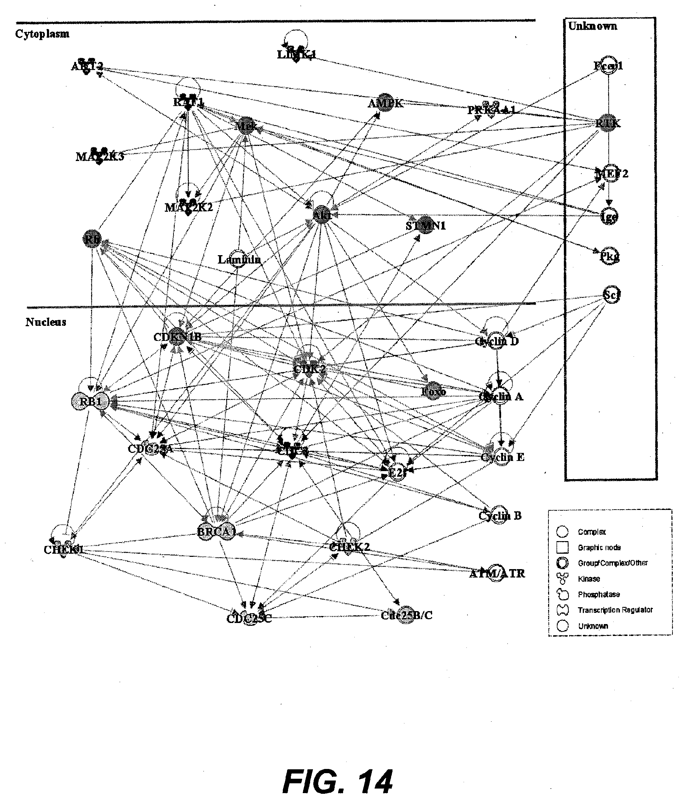

FIG. 14 depicts how CXCL13 regulates key molecules involved in cell cycle. Phospho-specific antibody microarrays were separately hybridized with CXCL1 3-treated or untreated PC3 cell lysates. Ratios of phosphorylated to unphosphorylated molecules were calculated and the datasets uploaded into the Ingenuity Pathways Analysis application. Networks were algorithmically generated based on the molecules' connectivity. Results were normalized to GAPDH levels. Colors represent fold changes in phosphorylation. Gray indicates no change in phosphorylation status, green indicates decreased phosphorylation, pink indicates baseline phosphorylation, and red indicates increased phosphorylation relative to baseline.

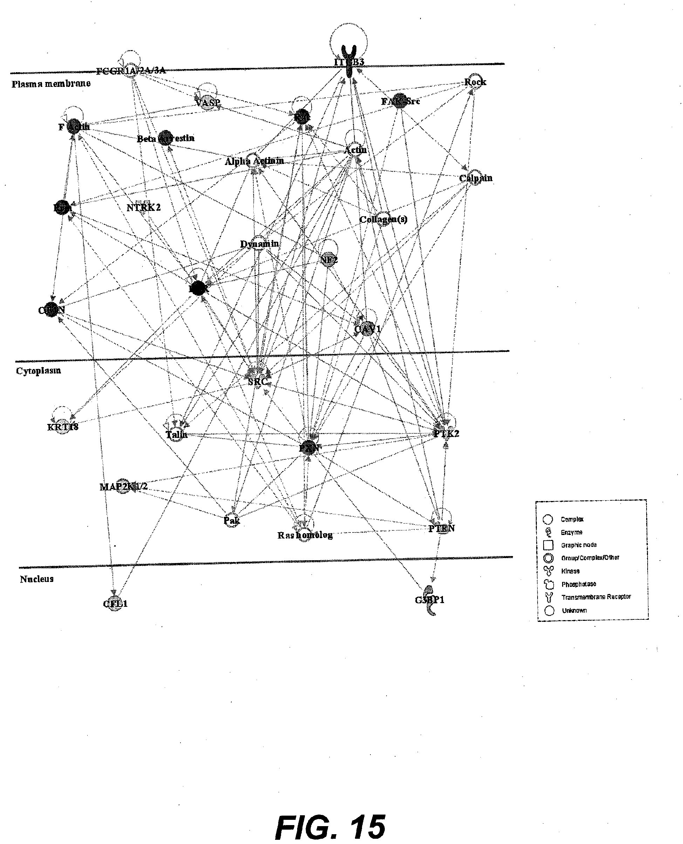

FIG. 15 depicts how CXCL13 regulates key molecules involved in cell migration. Phospho-specific antibody microarrays were separately hybridized with CXCL13-treated or untreated PC3 cell lysates. Ratios of phosphorylated to unphosphorylated molecules were calculated and the datasets uploaded into the Ingenuity Pathways Analysis application. Networks were algorithmically generated based on the molecules' connectivity. Results were normalized to GAPDH levels. Colors represent fold changes in phosphorylation. Gray indicates no change in phosphorylation status, green indicates decreased phosphorylation, pink indicates baseline phosphorylation, and red indicates increased phosphorylation relative to baseline.

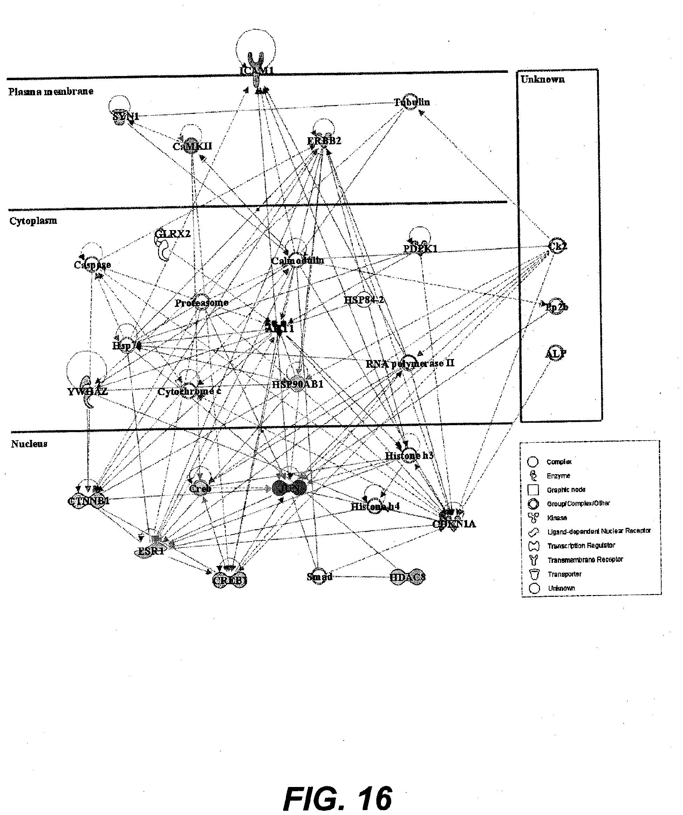

FIG. 16 depicts how CXCL13 regulates key molecules involved in cell survival and growth. Phospho-specific antibody microarrays were separately hybridized with CXCL13-treated or untreated PC3 cell lysates. Ratios of phosphorylated to unphosphorylated molecules were calculated and the datasets uploaded into the Ingenuity Pathways Analysis application. Networks were algorithmically generated based on the molecules' connectivity. Results were normalized to GAPDH levels. Colors represent fold changes in phosphorylation. Gray indicates no change in phosphorylation status, green indicates decreased phosphorylation, pink indicates baseline phosphorylation, and red indicates increased phosphorylation relative to baseline.

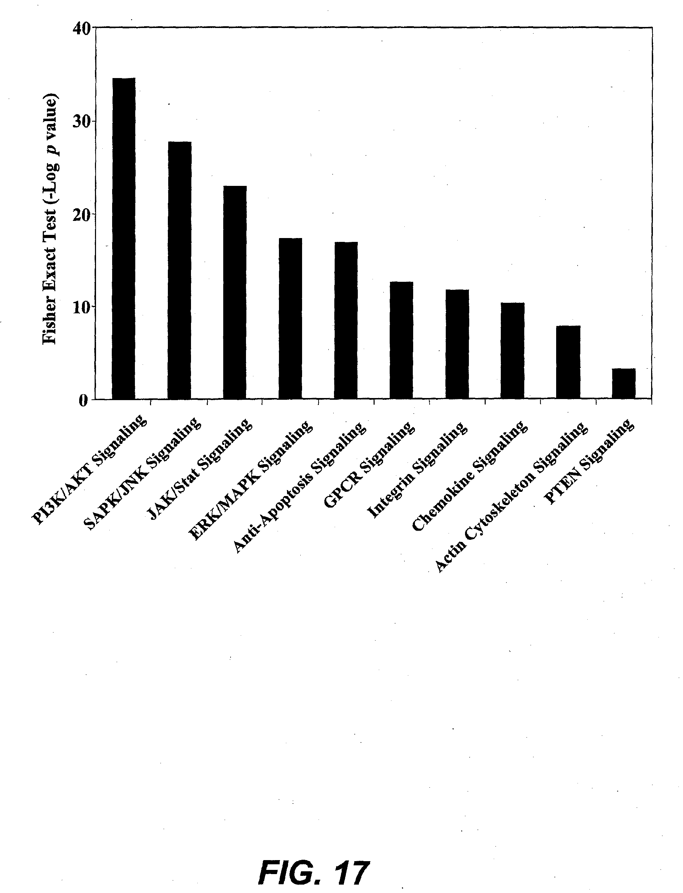

The top ten signaling pathways regulated by CXCL13 based on their significance (p-value) calculated using the right-tailed Fisher's Exact test using the entire dataset are shown in FIG. 17.

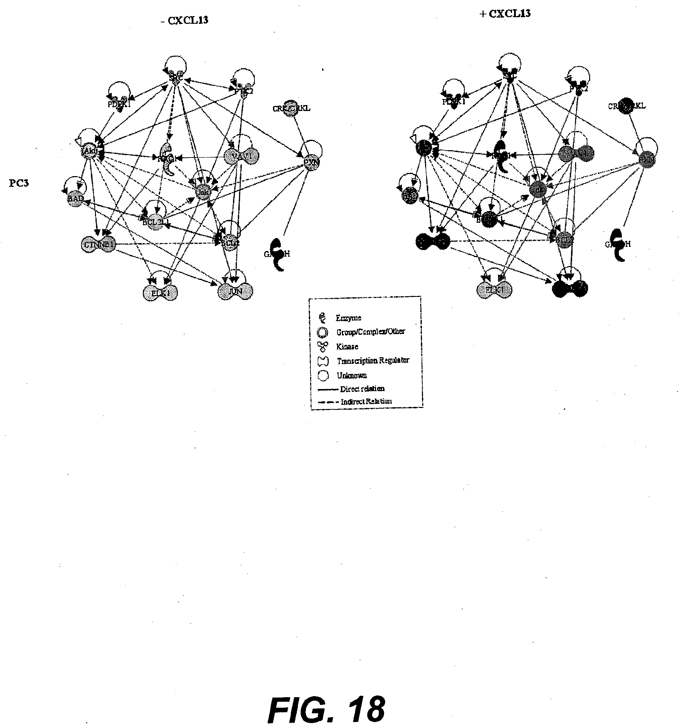

It was found that CXCL13 mediates differential phosphorylation of proteins (colored molecules) belonging to the P13K/Akt and SAPK/JNK signaling pathways, as shown in FIG. 18. The two canonical pathways were merged and overlaid with the analyzed microarray data from CXCL13-treated or untreated PC3 cells. Gray indicates no change in phosphorylation status, green indicates decreased phosphorylation, pink indicates baseline phosphorylation, and red indicates increased phosphorylation relative to baseline.

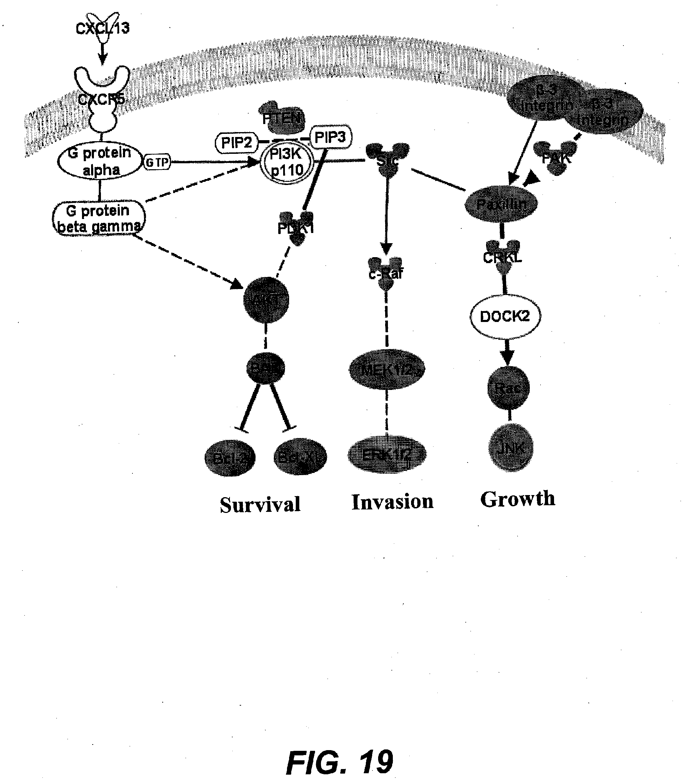

FIG. 19 summarizes the signaling pathways modulated by CXCL13-CXCR5 interactions. CXCL13 binding to CXCR5 results in the activation of PI3K/Akt, Raf/MEK/ERK, Integrin /33/Src/FAK, and DOCK2/Rac/JNK pathways involved in cell survival, invasion, and growth respectively.

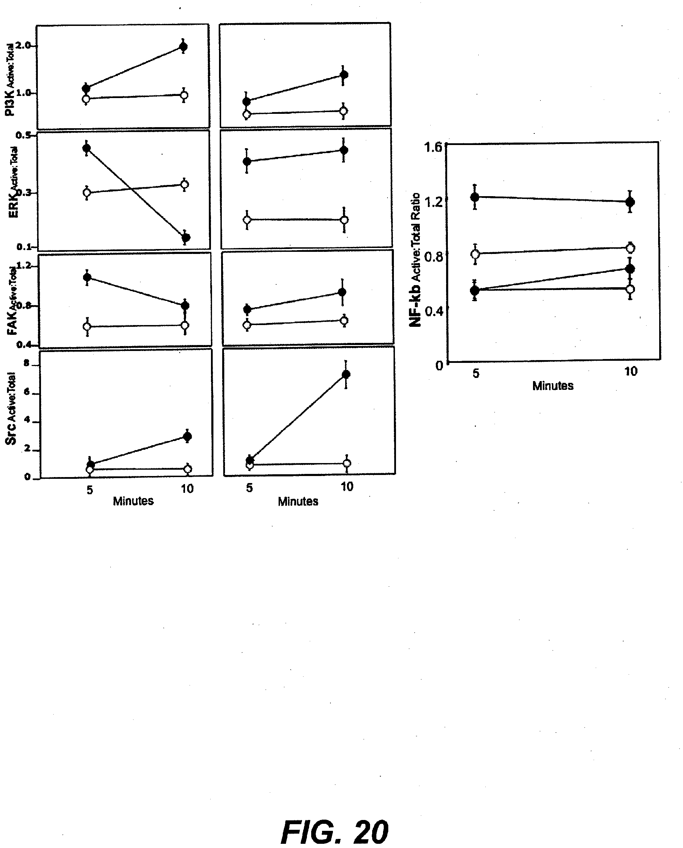

A confirmation of major CXCL13-CXCR5 cell signaling cascades is shown in FIG. 20. LNCaP (blue circles) or PC3 (magenta circles) cells received no additions (open circles) or 100 ng/ml of CXCL13 (closed circles) for 5 or 10 minutes. FACE™ assays (Active Motif, Carlsbad, CA) were used to detect both active and inactive (total) PI3K, ERK, FAK, Src kinase and NFkb proteins, 5 or 10 minutes after stimulation. Ratios of active (phosphorylated) to total proteins are presented $\pm$ SEM from 3 separate experiments performed in triplicate.

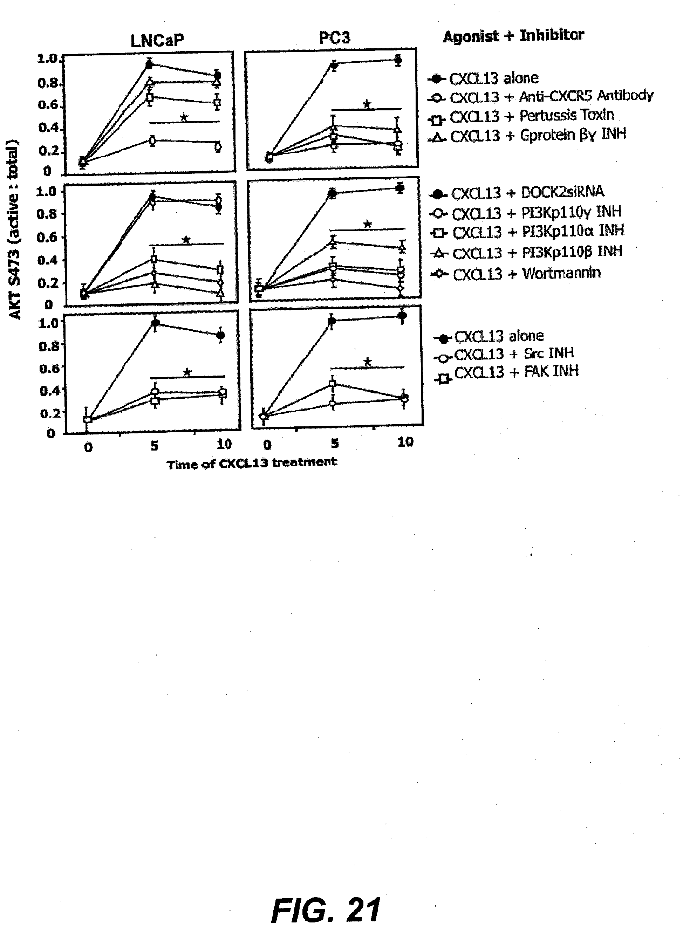

CXCL13-CXCR5 signaling events required for Akt activation are shown in FIG. 21. FACE assays were performed to measure active and total Akt levels in LNCaP and PC3 cell lines. Cells were treated with (or without) CXCL13 for 5 or 10 minutes, along with or without CXCR5 blockade, pertussis toxin, U-73122, wortmannin, PI-103, TGX221, and AS605240, DOCK2 siRNA, SU6656, and PF-573228. Experiments were performed in triplicate and results show the ratio of p-Akt to total Akt.

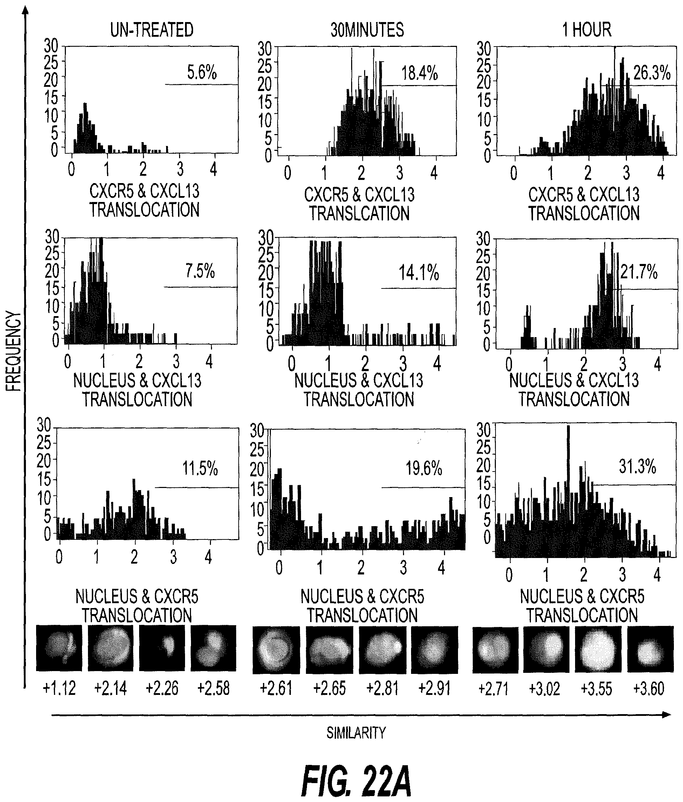

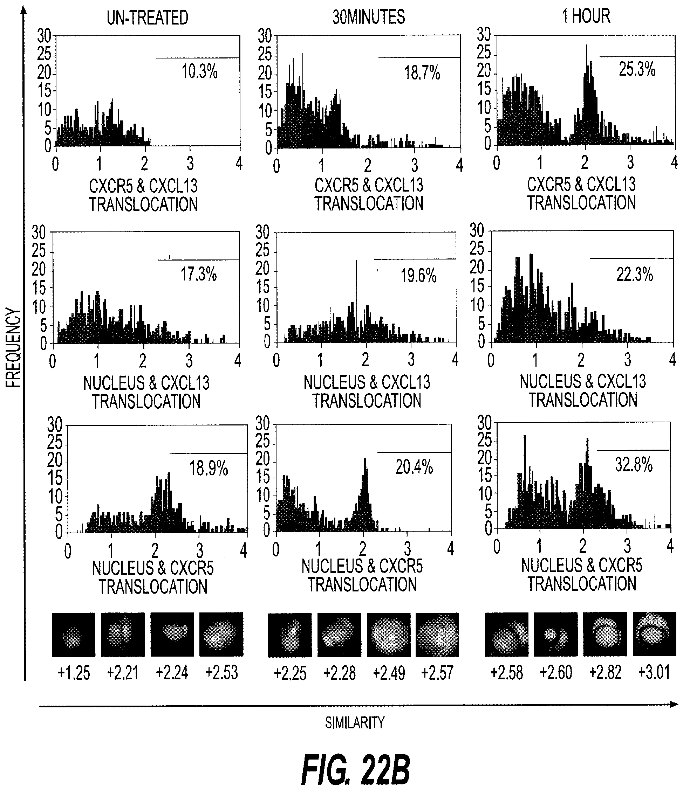

FIGS. 22A-B show CXCL13-mediated CXCR5 ligation and translocation to nuclei. LNCaP (FIG. 22A) and PC3 (FIG. 22B) cancer cell lines were stained with FITC (green)-conjugated anti-CXCR5 antibody, Alexa455 (orange)-conjugated anti-CXCL13 antibody and 7AAD (red) as a nuclear stain 30 and 60 minutes after treatment with 0 or 100 ng/ml of CXCL13. Histograms indicate the degree of signal correlation between CXCR5 and CXCL13 or these pairs with nuclei. The gate for positive population thresholds was determined by referencing dark field and 7AAD similarity scores with the Amnis Imagestream INSPIRE™ and IDEAS™ acquisition, and analysis software and system. The percentage of translocated cells are given above the 'Translocated' region bars.

EXAMPLE 2: Anti-CXCL13 Antibody Treament Inhibits Metastasis and Tumor Growth in Bone

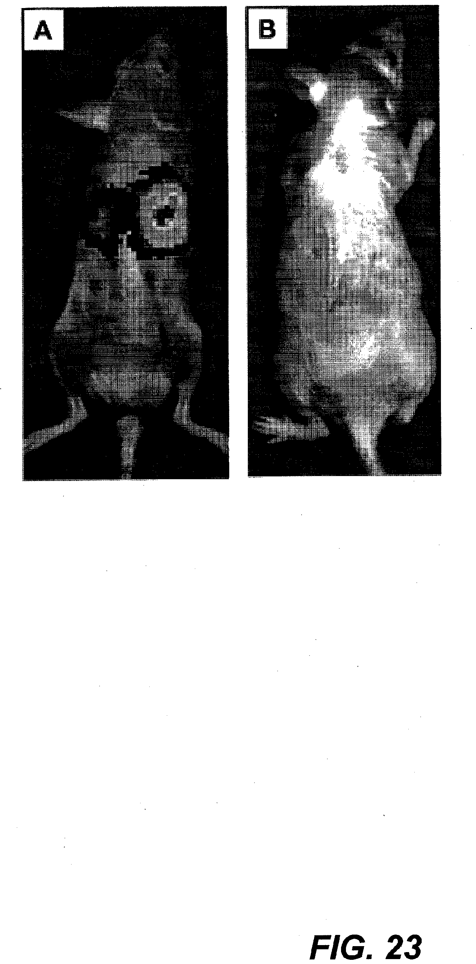

Anti-CXCL13 antibody treatment is shown in FIGS. 23A-B to inhibit prostate cancer progression and bone metastasis. Two groups of 10 ten-week old B6.Cg-Foxn-Nu/J male mice were challenged with $10^6$ luciferase-positive PC3 cells in 50 μl of saline by intracardiac injection. Prostate tumors were allowed to develop over 30 days; afterwards groups received either 0.5 μg of control (FIG. 23A) or anti-CXCL13 (FIG. 23B) antibodies every three days for an additional 30 days. This representative image shows the changes in tumor burden and bone metastasis that was analyzed by in vivo imaging using a Caliper/Xenogen IVIS 100 imaging system (Caliper, San Diego, CA).

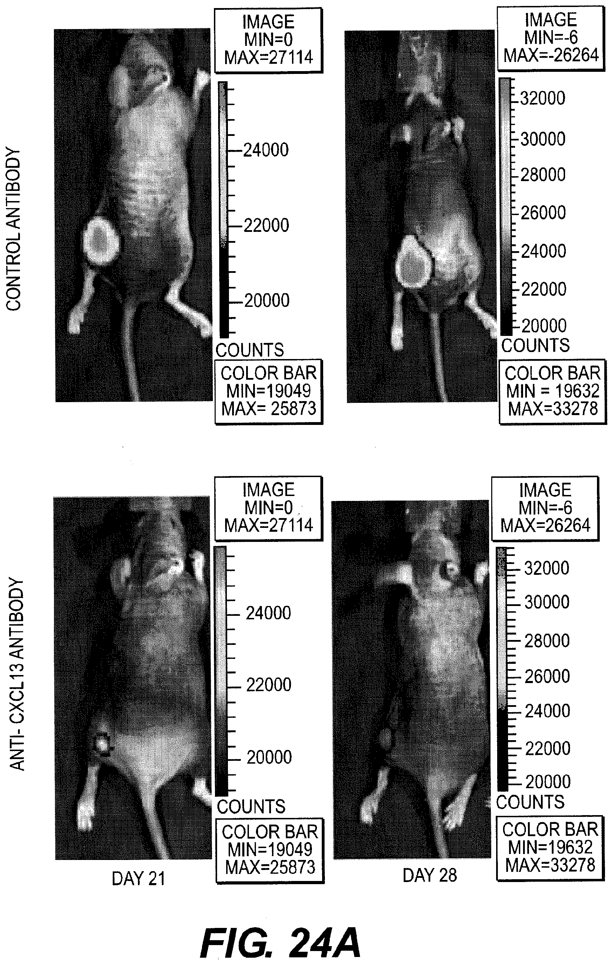

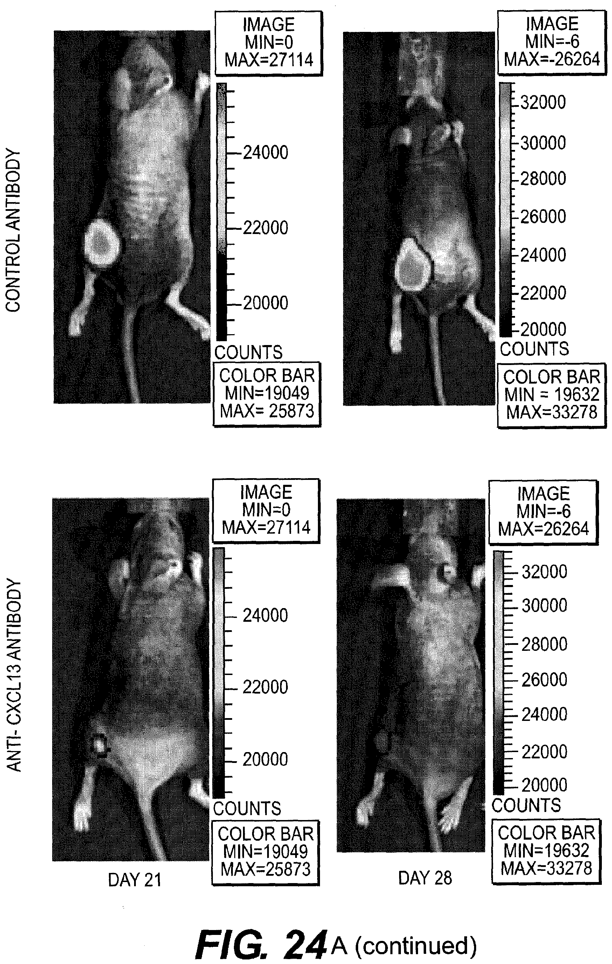

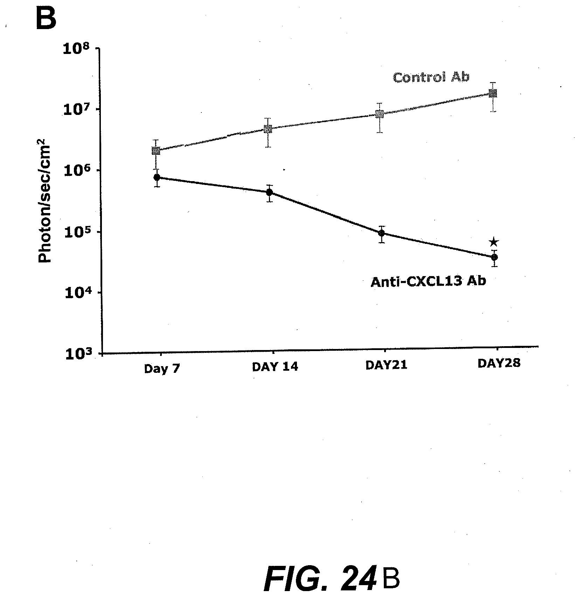

FIGS. 24A-B show that CXCL13 blockade inhibits prostate tumor growth in bone. Male Nu/Nu mice were intra-tibially injected with $10^6$ luciferase-positive PC3 (PC3-luc) cells and tumors were allowed to develop for one week. Subsequently, the mice were intraperitoneally injected with 475 μg/kg isotype control or anti-CXCL13 antibody suspended in 100 μl of sterile saline every 72 hours for one week. Experimental groups were imaged every week for four weeks using the Caliper/Xenogen In Vivo imaging system 100 and analyzed using the Caliper LIVING IMAGE® (Caliper, San Diego, CA) software. FIG. 24A displays representative images of PC3-luc tumor growth in bone. FIG. 24B shows luminescence (photons/sec/cm$^2$) ± SEM of PC3-luc tumors in bone 7, 14, 21, and 28 days post challenge. Asterisk (*) indicates significant ($p < 0.01$) differences between isotype control and anti-CXCL13 antibody-treated groups.

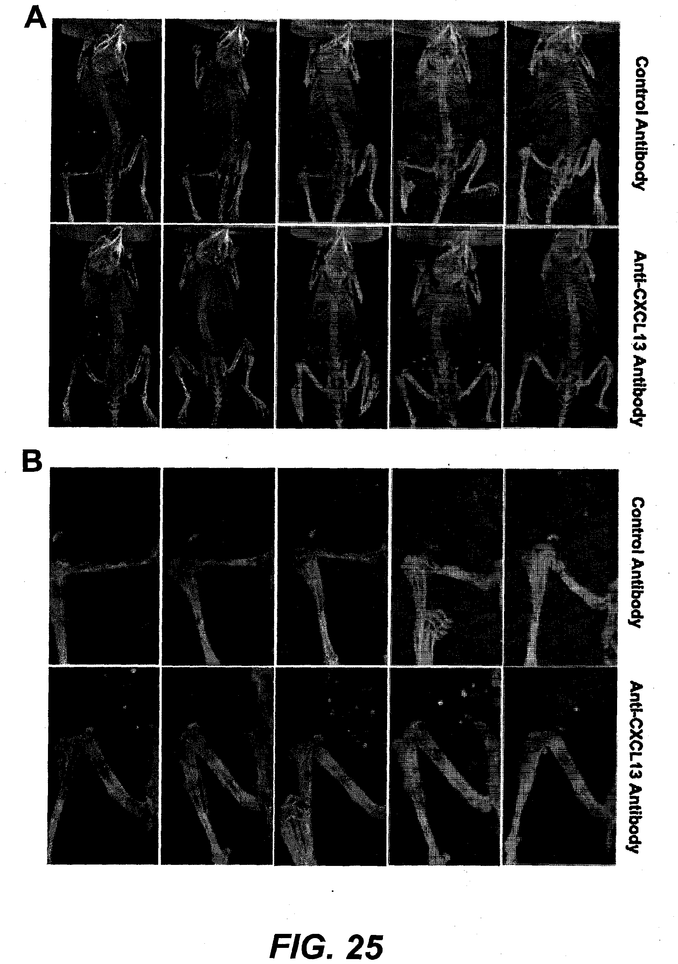

CXCL13 blockade also abrogates osteolytic prostate tumor growth in bone, as shown in FIGS. 25A-B. Male Nu/Nu mice were intra-tibially injected with $10^6$ luciferase-positive PC3 (PC3-luc) cells and tumors were allowed to develop for one week. Subsequently, mice were intraperitoneally injected with 475 μg/kg isotype control or anti-CXCL13 antibody suspended in 100 μl of sterile saline every 72 hours for one week. Experimental groups were imaged 28 days post challenge using a Siemens microCT Scan System. Low (FIG. 25A) and high (FIG. 25B) resolution images from five mice in each group were processed using OsiriX imaging software are shown.

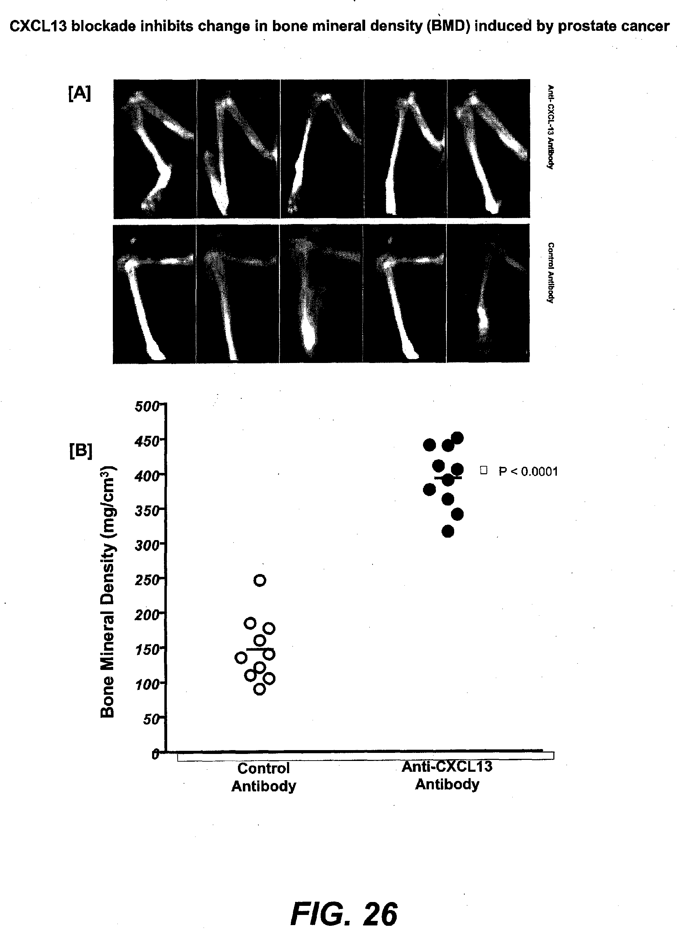

FIGS. 26A-B demonstrate that CXCL13 blockade inhibits loss of bone mineral density (BMD) induced by prostate cancer bone metastasis. Male Nu/Nu mice were intra-tibially injected with $10^6$ of luciferase-positive PC3 (PC3-luc) cell lines and tumors were allowed to develop for one week. Subsequently, the mice were intraperitoneally injected with 475 μg/kg isotype control or anti-CXCL13 antibody suspended in 100 μl of sterile saline every 72 hours for one week. Experimental groups were imaged 28 days post challenge using a Siemens microCT Scan System. FIG. 26A displays representative in mineral density images. FIG. 26B shows the femoral diaphysis BMD (mg/cm3) scans for each subject, which were quantified using MicroView software version 2.1.1 (General Electric Medical). Asterisk (*) indicates statistical significance ($p < 0.0001$) between isotype control or anti-CXCL13 antibody-treated group.

EXAMPLE 3: Detection of CXCL13 and CXCR5 Expression in Various Tumors

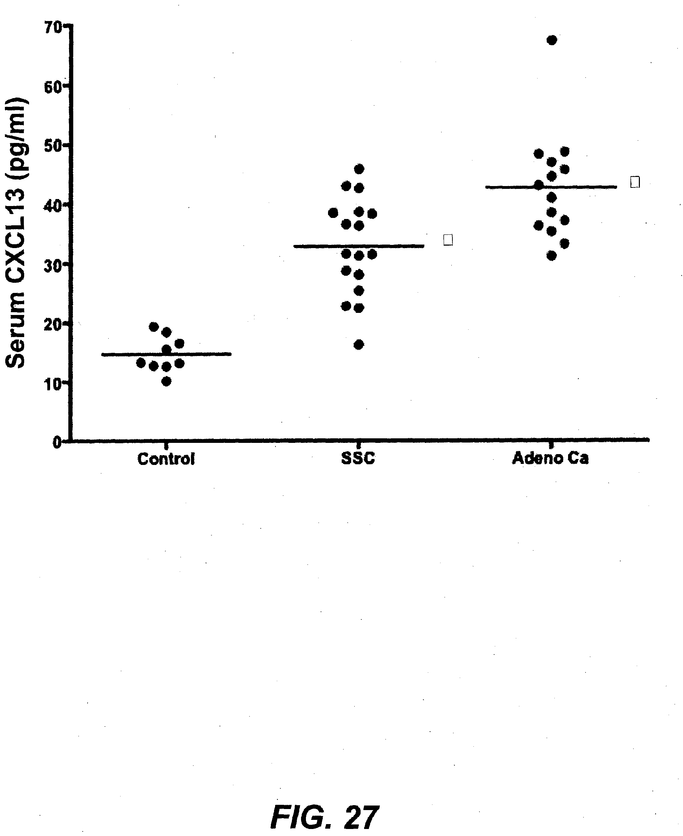

FIG. 27 shows CXCL13 levels in serum of normal healthy controls and lung cancer subjects. ELISA assays, capable of detecting >5 pg/mL of CXCL13, were performed to quantify CXCL13 levels in serum from normal healthy donors (n = 9) or patients diagnosed with squamous cell carcinoma (SSC; n=17) or adenocarcinoma (Adeno Ca; n=14). Solid circles indicate individual serum CXCL13 levels and lines show median concentrations for each group. Asterisks (*) show significant differences ($p < 0.01$) between normal healthy donor (*i.e.,* control) or lung cancer patient serum samples.

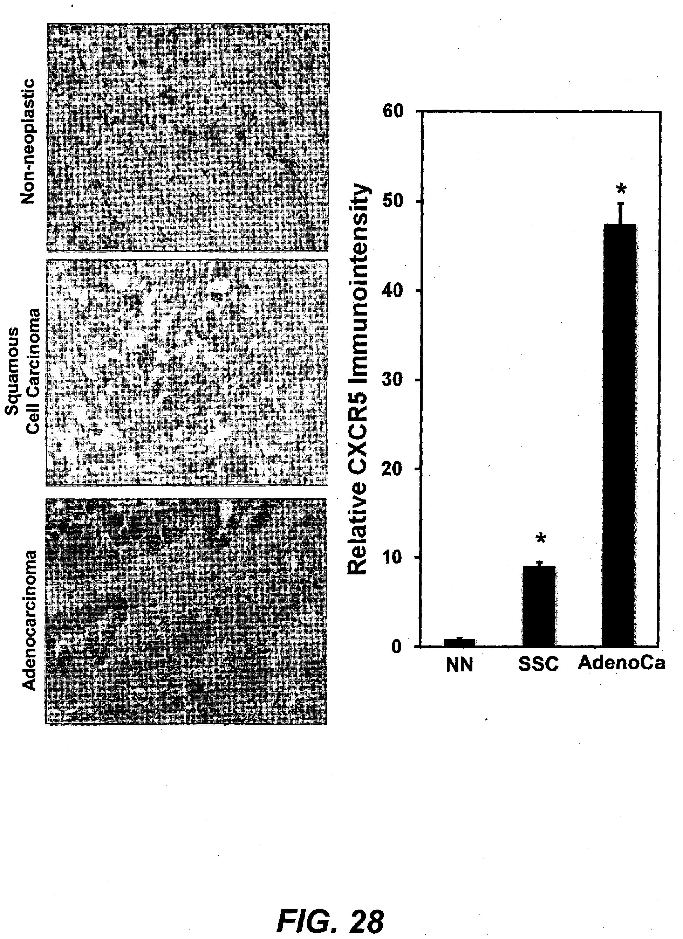

CXCR5 expression by non-neoplastic lung and lung cancer tissue is shown in FIG. 28. Lung tissue from non-neoplastic (NN; n=8), squamous cell carcinoma (SCC; n=24), and adenocarcinoma (AdenoCa; n=54) were stained with isotype control or anti-CXCR5 antibodies. Brown (DAB) color show CXCR5 staining. An Aperio ScanScope CS system with a 40X objective captured digital images of each slide. Representative cases are indicated and immuno-intensities of CXCR5 were quantified using image analysis Aperio ImageScope v.6.25 software. Asterisks (*) show significant differences ($p < 0.01$) between non-neoplastic and lung cancer tissue.

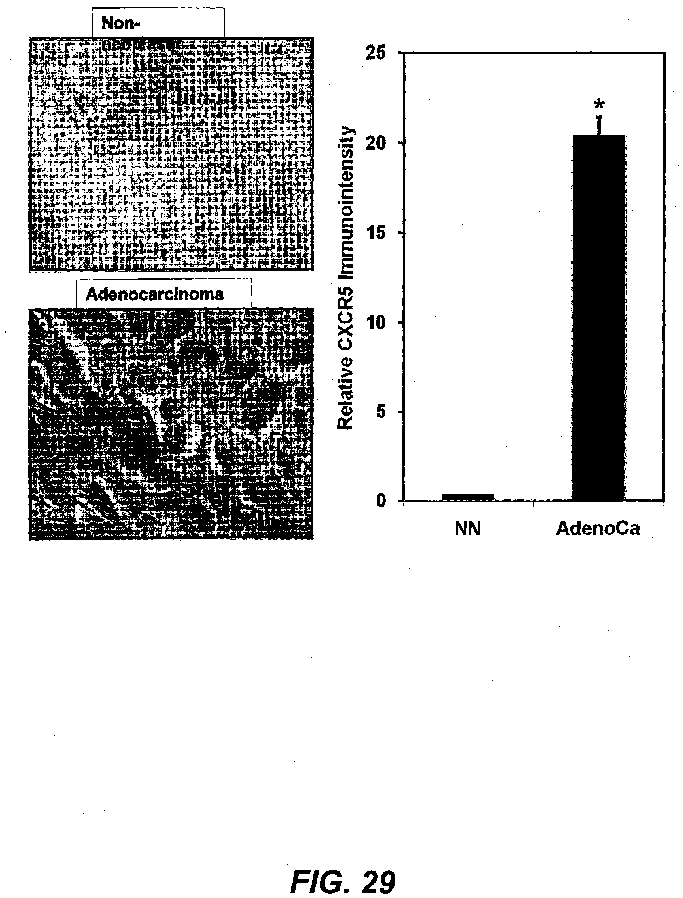

FIG. 29 illustrates CXCR5 expression by non-neoplastic mammary and breast cancer tissue. Breast tissue from non-neoplastic (NN; n=8) and adenocarcinoma (AdenoCa; n=16) were stained with isotype control or anti-CXCR5 antibodies. Brown (DAB) color show CXCR5 staining. An Aperio ScanScope CS system with a 40X objective captured digital images of each slide. Representative cases are indicated and immuno-intensities of CXCR5 were quantified using image analysis Aperio ImageScope v.6.25 software. Asterisks (*) show significant differences ($p < 0.01$) between non-neoplastic and cancerous tissue.

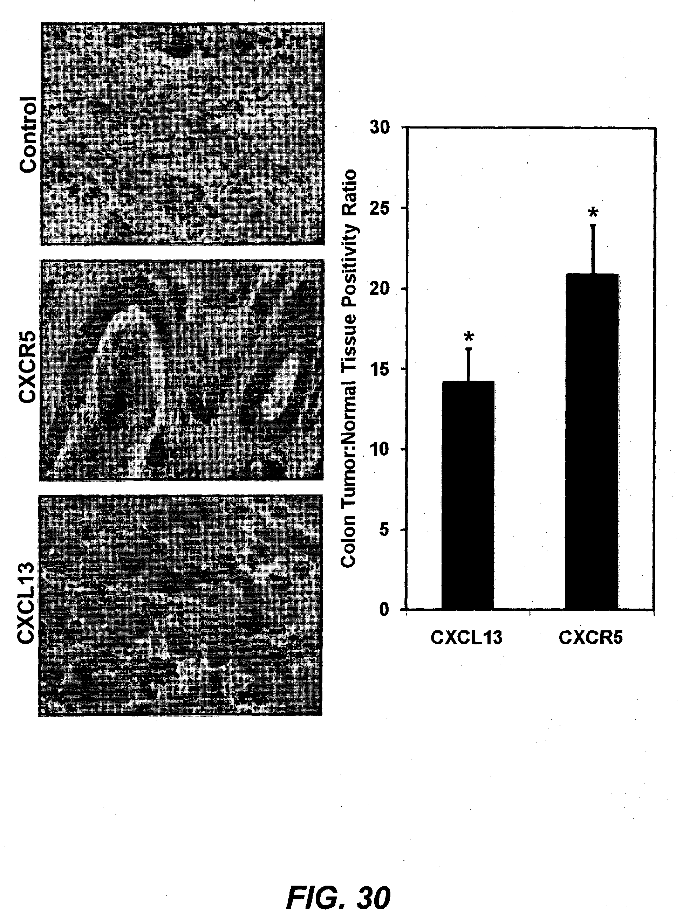

CXCR5 and CXCL13 expression are also increased in colon cancer tissue relative to non-neoplastic controls, as shown in FIG. 30. Colon tissue from non-neoplastic (n=8) and adenocarcinoma (n=16) were stained with isotype control, anti-CXCR5, or anti-CXCL13 antibody. Brown (DAB) and magenta stain indicates CXCR5 and CXCL13 positivity, respectively. An Aperio ScanScope CS system with a 40X objective captured digital images. Representative cases are shown along with relative colon cancer to non-neoplastic control tissue immuno-intensities ratios of CXCR5 and CXCL13 that were quantified using Aperio ImageScope v.6.25 software. Asterisks (*) show significant differences (p < 0.01) between non-neoplastic and cancerous tissue.

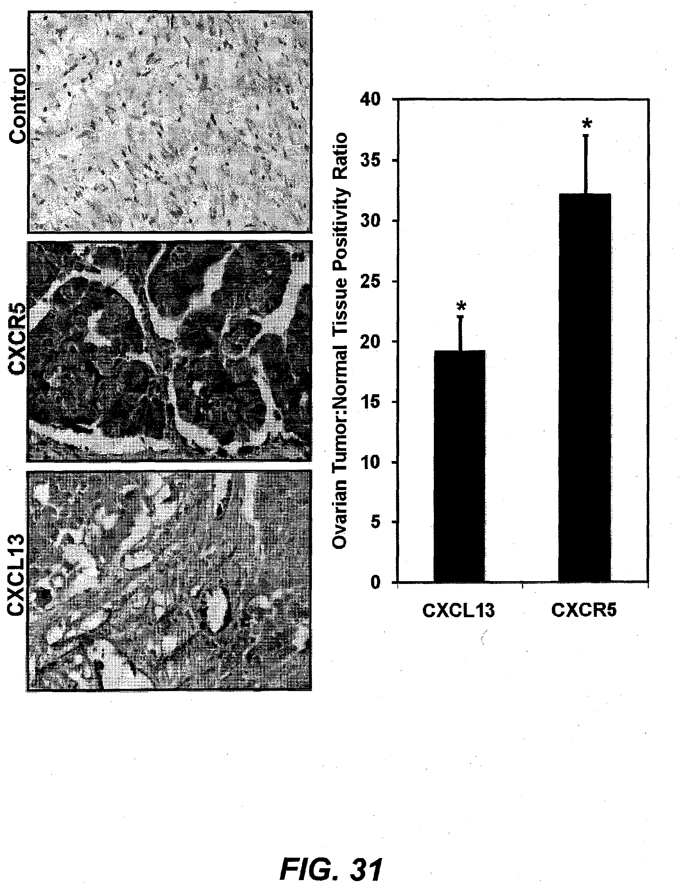

It was also found that CXCR5 and CXCL13 expression by ovarian cancer tissue relative to non-neoplastic controls is significantly higher, as shown in FIG. 31. Ovarian tissue from non-neoplastic (n=8) and adenocarcinoma (n=16) were stained with isotype control, anti-CXCR5, or anti-CXCL13 antibody. Brown (DAB) and magenta stain indicates CXCR5 and CXCL13 positivity, respectively. Aperio ScanScope CS system with a 40X objective captured digital images. Representative cases are shown along with relative ovarian cancer to non-neoplastic control tissue immuno-intensities ratios of CXCR5 and CXCL13 that were quantified using Aperio ImageScope v.6.25 software. Asterisks (*) show significant differences (p < 0.01) between non-neoplastic and cancerous tissue.

EXAMPLE 4: Detecting Chemokine Expression Levels With Real Time-PCR Analysis *Primer Design*

Messenger RNA sequences for CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR1, CXCR2, CXCR3, CXCR4, CXCR5, CXCR5a, CXCR5b, CXCR6, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, or CX3CL1 were obtained from the NIH-NCBI gene bank database. Primers were designed using the BeaconJ 2.0 computer program. Thermodynamic analysis of the primers was conducted using computer programs: Primer PremierJ and MIT Primer 3. The resulting primer sets were compared against the entire human genome to confirm specificity.

*Real Time PCR Analysis*

Cancer cell lines (ATCC, Rockville, MD) were cultured in RMPI-1640 containing 10% fetal calf serum supplemented with non-essential amino acids, L-glutamate, and sodium pyruvate (complete media). Primary tumor and normal-paired matched tissues were obtained from clinical isolates (Clinomics Biosciences, Frederick, MD and UAB Tissue Procurement, Birmingham, AL). Messenger RNA (mRNA) was isolated from 106 cells using TriReagent (Molecular Research Center, Cincinnati, OH) according to manufacturer's protocols. Potential genomic DNA contamination was removed from these samples by treatment with 10 U/F1 of RNase free DNase (Invitrogen, San Diego, CA) for 15 minutes at 37°C. RNA was then precipitated and resuspended in RNA Secure (Ambion, Austin, TX). The cDNA was generated by reverse transcribing approximately 2 $\mu$g of total RNA using Taqman7 reverse transcription reagents (Applied Biosystems, Foster City, CA) according to manufacturer's protocols. Subsequently, cDNAs were amplified with specific human cDNA primers, to CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR1, CXCR2, CXCR3, CXCR4, CXCR5, CXCR5a, CXCR5b, CXCR6, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, or CX3CL1, using SYBR7 Green PCR master mix reagents (Applied Biosystems) according to manufacturer's protocol. The level of copies of mRNA of these targets were evaluated by real-time PCR analysis using the BioRad Icycler and software (Hercules, CA).

The RT-PCR products obtained using CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR1, CXCR2, CXCR3, CXCR4, CXCR5, CXCR5a, CXCR5b, CXCR6, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, or CX3CL1 specific primer sets did not cross react with other gene targets due to exclusion of primers that annealed to host sequences (NIH-NCBI Genebank). The primers produced different size amplicon products relative the polymorphisms that resulted in CXCR5a versus CXCR5b and CCL25, CCL25-1, versus CCL25-2. To this end, RT-PCR analysis of adenoma, carcinoma, leukemia, lymphoma, melanoma, and/or myeloma cell lines and tumor tissue revealed that chemokines and chemokine receptors were differentially expressed by cancer cells.

EXAMPLE 5: Anti-chemokine and Anti-chemokine receptor Antibodies Inhibit Tumor Cell Growth *in vitro* and *in vivo*

*Anti-Sera Preparation*

The 15 amino acid peptides from CXCR1, CXCR2, CXCL1, CXCL2, CXCL3, CXCL5, CXCL6, CXCL7, CXCL8, CXCL12, CXCR5a, CXCR5b, CXCL13, CXCR6, CXCL16, CCL16, CCL25, CCL25-1, CCL25-2, CX3CR1, and CX3CL1 were synthesized (Sigma Genosys, The Woodlands, TX) and conjugated to hen egg lysozyme (Pierce, Rockford, IL) to generate the antigen for subsequent immunizations for anti-sera preparation or monoclonal antibody generation. The endotoxin levels of chemokine peptide conjugates were quantified by the chromogenic Limulus amebocyte lysate assay (Cape Cod, Inc., Falmouth,MS) and shown to be < 5 EU / mg. 100 μg of the antigen was used as the immunogen together with complete Freund's adjuvant Ribi Adjuvant system (RAS) for the first immunization in a final volume of 1.0 ml. This mixture was administered in 100 ml aliquots on two sites of the back of the rabbit subcutaneously and 400 ml intramuscularly in each hind leg muscle. Three to four weeks later, rabbits received 100 μg of the antigen in addition to incomplete Freund's adjuvant for 3 subsequent immunizations. Anti-sera were collected when anti -CXCR1, -CXCR2, -CXCL1, -CXCL2, -CXCL3, -CXCL5, - CXCL6 - CXCL7, -CXCL8, -CXCL12, -CXCR5a, -CXCR5b, -CXCL13, -CXCR6, -CXCL16, - CCL16, -CCL25, -CCL25-1, -CCL25-2, - CX3CR1, and -CX3CL1 antibody titers reached 1:1,000,000. Subsequently, normal or anti-sera were heat-inactivated and diluted 1:50 in PBS.

*Monoclonal Antibody Preparation*

The 15 amino acid peptides from CXCR1, CXCR2, CXCL1, CXCL2, CXCL3, CXCL5, CXCL6, CXCL7, CXCL8, CXCL12, CXCR5a, CXCR5b, CXCL13, CXCR6, CXCL16, CCL16, CCL25, CCL25-1, CCL25-2, CX3CR1, and CX3CL1 were synthesized (Sigma Genosys) and conjugated to hen egg lysozyme (Pierce) to generate the "antigen" for subsequent immunizations for anti-sera preparation or monoclonal antibody generation. The endotoxin levels of chemokine peptide conjugates were quantified by the chromogenic Limulus amebocyte lysate assay (Cape Cod, Inc., Falmouth,MS) and shown to be < 5 EU / mg. 100 μg of the antigen was used as the immunogen together with complete Freund's adjuvant Ribi Adjuvant system (RAS) for the first immunization in a final volume of 200 μl. This mixture was subcutaneously administered in 100 μl aliquots at two sites of the back of a rat, mouse, or immunoglobulin-humanized mouse. Two weeks later, animals received 100 μg of the antigen in addition to incomplete Freund's adjuvant for 3 subsequent immunizations. Serum were collected and when anti -CXCR1, -CXCR2, -CXCL1, -CXCL2, -CXCL3, -CXCL5, -CXCL6 -CXCL7, -CXCL8, -CXCL12, -CXCR5a, -CXCR5b, -CXCL13, -CXCR6, -CXCL16, -CCL16, -CCL25, -CCL25-1, -CCL25-2, -CX3CR1, or -CX3CL1 antibody titers reached 1:2,000,000, hosts were sacrificed and splenocytes were isolated for hybridoma generation. Briefly, B cells from the spleen or lymph nodes of immunized hosts were fused with immortal myeloma cell lines (*e.g.,* YB2/0). Hybridomas were next isolated after selective culturing conditions (*i.e.,* HAT-supplemented media) and limiting dilution methods of hybridoma cloning. Cells that produce antibodies with the desired specificity were selected using ELISA. Hybridomas from normal rats or mice were humanized with molecular biological techniques in common use. After cloning a high affinity and prolific hybridoma, antibodies were isolated from ascites or culture supernatants and adjusted to a titer of 1:2,000,000 and diluted 1:50 in PBS.

*Anti-sera or Monoclonal Antibody Treatment*

Immunodeficient nude NIH-III mice (8 to 12 weeks old, Charles River Laboratory, Wilmington, MA), which lack T, B, and NK cells, received $1 \times 10^6$ cancer cells, subcutaneously, for the establishment of a tumor. Correspondingly, freshly isolated or liquid nitrogen frozen 1g of tumor tissue were surgically implanted in the intestinal adipose tissue for the generation of tumor. Once the xenografted tumor growth reached 5 mm in size, the NIH-III mice received 200 μl intraperitoneal injections of either anti-sera or monoclonal antibodies every three days and the tumor was monitored for progression or regression of growth.

*Data Analysis*

SigmaStat 2000 (Chicago, IL) software was used to analyze and confirm the statistical significance of data. The data were subsequently analyzed by the Student's t- test, using a two-factor, unpaired test. In this analysis, treated samples were compared to untreated controls. The significance level was set at $p < 0.05$.

*In vitro Growth Studies*

The adenoma, carcinoma, leukemia, lymphoma, melanoma, and/or myeloma cell lines were grown in complete media in the presence or absence of antibodies specific for CXCR1, CXCR2, CXCL1, CXCL2, CXCL3, CXCL5, CXCL6

CXCL7, CXCL8, CXCR4, CXCL12, CXCR5a, CXCR5b, CXCL13, CXCR6, CXCL16, CCL16, CCR9, CCL25, CCL25-1, CCL25-2, CX3CR1, or CX3CL1. The growth of cancer cell lines expressing CXCR1 and/or CXCR2 were inhibited by antibodies to CXCR1, CXCR2, CXCL1, CXCL2, CXCL3, CXCL5, CXCL6, CXCL7, or CXCL8. Similarly, the growth of cancer cell lines expressing CXCR4 were inhibited by antibodies to CXCR4 or CXCL12. The growth of cancer cell lines expressing CXCR5a or CXCR5a were inhibited by antibodies to CXCR5a, CXCR5b, or CXCL13. The proliferation of cancer cell lines expressing CXCR6 were inhibited by antibodies to CXCR6 or CXCL16. The growth of cancer cell lines expressing CCR9 were inhibited by antibodies to CCR9, CCL25, CCL25-1, or CCL25-2. The propagation of cancer cell lines expressing CX3CR1 were inhibited by antibodies to CX3CR1 or CXC3L1. Of interest, antibodies against the soluble ligands, CXCL1, CXCL2, CXCL3, CXCL5, CXCL6, CXCL7, CXCL8, CXCL12, CXCL13, CXCL16, CCL16, CCL25, CCL25-1, CCL25-2, or CX3CL1, were more effective at growth inhibition that those directed against the membrane receptors.

*In vitro Angiogenesis Studies*

Microvascular endothelial cells (Cell Systems, Kirkland, WA) were grown according to supplier's protocols and allowed to form microvascular venules in an *in vitro* assay for angiogenesis (BD-Biocoat, Hercules, CA), in the presence or absence of antibodies specific for CXCR1, CXCR2, CXCL1, CXCL2, CXCL3, CXCL5, CXCL6, CXCL7, CXCL8, CXCR4, CXCL12, CXCR5a, CXCR5b, CXCL13, CXCR6, CXCL16, CCL16, CCR9, CCL25, CCL25-1, CCL25-2, CX3CR1, or CX3CL1. The angiogenesis was inhibited by antibodies against CXCR1, CXCR2, CXCL1, CXCL2, CXCL3, CXCL5, CXCL6, CXCL7, CXCL8, CXCR4, CXCL12, CXCR6 or CXCL16.

*In vivo Growth Studies*

Cancer cell lines or primary tumor tissue were adoptively transferred into NIH-III mice and allowed to form the xenograft tumor of interest. Antibodies directed against CXCR1, CXCR2, CXCL1, CXCL2, CXCL3, CXCL5, CXCL6, CXCL7, CXCL8, CXCR4, CXCL12, CXCR5a, CXCR5b, CXCL13, CXCR6, CXCL16, CCL16, CCR9, CCL25, CCL25-1, CCL25-2; CX3CR1, or CX3CL1 differentially affected the progression and regression of tumor size. In certain cases, antibodies directed towards CXCR1, CXCR2, CXCL1, CXCL2, CXCL3, CXCL5, CXCL6, CXCL7, CXCL8, CXCR4, CXCL12, CXCR6 or CXCL16 effectively lead to both regression and impeding progression of tumor growth. Antibodies directed against CXCR4, CXCL12, CXCR5a, CXCR5b, CXCL13, CCL16, CCR9, CCL25, CCL25-1, CCL25-2, CX3CR1, or CX3CL1 were effective at inhibiting the progression of tumor size.

The protein sequences of the chemokines used herein are recorded in NIH-NCBI GenBank as: (1) CXCR1 (ACCESSION# NP 000625), SEQ ID NO:1, (2) CXCR2(ACCESSION# NP 001548), SEQ ID NO:2, (3) CXCL1 (ACCESSION# NP 001502), SEQ ID NO:3, (4) CXCL2 (ACCESSION# NP 002080), SEQ ID NO:4, (5) CXCL3 (ACCESSION# NP 002081), SEQ ID NO:5, (6) CXCL5 (ACCESSION# NP 002985), SEQ ID NO:6, (7) CXCL6 (ACCESSION# NP 002984), SEQ ID NO:7, (8) CXCL7 (ACCESSION# NP 002695). SEQ ID NO:8, (9) CXCL8 (IL-8, ACCESSION# NP 000575), SEQ ID NO:9, (10) CXCR4 (ACCESSION# NP 003458), SEQ ID NO:10, (11) CXCL12 (ACCESSION# NP 000600), SEQ ID NO:11, (12) CXCR5A (ACCESSION# NP 116743), SEQ ID NO:12, (13) CXCR5B (ACCESSION# NP 001707), SEQ ID NO:13, (14) CXCL13 (ACCESSION# NP 006410), SEQ ID NO:14, (15) CXCR6 (ACCESSION# NP 006555), SEQ ID NO:15, (16) CXCL16 (ACCESSION# NP 071342), SEQ ID NO:16, (17) CCL16 (ACCESSION# NP 004581), SEQ ID NO:17, (18) CCL25 (ACCESSION# NP-005615.2), SEQ ID NO:18, (19) CCL25-1 (ACCESSION# NP 005615), SEQ ID NO:19, (20) CCL25-2 (ACCESSION# NP 683686), SEQ ID NO:20, (21) CX3CR1 (ACCESSION# NP 001328), SEQ ID NO:21, and (22) CX3CL1 (ACCESSION# NP 002987), SEQ ID NO:22.

The cDNA sequences are known and are available in NIH-NCBI GenBank under the following accession numbers: (23) CXCR1 (ACCESSION# NM 000634), SEQ ID NO:23, (24) CXCR2(ACCESSION# NM 001557), SEQ ID NO:24, (25) CXCL1 (ACCESSION# NM 001511), SEQ ID NO:25, (26) CXCL2 (ACCESSION# NM 002089), SEQ ID NO:26, (27) CXCL3 (ACCESSION# NM 002090), SEQ ID NO:27, (28) CXCL5 (ACCESSION# NM 002994), SEQ ID NO:28, (29) CXCL6 (ACCESSION# NM 002993), SEQ ID NO:29, (30) CXCL7 (ACCESSION# NM 002704), SEQ ID NO:30, (31) CXCL8 (IL-8, ACCESSION# NM 000584), SEQ ID NO:31, (32) CXCR4 (ACCESSION# NM 003467), SEQ ID NO:32, (33) CXCL12 (ACCESSION# NM 000609), SEQ ID NO:33, (34) CXCR5A (ACCESSION# NM 032966), SEQ ID NO:34, (35) CXCR5B (ACCESSION# NM 001716), SEQ ID NO:35, (36) CXCL13 (ACCESSION# NM 006419), SEQ ID NO:36, (37) CXCR6 (ACCESSION# NM 006564), SEQ ID NO:37, (38) CXCL16 (ACCESSION# NM 022059), SEQ ID NO:38, (39) CCL16 (ACCESSION# NM 004590), SEQ ID NO:39, (40) CCL25 (ACCESSION# NM_005624.3), SEQ ID NO:40, (41) CCL25-1 (ACCESSION# NM 005624), SEQ ID NO:41, (42) CCL25-2 (ACCESSION# NM 148888), SEQ ID NO:42, (43) CX3CR1 (ACCESSION# NM 001337), SEQ ID NO:43, and (44) CX3CL1 (ACCESSION# NM 002996), SEQ ID NO:44.

As shown in the table below, the particular chemokines which are most which any tumor expresses may vary. The methods of the present application may be customized for a particular patient, depending on the chemokines over-expressed by the patient's own tumor. It is possible to identify the particular chemokines which are over-expressed in the tumor using methods of the application and administer antibodies against that over-expressed chemokine. The tailoring of treatment for the cancer patient is novel, and is a particularly valuable aspect of the application.

The table on the following page indicates the differing amounts of particular chemokines over-expressed in particular tumors that were studied.

TABLE 3. Chemokine, Chemokine Receptor and Cancer Association (dependent on stage of disease).

| Cancer | Chemokine | Chemokine Receptor |
|---|---|---|
| Carcinoma | CCL1, CCL2, CCL4, CCL17, CCL19, CCL21, CCL22, CCL25<br>CXCL12, CXCL13, CXCL16 | CCR2, CCR7, CCR8, CCR9<br><br>CXCR4, CXCR5, CXCR6 CX3CR1 |
| Leukemia | CCL1, CCL4, CCL17, CCL19, CCL21, CCL22, CCL25<br>CXCL12 | CCR7, CCR8, CCR9<br><br>CXCR4, CXCR7 |
| Lymphoma | CXCL12, CXCL13 | CXCR4, CXCR5 |
| Melanoma | CCL25, CCL27<br>CXCL1, CXCL2, CXCL3, CXCL5, CXCL6, CXCL7, CXCL8, CXCL12,<br>CXCL13, CXCL16<br>CX3CL1 | CCR9, CCR10<br>CXCR1, CXCR2, CXCR4, CXCR5, CXCR6, CXCR7<br><br>CX3CR1 |
| Sarcoma | CCL1, CCL3, CCL4, CCL5, CCL7, CCL8, CCL11, CCL13, CCL17,<br>CCL22, CCL24<br>CXCL12<br>CX3CL1 | CCR3, CCR5, CCR8<br><br><br>CXCR4, CXCR7<br>CX3CR1 |

### EXAMPLE 6: CXCR5-CXCL13 Induced Anti-Apoptotic and/or Survival Signal Involved in PCa Chemo Resistance

LNCaP (hormone responsive, wild type p53 expression), PC3 (hormone refractory, p53 null), and DU145 (hormone refractory, p53 mutated) cell lines are grown with or without CXCL13 and with or without doxorubicin ($1\mu M/2\mu M/4\mu M$), etoposide ($20\mu M/40\mu M$), estramustine ($4\mu M/10\mu M$), or docetaxel (10nM/20nM/40nM) for 4, 8, 12, and 24 hours. Expression and activation of cell survival, pro- and anti-apoptotic signals (Akt, Src, CamKII, FAK, FKHR, FOXO, CREB, NF-$\kappa$B, Myc, Fos, Jun Apafl, Bax, Bcl2, BclX$_L$, BaK, Bad, Bik, Bim, TP53, Caspase-3, -6, -8, -9, survivin, vitronectin, $\beta$-Catenin) and molecules responsible for drug resistance or metabolism (Twist-1, Snail-1, Glutathione-S-transferase-$\pi$ (GST-$\pi$), p53, topoisomerase I, II$\alpha$, II$\beta$, and ABC drug transporters) are accessed by real-time PCR and Western blot. Briefly, after treatment of cells, changes in the gene expression is tested using real-time PCR. Activation of signaling molecules is also be tested by phosphorylation specific antibody (i.e., Western blot analysis). To further confirm the role of the activated signaling molecules, following CXCL13 treatment, expression or activity of the candidate molecules is inhibited using chemical inhibitors or siRNAs and target genes are analyzed by real-time PCR and Western blot analysis. Subsequently, the response of treated cells to chemotherapeutic drugs is evaluated by Vybrant apoptosis assay (Molecular probes) kit.

*RNA Isolation and Real-Time PCR*

Total RNA is isolated by TRIZOL™ (Invitrogen) method and quantified by UV spectrophotometry. Quality of RNA is analyzed by electrophoresis. The cDNA synthesis is completed using the ISCRIPT™ cDNA synthesis kit (BioRad) as described by the manufacturer. Real-time PCR is performed using IQ™ SYBR green supermix (BioRad) as described by manufacturer and specific primers designed against FAK, FKHR, FOXO, Apafl, Bax, Bcl2, BclX$_L$, BaK, Bad, Bid, XIAP, Bik, Bim, TP53, cytochrome C, Caspase-3, -6, -8, -9, survivin, lamin, CamKII, vitronectin, $\beta$-Catenin, cadherins, Twist-1, Snail-1, CREB, NF-$\kappa$B, Myc, Fos, Jun, $\beta$-actin and GAPDH. The results are calculated by delta delta Ct to quantify fold changes in mRNAs compared to untreated groups.

*Western Blotting*

Cells are harvested and resuspended in lysis buffer to extract total protein. Lysis buffer contains 50 mM Tris-HCl, pH 7.4, 150 mM NaCl, 1% Triton X-100, 1% deoxycholate, 0.1% SDS, 5 mM EDTA supplemented with protease inhibitors, 1 mM phenylmethylsulphonylfluoride, 1mM benzamidine, 10$\mu$g/mL soybean trypsin inhibitor, 50 $\mu$g/mL leupeptin, 1 $\mu$g/mL pepstatin and 20$\mu$g/mL aprotinin. Cell lysates are stored on ice for 30 min, centrifuged (14000xg) for 20 min at 4°C, and supernatant is used for Western blot analysis of genes demonstrating significant modulation in mRNA level. Similarly, phosphor-specific antibodies are used to test changes in the level of phophorylation of Akt1/2/3, mTOR, FAK, FKHR, FOXO, and GSK-3$\beta$. Moreover, activation of caspases and PARP, following cleavage are evaluated using specific antibodies. The results obtained after chemiluminescent detection of protein bands by ECL plus reagent (Pharmecia) on X-ray film is normalized to $\beta$-actin and/or GAPDH using Image J image analysis software (NIH).

*Detection of Cytochrome C Release*

Cells are collected and washed in PBS, and resuspended in extraction buffer containing 220 mM mannitol, 68 mM sucrose, 50 mM PIPES-KOH, pH 7.4, 50 mM KCl, 5 mM EGTA, 2 mM MgCl$_2$, 1 mM DTT, and protease inhibitors. After 30 min incubation on ice, cells are homogenized using Glass-Teflon homogenizer and homogenates will be spun at 14,000g for 15 min. Cytosolic extracts are used for Western blot analysis using anti-cytochrome C monoclonal antibody (PharMingen).

*siRNA Transfection, Chemical Inhibitor, and Apoptosis Detection*

Prostate cancer cell lines are transfected with gene specific and nonspecific control siRNAs (Dharmacon) using LipofectAMINE 2000 (Invitrogen). Optimum gene knock-down time and siRNA concentration are confirmed by western blot analysis and further evaluated for cell survival following drug treatment with or without CXCL16, control antibody, and/or anti-CXCR6 antibody. The detection of changes in live, apoptotic, and necrotic cells is evaluated as follows: cell survival is tested by Vybrant apoptosis as described by the manufacturer (Molecular probe), using FACScan flow cytometer and CellQuest™ software (BD Pharmingen). Change in down-stream gene expression after gene knockdown is tested using real-time PCR and Western blotting.

Cells treated with CXCL16 show enhanced expression of cell survival and drug transporter proteins which show differences in their expression pattern in hormone responsive and non responsive cells. Anti-CXCL16 Abs effectively reverse the effect of CXCL16 in PCa cells. Doxorubicin, estramustine, etoposide and docetaxel induce apoptosis in PCa cells without CXCL16 treatment (or CXCR6 blockade).

EXAMPLE 7: CXCR5-CXCL13 Induced Changes in ABC Drug Transporters

LNCaP, PC3, and DU145 cells are grown with or without CXCL13, control antibody, and/or anti-CXCR5 antibodies along with or without doxorubicin, estramustine, etoposide or docetaxel for 4, 8, 12 or 16 hours as described earlier. After treatment, changes in the ABC transporter and Twist-1 mRNA expression are quantified by real-time PCR, as described above, using specific primers directed for ABC and Twist-1 cDNA. The genes demonstrating significant alterations in mRNA expression are further tested by Western blot analysis. Nuclear extracts from treated cells are evaluated by chromatin immuno-precipitation (ChIP) assay to determine whether the transcriptional factors induced by CXCL13 bind the promoter region of ABC transporters and Twist-1.

*Chromatin Immuno-Precipitation (ChIP)*

The results from Example 6 provide information about the genes that are regulated as well as those that may modulate transcription factors activated by CXCR5-CXCL13 interaction. Based on these results, target transcription factors and genes are selected. Specific PCR primers are designed against the promoter region of these genes containing the binding sites of transcription factors. PCR primer are used to amplify the DNA being precipitated along with transcription factors. Cells are harvested by trypsinization in the presence of 20 mM butyrate. 50,000 cells are re-suspended in 500 $\mu$l PBS/butyrate. Proteins and DNA are cross-linked with 1% formaldehyde for 8 min at room temperature and cross-linking is stopped with 125 mM glycine for 5 min. Cells are centrifuged at 470g in a swing-out rotor with soft deceleration settings for 10 min at 4°C and washed twice in 0.5 ml ice-cold PBS/butyrate by vortexing followed by centrifugation. Cells are lysed by addition of lysis buffer (50 mM Tris-HCl, pH 8, 10mM EDTA, 1% SDS, protease inhibitor cocktail (Sigma-Aldrich), 1 mM PMSF, 20 mM butyrate, vortexing and subsequent centrifugation. This procedure is known to produce chromatin fragments of 500 bp. The sonicated lysate is diluted 8-fold in RIPA buffer containing a protease inhibitor cocktail, 1 mM PMSF, and 20 mM butyrate (RIPA ChIP buffer). RIPA ChIP buffer (330 $\mu$l) is added

to the pellet and mixed by vortexing. Immunoprecipitation and washes of the ChIP material is accomplished by the use of antibody-directed against specific transcription factors. Chromatin is aliquoted into tubes containing antibody-bead complexes. Input sample is placed in a tube for phenol-chloroform isoamyl alcohol isolation. The immunoprecipitated material is washed three times and transferred into a new tube while in TE. DNA elution in 1% SDS, cross-link reversal and proteinase K digestion is carried out in a single step for 2 h at 68°C. DNA is extracted with phenol-chloroform isoamylalcohol, and ethanol-precipitation in presence of acrylamide carrier (Sigma-Aldrich) and dissolved in TE. Immunoprecipitated DNA from 3-4 independent ChIPs is analyzed by real time PCR. Real-time PCR data is expressed as percent ($\pm$SD) precipitated (antibody-bound) DNA relative to input DNA, in three independent replicate ChIP assays.

Phosphorylation and activation of transcription factors such as CREB, Fos, Jun, and NFkB via CXCR5-CXCL13 signaling subsequently leads to increases in expression of ABC transporters and Twist-1. Decreases in gene expression are observed if negative regulatory elements are present in the same promoter. Since hormone-dependent and refractory PCa cells have differences in the expression of these intracellular signaling molecules, they show variations in genes to be modulated by hormone dependent and refractory conditions. The modulation in gene expression shows differences with drug treatment in presence of CXCL13 and in absence of CXCL13 treatment.

EXAMPLE 8: *In vivo* Evaluation of CXCL13-directed Therapy

Male nude mice are subcutaneously challenged by luciferase expressing androgen responsive (LNCaP-Luc) and non-responsive (PC3-Luc) cells. Tumor development is measured non-invasively using in vivo imaging system. After establishment of a measurable tumor, mice are divided into treatment (A, B, C, D and E) and control groups (F, G, H, I, J and K). Group "A" receives CXCL13 neutralizing antibodies (12.5 mg/kg/day) every alternate day and controls (group F) receive isotype control antibodies (12.5 mg/kg/day). Group "B," "C," "D" and "E" receive CXCL13 neutralizing antibodies (12.5 mg/kg/day) with intraperitoneal injection of doxorubicin (5 mg/kg/day on days 1 to 3 followed by administration on days 15 to 17), intravenous injection of etoposide (10 mg/kg/day; on day 1, 5, 9, 14, 19 and 24), intravenous injection of estramustine (4 mg/kg/day on day 1-5 and day 26-31), or intraperitoneal injection of docetaxel (8 mg/kg/day twice a week for 4 weeks), respectively. Controls for these treatment groups ("G," "H," "I" and "J," respectively) receive these drugs using similar concentration and injection protocol with isotype control antibodies (12.5 mg/kg/day). Group "K" receives PBS and serves as placebo. Tumor progression and regression in treatment and controls are evaluated by non-invasive in vivo imaging. The tumor from treated groups and untreated control groups is excised and evaluated for the changes in the cell survival and drug resistance proteins by immunohistochemistry.

*Statistics (Significance) and Sample Size*

Sample size (or power) calculations are relevant to the design of preliminary studies and determining the requirements for proposed experiments. To interpret our results, significance tests and statistical analysis are also critical. The traditional $\alpha$-value, *i.e.,* p = 0.01, is used to evaluate the statistical significance of this study. The proposed experiment will require a minimum of 10 mice per group. The data is expressed as the mean $\pm$ SEM and compared using a two-tailed paired (or unpaired) student's t-test for normally distributed samples or an unpaired Mann Whitney U test as a non-parametric test for samples not normally distributed. The results are analyzed using SYSTAT (Systat software Inc.) statistical program. Single-factor and two-factor variance ANOVA analyses are used to evaluate groups and subgroups, respectively. Hence, results are considered statistically significant if p values are < 0.05.

*Animals*

Six to eight week old male nude mice are subcutaneously injected with PCa cells. Briefly, $5 \times 10^6$ Luciferase expressing PC3 cells are resuspended in 100$\mu$l of sterile PBS and injected into the flanks of nude mice under isoflurane anesthesia. Luciferase expressing LNCaP cells ($5 \times 10^6$ cell) are mixed with 50% Matrigel (Becton Dickinson) and injected in the flanks of nude mice under isoflurane anesthesia.

*Analysis of In Vivo Tumor Growth*

Tumor bearing nude mice receive 150 mg/kg D-Luciferin (Xenogen) by intraperitoneal injection Using 25x5/8" gauge needle 15 minutes before imaging. The mice are imaged using the IVIS100 *in vivo* imaging system and results expressed in photons/sec/cm$^2$/sr. Tumor volume is measured by use of calipers and calculated by the formula (Larger diameter) x(smaller diameter)$^2$x 0.5.

*Cell Survival, Apoptotic and Drug Resistant Gene Expression Analysis*

Tumors from all groups are excised three days after completion of treatment protocols. Tumors are fixed in 4% PFA and embedded in paraffin. Paraffin sections (thickness 7μm) are mounted on glass slides, deparaffinized and re-hydrated (Xylene for 5 min; absolute, 95% and 70% ethanol for 1 min each). The rehydrated sections are used for peroxidase based immunohistochemical staining for drug transporters, PI3K, Akt, FAK, FKHR, FOXO, Apafl, Bax, Bcl2, BclX$_L$, BaK, Bad, Bid, XIAP, Bik, Bim, TP53, Cytochrome C, Caspase-3, -6, -8, -9, survivin, lamin, CamKII, vitronectin, β-Catenin, cadherins, Twist-1, CREB, NF-κB, Myc, Fos, Jun, CXCR5 and CXCL13. After staining, slides are scanned and analyzed by the Aperio scanscope (Aperio) system.

CXCL13 neutralization leads to decreased cell survival in response to drugs, thus reduction of tumor volume. However, the response also varies among the tumors formed by hormone sensitive (LNCaP) and hormone refractory (PC3 cells). Further, chemotherapeutic drugs have lower efficacy in the tumors with a functional CXCR5-CXCL13 axis, which may enhance the expression of ABC proteins known to transport these drugs out of the cell.

The above description is for the purpose of teaching the person of ordinary skill in the art how to practice the present invention, and is not intended to detail all those obvious modifications and variations of it that will become apparent to the skilled worker upon reading the description. The claims are intended to cover the components and steps in any sequence that is effective to meet the objectives there intended, unless the context specifically indicates the contrary.

**Claims**

**1.** A method for detecting the presence of cancer in a subject, comprising: detecting the level of expression of one or more cancer markers in a biological sample obtained from said subject; and

comparing the level of expression of said one or more cancer markers in said biological sample to a normal level of expression of said one or more cancer markers,

wherein a higher than normal level of expression of said one or more cancer markers in said biological sample is indicative of the presence of cancer in said subject,

wherein said normal level of expression of said one or more cancer markers is a predetermined value or is obtained from a control sample of known normal non-cancerous cells of the same origin or type as said biological sample, and

wherein said cancer is ovarian cancer, prostate cancer, colon cancer, breast cancer, or lung cancer, and

wherein said one or more cancer markers comprises CXCL13 or CXCR5 or both CXCL13 and CXCR5.

**2.** The method of Claim 1, wherein:

said one or more cancer markers further comprises CXCL16 or CXCR6 or both CXCL16 and CXCR6 and optionally wherein said one or more cancer markers further comprises one or more cancer markers selected from the group consisting of CXCL1, CXCL2, CXCL3, CXCL4, CXCL5, CXCL6, CXCL7, CXCL8, CXCL9, CXCL10, CXCL11, CXCL12, CXCL14, CXCL15, CXCR1, CXCR2, CXCR3, CXCR4, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, CX3CL1, RNA binding motif 3 ("RBM3"), carcinoembryonic antigen (CEA), prostate specific antigen (PSA), chromogranin A (CGA), dehydroepiandrosterone (DHEA), neuron-specific enolase (NSE), prostatic acid phosphatase (PAP), prolactin, B7-H3, seprase polypeptide, anti-p53, osteopontin, ferritin, lysophosphatidyl choline, kinesin family member 4A (KIF4A), Neural pentraxin I (NPTX1) and fibroblast growth factor receptor 1 oncogene partner (FGFRIOP) protein; or

wherein said biological sample is a plasma sample, a saliva sample, or an urine sample.

**3.** The method of Claim 1,

wherein said cancer is breast cancer and wherein one or more cancer markers further comprises one or more cancer markers selected from the group consisting of CXCL16, CXCR6, CCL1, CCL4, CCL17, CCL19, CCL21, CCL22, CCL25, CXCL12, CCR7, CCR8, CCR9, CXCR4, CX3CR1, RNA binding motif 3 ("RBM3") and carcinoembryonic Antigen (CEA)

wherein said cancer is prostate cancer and wherein one or more cancer markers further comprises one or more cancer markers selected from the group consisting of CXCL16, CXCR6, CCL1, CCL4, CCL17, CCL19, CCL21, CCL22, CCL25, CXCL12, CCR7, CCR8, CCR9, CXCR4, CX3CR1, PSA, CEA, CGA, DHEA, NSE, PAP, prolactin and B7-H3; or

wherein said cancer is colorectal cancer and wherein one or more cancer markers further comprises one or more cancer markers selected from the group consisting of CXCL16, CXCR6, CCL1 , CCL4, CCL17, CCL19, CCL21 , CCL22, CCL25, CXCL12, CCR7, CCR8, CCR9, CXCR4, CX3CR1, seprase polypeptide, anti-p53, osteopontin, and ferritin; or

wherein said cancer is ovarian cancer and wherein one or more cancer markers further comprises one or more cancer markers selected from the group consisting of CXCL16, CXCR6, CCL1 , CCL4, CCL17, CCL19, CCL21 , CCL22, CCL25, CXCL12, CCR7, CCR8, CCR9, CXCR4, CX3CR1, cancer antigen 125 (CA-125), HE-4, OVX-1 macrophage colony stimulating factor (M-CSF) and lysophosphatidyl choline

wherein said cancer is lung cancer and wherein one or more cancer markers further comprises one or more cancer markers selected from the group consisting of CXCL16, CXCR6, CCL1 , CCL4, CCL17, CCL19, CCL21 , CCL22, CCL25, CXCL12, CCR7, CCR8, CCR9, CXCR4, CX3CR1 , kinesin family member 4A (IF4A), Neural pentraxin I (NPTX1), fibroblast growth factor receptor 1 oncogene partner (FGFRI OP) protein and CEA.

4. The method of Claim 1

wherein a higher level of expression of said one or more cancer markers in the biological sample relative to said normal level indicates that the prognosis of said subject is poor,

wherein a lower or similar level of expression of said one or more cancer markers in said biological sample relative to said normal level indicates that the prognosis of said subject is good,

wherein a poor prognosis indicates that said cancer is of an aggressive or invasive type, wherein said cancer is ovarian cancer, prostate cancer, colon cancer, breast cancer, or lung cancer,

wherein said one or more cancer markers comprise CXCL13 or CXCR5 or both CXCL13 and CXCR5, and optionally wherein said one or more cancer markers further comprise CXCL16 or CXCR6 or both CXCL16 and CXCR6.

5. The method of Claim 1

wherein said normal level of said one or more cancer markers is a pre-treatment level of said one or more cancer markers in said subject or a predetermined reference value,

wherein said treatment is deemed efficacious if said one or more cancer markers in said one or more biological samples is similar to or lower than said normal level,

wherein said cancer is ovarian cancer, prostate cancer, colon cancer, breast cancer, or lung cancer,

wherein said one or more cancer markers comprise CXCL13 or CXCR5 or both CXCL13 and CXCR5 and optionally wherein said one or more cancer markers further comprise CXCL16 or CXCR6 or both CXCL16 and CXCR6.

6. A kit for detecting cancer or monitoring cancer progression, comprising:

reagents for determining expression of CXCL13 and/or CXCR5 in a biological sample; and instructions for how to use said reagents,

wherein said reagents comprise an anti-CXCL13 antibody, an anti-CXCR5 antibody, or both and wherein said cancer is ovarian cancer, prostate cancer, colon cancer, breast cancer, or lung cancer and

wherein the kit further comprises:

reagents for determining expression of CXCL16 and/or CXCR6 in a biological sample; and instructions for how to use said reagents,

wherein said reagents comprise an anti-CXCL16 antibody, an anti-CXCR6 antibody, or both.

7. Composition comprising a therapeutically effective amount of an anti-CXCL13 antibody, or an anti-CXCR5 antibody, or a combination thereof for use in a method for the treatment of cancer,

wherein said cancer is ovarian cancer, prostate cancer, colon cancer, breast cancer, lung cancer, and wherein said anti-CXCL13 antibody, or said anti-CXCR5 antibody, or said combination of anti-CXCL13 antibody and said anti-CXCR5 antibody is given in a dosage range of 1 ng/kg body weight/day to about 100 mg/kg body weight/day.

8. The composition for use according to Claim 7, wherein:

the composition further comprises one or more antibodies that bind specifically to one or more chemokines or chemokine receptors selected from the group consisting of CCL1, CCL2, CCL4, CCL17, CCL19, CCL21, CCL22, CCL25, CXCL12, CXCL16, CCR2, CCR7, CCR8, CCR9, CXCR4, CXCR6, CX3CL1 and CX3CR1; or

wherein the cancer results in elevated CXCL13 and/or CXCR5 expression in cancer cells.

9. The composition for use according to Claim 7, wherein said anti-CXCL13 antibody, or said anti-CXCR5 antibody, or said combination of anti-CXCL13 antibody and said anti-CXCR5 antibody is given in a dosage range of 1 ng/kg body weight/day to about 100 ng/kg body weight/day, 10 ng/kg body weight/day to about 1 μg/kg body weight/day, 100 ng/kg body weight/day to about 10 μg/kg body weight/day, 1 μg/kg body weight/day to about 100 μg/kg body weight/day, 10 μg/kg body weight/day to about 1 mg/kg body weight/day, or 100 μg/kg body weight/day to about 10 mg/kg body weight/day, or
wherein the composition further comprises a chemotherapeutic agent.

**Patentansprüche**

1. Verfahren zum Nachweisen des Vorhandenseins von Krebs bei einem Individuum, umfassend: Detektieren des Expressionsniveaus von einem oder mehreren Krebsmarkern in einer biologischen Probe, die von dem Individuum erhalten wurde; und
Vergleichen des Expressionsniveaus des einen oder der mehreren Krebsmarker in der biologischen Probe mit einem normalen Expressionsniveau des einen oder der mehreren Krebsmarker,
wobei ein höheres als normales Expressionsniveau des einen oder der mehreren Krebsmarker in der biologischen Probe das Vorhandensein von Krebs in dem Individuum anzeigt,
wobei das normale Expressionsniveau des einen oder der mehreren Krebsmarker ein vorbestimmter Wert ist oder von einer Kontrollprobe bekannter normaler nicht-kanzeröser Zellen des gleichen Ursprungs oder Typs wie die biologische Probe erhalten wird, und
wobei es sich bei dem Krebs um Ovarialkrebs, Prostatakrebs, Kolonkrebs, Brustkrebs oder Lungenkrebs handelt und wobei der eine oder die mehreren Krebsmarker CXCL13 oder CXCR5 oder sowohl CXCL13 als auch CXCR5 umfassen.

2. Verfahren nach Anspruch 1, wobei:

   der eine oder die mehreren Krebsmarker ferner CXCL16 oder CXCR6 oder sowohl CXCL16 als auch CXCR6 umfassen und wobei gegebenenfalls der eine oder die mehreren Krebsmarker ferner einen oder mehrere Krebsmarker ausgewählt aus der Gruppe bestehend aus CXCL1, CXCL2, CXCL3, CXCL4, CXCL5, CXCL6, CXCL7, CXCL8, CXCL9, CXCL10, CXCL11, CXCL12, CXCL14, CXCL15, CXCR1, CXCR2, CXCR3, CXCR4, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9 , CCR10, CCR11, XCL1, XCL2, XCR1, CX3CR1, CX3CL1, RNA-Bindungsmotiv 3 ("RBM3"), carcinoembryonalem Antigen (CEA), prostataspezifischem Antigen (PSA), Chromogranin A (CGA), Dehydroepiandrosteron (DHEA), neuronenspezifischer Enolase (NSE), Prostatasäurephosphatase (PAP), Prolaktin, B7-H3, Seprase-Polypeptid, Anti-p53, Osteopontin, Ferritin, Lysophosphatidylcholin, Kinesin-Familienmitglied 4A (KIF4A), Neuralpentraxin I (NPTX1) und Fibroblasten-Wachstumsfaktor-Rezeptor-1-Onkogenpartner(FGFR1OP)-Protein umfassen,
   wobei es sich bei der biologischen Probe um eine Plasmaprobe, eine Speichelprobe oder eine Urinprobe handelt.

3. Verfahren nach Anspruch 1,
wobei es sich bei dem Krebs um Brustkrebs handelt und wobei einer oder mehrere Krebsmarker ferner einen oder mehrere Krebsmarker umfassen, die aus der Gruppe bestehend aus CXCL16, CXCR6, CCL1, CCL4, CCL17, CCL19, CCL21, CCL22, CCL25, CXCL12, CCR7, CCR8, CCR9, CXCR4, CX3CR1, RNA-Bindungsmotif 3 ("RMB3") und carcinoembryonalem Antigen (CEA) ausgewählt sind,
wobei es sich bei dem Krebs um Prostatakrebs handelt und wobei einer oder mehrere Krebsmarker ferner einen oder mehrere Krebsmarker umfassen, die aus der Gruppe bestehend aus CXCL16, CXCR6, CCL1, CCL4, CCL17, CCL19, CCL21, CCL22, CCL25, CXCL12, CCR7, CCR8, CCR9, CXCR4, CX3CR1, PSA, CEA, CGA, DHEA, NSE, PAP, Prolactin und B7-H3 ausgewählt sind, oder
wobei es sich bei dem Krebs um Kolorektalkrebs handelt und wobei einer oder mehrere Krebsmarker ferner einen oder mehrere Krebsmarker umfassen, die aus der Gruppe bestehend aus CXCL16, CXCR6, CCL1, CCL4, CCL17, CCL19, CCL21, CCL22, CCL25, CXCL12, CCR7, CCR8, CCR9, CXCR4, CX3CR1, Seprasepolypeptid, Anti-p53, Osteopontin und Ferritin ausgewählt sind; oder
wobei es sich bei dem Krebs um Ovarialkrebs handelt und wobei einer oder mehrere Krebsmarker ferner einen oder mehrere Krebsmarker umfassen, die aus der Gruppe bestehend aus CXCL16, CXCR6, CCL1, CCL4, CCL17, CCL19, CCL21, CCL22, CCL25, CXCL12, CCR7, CCR8, CCR9, CXCR4, CX3CR1, Krebsantigen 125 (CA-125), HE-4, OVX-1 Makrophagenkoloniestimulierendem Faktor (M-CSF) und Lysophosphatidylcholin ausgewählt sind,
wobei es sich bei dem Krebs um Lungenkrebs handelt und wobei einer oder mehrere Krebsmarker ferner einen oder mehrere Krebsmarker umfassen, die aus der Gruppe bestehend aus CXCL16, CXCR6, CCL1, CCL4, CCL17, CCL19, CCL21, CCL22, CCL25, CXCL12, CCR7, CCR8, CCR9, CXCR4, CX3CR1, Kinesin-Familienmitglied 4A (IF4A), Neuralpentraxin I (NPTXI), Fibroblasten-Wachstumsfaktor-Rezeptor 1-Onkogenpartner(FGFR10P) -Protein und CEA ausgewählt sind.

4. Verfahren nach Anspruch 1,
wobei ein höheres Expressionsniveau des einen oder der mehreren Krebsmarker in der biologischen Probe relativ zu dem normalen Niveau anzeigt, dass die Prognose für den Patienten ungünstig ist,
wobei ein niedrigeres oder ein ähnliches Expressionsniveau des einen oder der mehreren Krebsmarker in der biologischen Probe relativ zu dem normalen Niveau anzeigt, dass die Prognose für den Patienten günstig ist,
wobei eine ungünstige Prognose anzeigt, dass der Krebs aggressiv oder invasiv ist, wobei es sich bei dem Krebs um Ovarialkrebs, Prostatakrebs, Kolonkrebs, Brustkrebs oder Lungenkrebs handelt,
wobei der eine oder die mehreren Krebsmarker CXCL13 oder CXCR5 oder sowohl CXCL13 als auch CXCR5 umfassen und gegebenenfalls
wobei der eine oder die mehreren Krebsmarker ferner CXCL16 oder CXCR6 oder sowohl CXCL16 als auch CXCR7 umfassen.

5. Verfahren nach Anspruch 1,
wobei das normale Niveau des einen oder der mehreren Krebsmarker ein Vorbehandlungsniveau des einen oder der mehreren Krebsmarker in dem Individuum oder ein vorbestimmter Referenzwert ist,
wobei die Behandlung als wirksam erachtet wird, wenn der eine oder die mehreren Krebsmarker in der einen oder den mehreren biologischen Proben dem normalen Niveau ähnlich oder niedriger sind,
wobei es sich bei dem Krebs um Ovarialkrebs, Prostatakrebs, Kolonkrebs, Brustkrebs oder Lungenkrebs handelt,
wobei der eine oder die mehreren Krebsmarker CXCL13 oder CXCR5 oder sowohl CXCL13 als auch CXCR5 umfassen und gegebenenfalls
wobei der eine oder die mehreren Krebsmarker ferner CXCL16 oder CXCR6 oder sowohl CXCL16 als auch CXCR7 umfassen.

6. Kit zum Detektieren von Krebs oder zur Überwachung des Fortschreitens von Krebs, umfassend:

Reagenzien zur Bestimmung der Expression von CXCL13 und/oder CXCR5 in einer biologischen Probe; und Anweisungen zur Verwendung der Reagenzien,
wobei die Reagenzien einen Anti-CXCL13-Antikörper, einen Anti-CXCR5-Antikörper oder beide umfassen und wobei es sich bei dem Krebs um Ovarialkrebs, Prostatakrebs, Kolonkrebs, Brustkrebs oder Lungenkrebs handelt und
wobei das Kit ferner umfasst:

Reagenzien zum Bestimmen der Expression von CXCL16 und/oder CXCR6 in einer biologischen Probe; und Anweisungen zur Verwendung der Reagenzien,
wobei die Reagenzien einen Anti-CXCL16-Antikörper, einen Anti-CXCR6-Antikörper oder beides umfassen.

7. Zusammensetzung, umfassend eine therapeutisch wirksame Menge eines Anti-CXCL13-Antikörpers oder eines Anti-CXCR5-Antikörpers oder eine Kombination davon zur Verwendung in einem Verfahren für die Behandlung von Krebs,
wobei es sich bei dem Krebs um Ovarialkrebs, Prostatakrebs, Kolonkrebs, Brustkrebs, Lungenkrebs handelt und wobei der Anti-CXCL13-Antikörper oder der Anti-CXCR5-Antikörper oder die Kombination aus dem Anti-CXCL13-Antikörper und dem Anti-CXCR5-Antikörper in einem Dosierungsbereich von 1 ng pro kg Körpergewicht pro Tag bis ungefähr 100 mg pro kg Körpergewicht pro Tag verabreicht wird.

8. Zusammensetzung zur Verwendung nach Anspruch 7, wobei:

die Zusammensetzung ferner einen oder mehrere Antikörper umfasst, die spezifisch an eines oder mehrere Chemokine oder an einen oder mehrere Chemokinrezeptoren binden, die aus der Gruppe bestehend aus CCL1, CCL2, CCL4, CCL17, CCL19, CCL21, CCL22, CCL25, CXCL12, CXCL16, CCR2, CCR7, CCR8, CCR9, CXCR4, CXCR6, CX3CL1 und CX3CR1 ausgewählt sind; oder
wobei der Krebs zu einer erhöhten CXCL13- und/oder CXCR5-Expression in Krebszellen führt.

9. Zusammensetzung zur Verwendung nach Anspruch 7, wobei der Anti-CXCL13-Antikörper oder der Anti-CXCR5-Antikörper oder die Kombination aus dem Anti-CXCL13-Antikörper und dem Anti-CXCR5-Antikörper in einem Dosierungsbereich von 1 ng pro kg Körpergewicht pro Tag bis ungefähr 100 mg pro kg Körpergewicht pro Tag, 10 ng pro kg Körpergewicht pro Tag bis ungefähr 1 $\mu$g pro kg Körpergewicht pro Tag, 100 ng pro kg Körpergewicht pro Tag bis ungefähr 10 $\mu$g pro kg Körpergewicht pro Tag, 1 $\mu$g pro kg Körpergewicht pro Tag bis ungefähr 100 $\mu$g pro kg Körpergewicht pro Tag, 10 $\mu$g pro kg Körpergewicht pro Tag bis ungefähr 1 mg pro kg Körpergewicht pro Tag oder 100 $\mu$g pro kg Körpergewicht pro Tag bis ungefähr 10 mg pro kg Körpergewicht pro Tag verabreicht wird oder wobei die Zusammensetzung ferner ein Chemotherapeutikum umfasst.

**Revendications**

1. Procédé pour détecter la présence d'un cancer chez un sujet, comprenant le fait : de détecter le niveau d'expression d'un ou de plusieurs marqueur(s) de cancer dans un échantillon biologique obtenu à partir dudit sujet ; et

   de comparer le niveau d'expression dudit ou desdits plusieurs marqueur(s) de cancer dans ledit échantillon biologique à un niveau normal d'expression dudit ou desdits plusieurs marqueur(s) de cancer,

   dans lequel un niveau d'expression supérieur à la normale dudit ou desdits plusieurs marqueur(s) de cancer dans ledit échantillon biologique indique la présence d'un cancer chez ledit sujet,

   dans lequel ledit niveau d'expression normal dudit ou desdits plusieurs marqueur(s) de cancer est une valeur prédéterminée ou est obtenu à partir d'un échantillon témoin de cellules non cancéreuses normales connues de même origine ou type que ledit échantillon biologique, et

   dans lequel ledit cancer est le cancer de l'ovaire, le cancer de la prostate, le cancer du côlon, le cancer du sein ou le cancer du poumon, et

   dans lequel ledit ou lesdits plusieurs marqueur(s) de cancer comprend/comprennent CXCL13 ou CXCR5 ou à la fois CXCL13 et CXCR5.

2. Procédé de la revendication 1, dans lequel :

   ledit ou lesdits plusieurs marqueur(s) de cancer comprend/comprennent en outre CXCL16 ou CXCR6 ou à la fois CXCL16 et CXCR6 et facultativement dans lequel ledit ou lesdits plusieurs marqueur(s) de cancer comprend/comprennent en outre un ou plusieurs marqueur(s) de cancer choisi(s) dans le groupe constitué de CXCL1, CXCL2, CXCL3, CXCL4 CXCL5, CXCL6, CXCL7, CXCL8, CXCL9, CXCL10, CXCL11, CXCL12, CXCL14, CXCL15, CXCR1, CXCR2, CXCR3, CXCR4, CXCR7, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL24, CCL25, CCL25-1, CCL25-2, CCL27, CCL28, CCR1, CCR2, CCR3, CCR4, CCR5, CCR6, CCR7, CCR8, CCR9, CCR10, CCR11, XCL1, XCL2, XCR1, CX3CR1, CX3CL1, du motif de liaison à l'ARN 3 (« RBM3 »), de l'antigène carcino-embryonnaire (CEA), de l'antigène spécifique de la prostate (PSA), de la chromogranine A (CGA), de la déhydroépiandrostérone (DHEA), de l'énolase spécifique des neurones (NSE), de la phosphatase acide prostatique (PAP), de la prolactine, de B7-H3, d'un polypeptide séprase, d'anti-p53, de l'ostéopontine, de la ferritine, de la lysophosphatidylcholine, du membre 4A de la famille des kinésines (KIF4A), de la pentraxine neurale I (NPTX1) et de la protéine partenaire oncogène du récepteur 1 du facteur de croissance des fibroblastes (FGFR1OP) ; ou

   dans lequel ledit échantillon biologique est un échantillon de plasma, un échantillon de salive ou un échantillon d'urine.

3. Procédé de la revendication 1,

   dans lequel ledit cancer est le cancer du sein et dans lequel un ou plusieurs marqueur(s) de cancer comprend/comprennent en outre un ou plusieurs marqueur(s) de cancer choisi(s) dans le groupe constitué de CXCL16, CXCR6, CCL1, CCL4, CCL17, CCL19, CCL21, CCL22, CCL25, CXCL12, CCR7, CCR8, CCR9, CXCR4, CX3CR1, du motif 3 de liaison à l'ARN (« RBM3 ») et de l'antigène carcino-embryonnaire (CEA),

   dans lequel ledit cancer est un cancer de la prostate et dans lequel un ou plusieurs marqueur(s) de cancer comprend/comprennent en outre un ou plusieurs marqueur(s) de cancer choisi(s) dans le groupe constitué de CXCL16, CXCR6, CCL1, CCL4, CCL17, CCL19, CCL21, CCL22, CCL25, CXCL12, CCR7, CCR8, CCR9, CXCR4, CX3CR1, PSA, CEA, CGA, DHEA, NSE, PAP, de la prolactine et de B7-H3 ; ou

   dans lequel ledit cancer est un cancer colorectal et dans lequel un ou plusieurs marqueur(s) de cancer comprend/comprennent en outre un ou plusieurs marqueur(s) de cancer choisi(s) dans le groupe constitué de CXCL16, CXCR6, CCL1, CCL4, CCL17, CCL19, CCL21, CCL22, CCL25, CXCL12, CCR7, CCR8, CCR9, CXCR4, CX3CR1, d'un polypeptide séprase, d'anti-p53, de l'ostéopontine et de la ferritine ; ou

   dans lequel ledit cancer est un cancer de l'ovaire et dans lequel un ou plusieurs marqueur(s) de cancer comprend/comprennent en outre un ou plusieurs marqueur (s) de cancer choisi(s) dans le groupe constitué de CXCL16, CXCR6, CCL1, CCL4, CCL17, CCL19, CCL21, CCL22, CCL25, CXCL12, CCR7, CCR8, CCR9, CXCR4, CX3CR1, de l'antigène de cancer 125 (CA-125), de HE-4, d'OVX-1, du facteur de stimulation des colonies de macrophages (M-CSF) et de la lysophosphatidylcholine,

dans lequel ledit cancer est un cancer du poumon et dans lequel un ou plusieurs marqueur(s) de cancer comprend/comprennent en outre un ou plusieurs marqueur(s) de cancer choisi(s) dans le groupe constitué de CXCL16, CXCR6, CCL1, CCL4, CCL17, CCL19, CCL21, CCL22, CCL25, CXCL12, CCR7, CCR8, CCR9, CXCR4, CX3CR1, du membre 4A de la famille des kinésines (IF4A), de la pentraxine neurale 1 (NPTX1), de la protéine partenaire oncogène du récepteur 1 du facteur de croissance des fibroblastes (FGFR1OP) et de CEA.

4.  Procédé de la revendication 1,
    dans lequel un niveau d'expression plus élevé dudit ou desdits plusieurs marqueur(s) de cancer dans l'échantillon biologique par rapport audit niveau normal indique que le pronostic dudit sujet est mauvais,
    dans lequel un niveau d'expression plus faible ou similaire dudit ou desdits plusieurs marqueur(s) de cancer dans ledit échantillon biologique par rapport audit niveau normal indique que le pronostic dudit sujet est bon,
    dans lequel un mauvais pronostic indique que ledit cancer est de type agressif ou invasif, dans lequel ledit cancer est le cancer de l'ovaire, le cancer de la prostate, le cancer du côlon, le cancer du sein ou le cancer du poumon,
    dans lequel ledit ou lesdits plusieurs marqueur(s) de cancer comprend/comprenant CXCL13 ou CXCR5 ou à la fois CXCL13 et CXCR5, et facultativement
    dans lequel ledit ou lesdits plusieurs marqueur(s) de cancer comprend/comprenant en outre CXCL16 ou CXCR6 ou à la fois CXCL16 et CXCR6.

5.  Procédé de la revendication 1
    dans lequel ledit niveau normal dudit ou desdits plusieurs marqueur(s) de cancer est un niveau de prétraitement dudit ou desdits plusieurs marqueur(s) de cancer chez ledit sujet ou une valeur de référence prédéterminée,
    dans lequel ledit traitement est jugé efficace si ledit ou lesdits plusieurs marqueur(s) de cancer dans ledit ou lesdits plusieurs échantillon(s) biologique(s) est/sont similaire(s) audit niveau normal ou inférieur(s) à celui-ci,
    dans lequel ledit cancer est le cancer de l'ovaire, le cancer de la prostate, le cancer du côlon, le cancer du sein ou le cancer du poumon,
    dans lequel ledit ou lesdits plusieurs marqueur(s) de cancer comprend/comprenant CXCL13 ou CXCR5 ou à la fois CXCL13 et CXCR5 et facultativement
    dans lequel ledit ou lesdits plusieurs marqueur(s) de cancer comprend/comprennent en outre CXCL16 ou CXCR6 ou à la fois CXCL16 et CXCR6.

6.  Kit pour détecter un cancer ou surveiller la progression de cancer, comprenant :

    des réactifs pour déterminer l'expression de CXCL13 et/ou CXCR5 dans un échantillon biologique ; et des instructions pour savoir comment utiliser lesdits réactifs,
    dans lequel lesdits réactifs comprennent un anticorps anti-CXCL13, un anticorps anti-CXCR5 ou les deux et
    dans lequel ledit cancer est le cancer de l'ovaire, le cancer de la prostate, le cancer du côlon, le cancer du sein ou le cancer du poumon et
    dans lequel le kit comprend en outre :

    des réactifs pour déterminer l'expression de CXCL16 et/ou CXCR6 dans un échantillon biologique ; et des instructions pour savoir comment utiliser lesdits réactifs,
    dans lequel lesdits réactifs comprennent un anticorps anti-CXCL16, un anticorps anti-CXCR6, ou les deux.

7.  Composition comprenant une quantité thérapeutiquement efficace d'un anticorps anti-CXCL13, ou d'un anticorps anti-CXCR5, ou d'une combinaison de ceux-ci pour une utilisation dans un procédé pour le traitement de cancer, où ledit cancer est le cancer de l'ovaire, le cancer de la prostate, le cancer du côlon, le cancer du sein, le cancer du poumon, et où ledit anticorps anti-CXCL13, ou ledit anticorps anti-CXCR5, ou ladite combinaison d'anticorps anti-CXCL13 et dudit anticorps anti-CXCR5 est administré(e) dans une plage posologique allant de 1 ng/kg de poids corporel/jour à environ 100 mg/kg de poids corporel/jour.

8.  Composition pour une utilisation selon la revendication 7, dans laquelle :

    la composition comprend en outre un ou plusieurs anticorps qui se lie/lient spécifiquement à un(e) ou plusieurs chimiokine(s) ou récepteur(s) de chimiokine(s) choisi(e)(s) dans le groupe constitué de CCL1, CCL2, CCL4, CCL17, CCL19, CCL21, CCL22, CCL25, CXCL12, CXCL16, CCR2, CCR7, CCR8, CCR9, CXCR4, CXCR6, CX3CL1 et CX3CR1 ; ou

dans laquelle le cancer entraîne une expression élevée de CXCL13 et/ou CXCR5 dans des cellules cancéreuses.

9. Composition pour une utilisation selon la revendication 7, dans laquelle ledit anticorps anti-CXCL13, ou ledit anticorps anti-CXCR5, ou ladite combinaison d'anticorps anti-CXCL13 et dudit anticorps anti-CXCR5 est administré(e) dans une plage posologique allant de 1 ng/kg de poids corporel/jour à environ 100 ng/kg de poids corporel/jour, de 10 ng/kg de poids corporel/jour à environ 1 $\mu$g/kg de poids corporel/jour, de 100 ng/kg de poids corporel/jour à environ 10 $\mu$g/kg de poids corporel/jour, de 1 $\mu$g/kg de poids corporel/jour à environ 100 $\mu$g/kg de poids corporel/jour, de 10 $\mu$g/kg de poids corporel/jour à environ 1 mg/kg de poids corporel/jour, ou de 100 $\mu$g/kg de poids corporel/jour à environ 10 mg/kg de poids corporel/jour, ou

dans laquelle la composition comprend en outre un agent chimiothérapeutique.

FIG. 1

**FIG. 2**

FIG. 3

| CXCL13 | - | - | - | + | + | + |
|---|---|---|---|---|---|---|
| DOCK2 siRNA | - | + | - | - | + | - |
| Control siRNA | - | - | + | - | - | + |

p-JNK

GAPDH

RWPE-1

p-JNK

GAPDH

LNCaP

p-JNK

GAPDH

PC3

*FIG. 4*

FIG. 5

**FIG. 6**

FIG. 7

**FIG. 8**

FIG. 9

FIG. 10

*FIG. 11*

FIG. 12

FIG. 13

**FIG. 14**

FIG. 15

**FIG. 16**

**FIG. 17**

FIG. 18

**Survival    Invasion    Growth**

*FIG. 19*

FIG. 20

**FIG. 21**

FIG. 22A

FIG. 22B

*FIG. 23*

FIG. 24A

FIG. 24A (continued)

**FIG. 24B**

**FIG. 25**

**CXCL13 blockade inhibits change in bone mineral density (BMD) induced by prostate cancer**

FIG. 26

*FIG. 27*

FIG. 28

**FIG. 29**

FIG. 30

FIG. 31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 24890411 A
- US 13233769 B
- US 12967273 B
- US 31234311 A
- US 96727310 A
- WO 2010003771 A
- WO 2008102123 A
- WO 2012031099 A
- WO 0137872 A
- WO 2004015426 A
- US 4816567 A
- US 4676980 A
- US 5719060 A, Hutchens & Yip
- WO 9859361 A, Hutchens & Yip
- US 4868116 A
- US 4980286 A
- WO 9002806 A
- WO 8907136 A
- US 6261834 B
- US 5135917 A
- US 5994320 A
- US 6046319 A
- US 6057437 A
- US 5543293 A
- US 5476766 A
- US 5861254 A
- US 6030776 A
- US 6051698 A

- US 5334711 A
- US 5861288 A
- WO 9858058 A
- WO 9718312 A
- US 5631115 A
- US 6022962 A
- US 5595873 A
- US 5652107 A
- US 5580967 A
- US 5910408 A
- US 5646042 A
- US 5869253 A
- US 5989906 A
- US 6017756 A
- US 5176996 A
- US 5683874 A
- US 5874566 A
- US 5962426 A
- WO 9203566 A, Yale
- WO 9322434 A, Yale
- WO 9524489 A, Yale
- US 5168053 A
- US 5624824 A
- US 5683873 A
- US 5728521 A
- US 5869248 A
- US 5877162 A
- US 4522811 A

### Non-patent literature cited in the description

- **W. VERMI et al.** *Identification of CXCL13 as a new marker for follicular dendritic cell sarcoma*
- **S YUVARAJ et al.** *A novel function of CXCL13 to Stimulate RANK Ligand Expression in Oral Squamous Cell Carcinoma Cells*
- **RODRIGUES-PINILLA SOCORRO MARIA et al.** *Peripheral T-cell lymphoma with follicular T-cell markers*
- **A BURKLE et al.** *Overexpression of the CXCR5 chemokine receptor, and its ligand, CXCL13 in B-cell chronic lymphocytic leukemia*
- **SUBRAMANYA N.M. PANDRUVADA et al.** *Role of CXC chemokine ligand 13 in oral squamous cell carcinoma associated osteolysis in athymic mice*
- **R&D SYSTEMS.** *Monoclonal anti-human CXCL13/BLC/BCA-1 antibody*

- **MERAV DARASH-YAHANA et al.** *The Chemokine CXCL16 and its Receptor, CXCR6, as Markers and Promoters of Inflammation-Associated Cancers*
- **S. HOJO et al.** *High-Level Expression of Chemokine CXCL 16 by Tumor Cells Correlates with a Good Prognosis and Increased Tumor-Infiltrating Lymphocytes in Colorectal Cancer*
- **GUTWEIN P et al.** *Tumoural CXCL16 expression is a novel prognostic marker of longer survival times in renal cell cancer patients*
- **MORRISON et al.** *Proc. Natl. Acad. Sci. USA,* 1984, vol. 81, 6851-6855
- **MULLIGAN.** *Science,* 1993, vol. 260, 926-932
- **SUN et al.** *Nature genetics,* 1994, vol. 8, 33-41
- **COTTER ; ROBERTSON.** *Curr Opin Mol Ther,* 1999, vol. 5, 633-644

- **SCHARF et al.** *Results Probl Cell Differ,* 1994, vol. 20, 125-62
- **BITTER et al.** *Methods in Enzymol,* 1987, vol. 153, 516-544
- **HAMMOND et al.** *Nature Rev Gen,* 2001, vol. 2, 110-119
- **SHARP.** *Genes Dev,* 2001, vol. 15, 485-490
- **WATERHOUSE et al.** *Proc. Natl. Acad. Sci. USA,* 1998, vol. 95 (23), 13959-13964
- **MARRO et al.** *Biochem Biophys Res Commun,* 13 October 2006, vol. 349, 270-6
- **FORSTER ; ALTMAN.** *Science,* 1990, vol. 238, 407-409
- **YUAN et al.** *Proc. Natl. Acad. Sci. USA,* 1992, vol. 89, 8006-8010
- **YUAN ; ALTMAN.** *EMBO J,* 1995, vol. 14, 159-168
- **CARRARA et al.** *Proc. Natl. Acad. Sci. USA,* 1995, vol. 92, 2627-2631